# EUROPEAN PATENT APPLICATION

(11) **EP 4 056 672 A1**
(43) Date of publication of application: **14.09.2022**
(21) Application number: 20886869.5
(22) Date of filing: 29.09.2020
(51) Int. Cl.: C12M 1/34, G01N 21/27, G06T 7/00, C12Q 1/02

(54) **METHOD FOR ASSESSING ASSESSMENT TARGETS, IMAGE PROCESSING DEVICE, SYSTEM FOR ASSESSING ASSESSMENT TARGETS**

(30) Priority: 12.11.2019 JP 2019205016
(71) Applicant: SUMITOMO CHEMICAL COMPANY, LIMITED, Tokyo 103-6020 (JP)
(72) Inventor: OZAKI Maya, Niihama-shi, Ehime 792-8521 (JP); INOUE Yoko, Osaka-shi, Osaka 554-8558 (JP); YAMADA Sachiko, Osaka-shi, Osaka 554-8558 (JP); MATSUYAMA Ryoko, Osaka-shi, Osaka 554-8558 (JP); KANO Hanaho, Osaka-shi, Osaka 554-8558 (JP)
(74) Representative: Winter, Brandl - Partnerschaft mbB
(86) International application number: PCT/JP2020/037005
(87) International publication number: WO 2021/095381

(57) **Abstract**

A method for assessing an assessment target according to an embodiment includes: a step of acquiring statistical information based on at least one color feature quantity with respect to each of a plurality of assessment regions in a plate image corresponding to an image of an assessment plate that holds an assessment target in a plurality of wells provided in the plate; and a step of determining a color of the plurality of assessment regions by using the statistical information. The assessment target includes a tester, the plurality of wells include a test substance well holding the assessment target that further includes a test substance, the plate image includes a plurality of well images corresponding to the plurality of wells, and each of the plurality of assessment regions includes at least one well image corresponding to at least one of the wells.

## Description

### Technical Field

The present invention relates to a method for assessing assessment targets, an image processing device, and a system for assessing assessment targets.

### Background Art

In biological safety assessment, it is known that a test substance is added to a tester and a change thereof (for example, a color change) is observed. For example, in an Ames test, cells modified so that synthesis of amino acid cannot be performed by genetically manipulating an amino acid synthesis gene are used as the tester. In addition, in the Ames test, a test substance is added to the cells, and the cells are cultured under constant conditions. When mutation occurs in the amino acid synthesis gene of the cells due to the test substance, the cells can synthesize the amino acid again. Accordingly, assessment on whether or not the mutation has occurred is made by confirming presence or absence of cell proliferation. At this time, for example, when using an indicator whose color varies due to cell proliferation, the cell proliferation can be assessed by the color change.

In the related art, in the safety assessment, a petri dish is frequently used for retaining the tester and the test substance. In this case, for example, when assessing safety by changing a concentration of the test substances with respect to the same tester, the petri dish is prepared for every concentration. Accordingly, it is necessary to prepare a large amount of test substance. In contrast, recently, in order to reduce the amount of the tester and the test substance, or the like, for example, as disclosed in Non Patent Literature 1, Non Patent Literature 2, and Non Patent Literature 3, an Ames test using a plate including a plurality of wells is known.

### Citation List

### Non Patent Literature

Non Patent Literature 1: Fluckiger-Isler S., Kamber M., "Direct comparison of the Ames microplate format (MPF) test in liquid medium with the standard Ames pre-incubation assay on agar plates by use of equivocal to weakly positive test compounds.", Mutation Research/Genetic Toxicology and Environmental Mutagenesis 747, p 36 to 45, 2012.

Non Patent Literature 2: Sui H., Kawakami K., Sakurai N., Hara T., Nohmi T., "Improvement and evaluation of high throughput fluctuation Ames test using 384-well plate with Salmonella typhimurium TA100 and TA98.", Genes and Environment 31(2), p 47 to 55, 2009.

Non Patent Literature 3: Kamber M., Fluckiger-Isler S., Engelhardt G., Jaeckh R., Zeiger E., "Comparison of the Ames II and traditional Ames test responses with respect to mutagenicity, strain specificities, need for metabolism and correlation with rodent carcinogenicity", Mutagenesis 24(4), p 359 to 366, 2009.

### Summary of Invention

### Technical Problem

In the biological safety assessment, a change when adding the test substance to the tester (for example, proliferation in a case where the tester is a cell, a color change in the case of using an indicator, or the like) is assessed with naked eyes of an assessment member in the related art. Therefore, for example, in the case of performing the safety assessment by using the plate including the plurality of wells, a burden on the assessment member becomes heavy, and thus there is a concern that objectivity and accuracy of assessment deteriorate.

Here, an object of the invention is to provide a method for assessing an assessment target, an image processing device, and a system for assessing an assessment target which are capable of performing assessment objectively and with more accuracy in biological safety assessment.

### Solution to Problem

According to an aspect of the invention, there is provided a method for assessing an assessment target. The method includes: a step of acquiring statistical information based on at least one color feature quantity with respect to each of a plurality of assessment regions in a plate image corresponding to an image of an assessment plate that holds an assessment target in a plurality of wells provided in the plate; and a step of determining a color of the plurality of assessment regions by using the statistical information. The assessment target includes a tester, the plurality of wells include a test substance well holding the assessment target that further includes a test substance, the plate image includes a plurality of well images corresponding to the plurality of wells, and each of the plurality of assessment regions includes at least one well image corresponding to at least one of the wells.

In the assessment method, statistical information based on at least one color feature quantity is acquired with respect to a plurality of assessment regions in the plate image. The color of the assessment regions is determined by using the statistical information acquired with respect to the plurality of assessment regions. According to this, the color of the assessment regions can be determined objectively and with high accuracy. As a result, safety of the test substance can be assessed objectively and with high accuracy on the basis of the color of the assessment regions.

The at least one color feature quantity may be two or more color feature quantities. An example of the two or more color feature quantities may be hue and saturation. The statistical information may be a frequency distribution in which the two or more color feature quantities are variables.

The at least one color feature quantity may be one color feature quantity, and the statistical information may include at least one kind of statistical quantity for the one color feature quantity.

In the step of determining the color of the plurality of assessment regions, the color of the plurality of assessment regions may be determined based on a similarity of the statistical information.

The step of determining the color of the plurality of assessment regions may include a step of performing a primary determination of the color of the plurality of assessment regions among a plurality of color candidates based on the similarity of the statistical information, a step of acquiring first reference statistical information for the at least one color feature quantity with respect to the plurality of color candidates based on a result of the primary determination, and a step of performing a final determination of the color of the plurality of assessment regions by using the first reference statistical information and the similarity of the statistical information, and in the step of acquiring the first reference statistical information, the first reference statistical information may be acquired based on a statistical information of at least one assessment region determined by each color in the result of the primary determination among the statistical information for the plurality of assessment regions. In this case, since determination is performed two times, the color of the assessment regions can be more accurately determined.

The plurality of assessment regions may be the plurality of well images, the step of determining the color of the plurality of assessment regions may include a step of performing a primary determination of a color of the plurality of well images among a plurality of color candidates based on the similarity of the statistical information, a step of acquiring a first reference statistical information for the at least one color feature quantity with respect to the plurality of color candidates based on a result of the primary determination, a step of performing a secondary determination of a color of the plurality of well images among the plurality of color candidates by using a similarity between the statistical information and the first reference statistical information, a step of acquiring a second reference statistical information for the at least one color feature quantity with respect to the plurality of color candidates based on a result of the secondary determination, and a step of performing a tertiary determination of a color of the plurality of well images by using the second reference statistical information, first to N^{th} sections (N is an integer of two or greater) including at least one well among the plurality of wells are set in the plate, in the step of acquiring the second reference statistical information, in an i^{th} section image among first to N^{th} section images corresponding to the first to N^{th} sections in the plate image, the second reference statistical information for the plurality of color candidates may be acquired based on a statistical information of at least one well image determined as the same color candidate among the statistical information for the plurality of well images, in the step of performing the tertiary determination of the color of the plurality of well images, the color of the plurality of well images may be finally determined by determining the color of a well image pertaining to the i^{th} section among the plurality of well images based on a similarity between the second reference statistical information acquired for the i^{th} section image and a statistical information of at least one well pertaining to the i^{th} section image among the statistical information for the plurality of well images. In this case, since the color is determined three times, the color of the assessment regions can be more accurately determined.

In the step of determining the color of the plurality of assessment regions, the color of the plurality of assessment regions may be determined based on a similarity between the statistical information and a reference statistical information that is reference statistical information for each of a plurality of color candidates and is based on the at least one color feature quantity. In this case, the color of the assessment region can be assessed in a simple manner.

The method for assessing an assessment target according to the embodiment may further include a step of detecting a transparency change of the plurality of assessment regions based on the statistical information and a similarity between a reference statistical information for detecting a transparency change based on the at least one color feature quantity. For example, when there is change such as cell death and precipitation of a test substance, transparency of the assessment target changes. Accordingly, in the method, for example, it is possible to assess whether or not cells die or whether or not the test substance is precipitated.

The plurality of wells may further include at least one a solvent control well for solvent control, one of the plurality of assessment regions may be a solvent control region including at least one solvent control well image corresponding to the at least one solvent control well, and in the step of detecting the transparency change of the plurality of assessment regions, a statistical information of the solvent control region among the statistical information for the plurality of assessment regions may be set as reference statistical information for detecting a transparency change, and a transparency change of the plurality of assessment regions may be detected based on a similarity between the statistical information of the solvent control region and a remaining statistical information among the statistical information for the plurality of assessment regions. In this case, since the transparency change is detected on the basis of the statistical information of the solvent control region in the same plate image, an influence by test conditions (for example, culture temperature conditions) or the like can be reduced, and the transparency change can be more accurately detected.

The method for assessing an assessment target according to the embodiment may further include a step of detecting presence or absence of a shape change of the tester of the assessment target held in the plurality of wells based on the plurality of well images. According to this, for example, in the case of using cells as the tester, colonies due to cell proliferation, and the like can be detected.

The method for assessing an assessment target according to the embodiment may further include a step of detecting presence or absence of a foreign matter in the plurality of wells based on the plurality of well images. According to this, for example, in the case of using cells as the tester, the foreign matter due to death of cells, and the like can be detected. In this specification, the foreign matter is different from a structure caused by a shape change of the tester. Accordingly, the content of the foreign matter is different from the content of the structure caused by the shape change of the tester.

The step of detecting presence or absence of the foreign matters in the plurality of wells may include a step of detecting presence or absence of the foreign matters based on a first luminance threshold value set to each of the plurality of well images, and a step of detecting presence or absence of the foreign matters in an outer region based on a second luminance threshold value set in the outer region that surrounds an inner region corresponding to a bottom region of a well when viewed from a depth direction of the well in each of the plurality of well image, the first luminance threshold value and the second luminance threshold value may be different from each other, and an area of the bottom in each of the plurality of wells may be smaller than an area of opening opposite to the bottom. For example, the outer region is darker than the inner region of the well, and thus the foreign matter is less likely to be detected. However, in the configuration, since detection is performed by setting the luminance threshold value to the outer region, and thus the foreign matter located in the outer region can also be reliably detected.

The plate may be a plate that is determined to be appropriate by a plate assessing method in which the plate is determined to be appropriate in a case at least one assessment well among a plurality of assessment wells holds the foreign matter after a constant time has elapsed since an assessment sample including the tester and the test substance selected and adjusted so that the foreign matters occur is injected into a plurality of assessment wells provided in a plate to be assessed. "Plate determined to be appropriate" is intended also to include a plate to be determined in a case where the determination method is applied as well as a plate that has been determined by carrying out the plate determination method.

In this case, the plate determined to be appropriate in the plate determination method is a plate capable of holding the foreign matter. Accordingly, it is possible to appropriately detect the foreign matter in the assessment plate (specifically, an assessment target) after a constant time has elapsed from the injection of the assessment target into the wells, and thus the assessment target can be more accurately assessed.

The plate may be a polystyrene plate with no surface coating, a polystyrene plate in which a surface is coated with Poly-D-Lysine, a cyclic olefin copolymer plate in which a tissue culture treatment is performed on a surface, or a cycloolefin polymer plate with no surface coating.

The plate may be a plate including a glass plate, and a resin layer provided on a surface of the glass plate, the resin layer may be formed from a cyclic olefin copolymer or polystyrene, a plurality of through-holes corresponding to the plurality of wells may be formed in the resin layer, and regions exposed by the plurality of through-holes in the surface may not be subjected to coating.

Alternatively, the plate may be a plate including a glass plate, and a resin layer provided on a surface of the glass plate, the resin layer may be formed from a cyclic olefin copolymer or polystyrene, a plurality of through-holes corresponding to the plurality of wells may be formed in the resin layer, a coating layer may be formed in regions exposed by the plurality of through-holes in the surface, and a material of the coating layer may be fibronectin, collagen, or laminin.

Such a plate can be determined to be appropriate in the plate determination process.

According to another aspect of the invention, there is provided a method for assessing an assessment target. The method includes: a step of acquiring statistical information based on at least one color feature quantity with respect to each of a plurality of assessment regions in a plate image corresponding to an image of an assessment plate that holds an assessment target in a plurality of wells provided in a plate; and a step of detecting a transparency change of the plurality of assessment regions based on a similarity between the statistical information and a reference statistical information for detecting a transparency change based on the at least one color feature quantity. The assessment target includes a tester, the plurality of wells include a test substance well holding the assessment target that further includes a test substance, and each of the plurality of assessment regions includes at least one well image corresponding to at least one of the wells.

In the assessment method, statistical information based on at least one color feature quantity is acquired with respect to a plurality of assessment regions in the plate image. The transparency change of the assessment regions is detected by using the statistical information acquired with respect to the plurality of assessment regions. Accordingly, the transparency change of the assessment region can be detected objectively and with high accuracy. As a result, safety of the test substance can be assessed objectively and with high accuracy on the basis of the transparency change of the assessment region.

The method for assessing an assessment target according to an embodiment may further include an irradiation step of irradiating the assessment plate with light. In in the irradiation step, the assessment plate may be irradiated with light having a wavelength within at least one wavelength range between a wavelength range of 430 to 460 nm and a wavelength range of 520 to 620 nm.

In this case, a luminance difference between wells different in the state of the assessment target or the like increases. Accordingly, for example, the difference in color becomes clearer. As a result, the assessment target can be more accurately assessed.

The light emitted to the assessment plate in the irradiation step may be light having a first wavelength selected in a wavelength range of 430 to 460 nm and a second wavelength selected in a wavelength range of 520 to 620 nm.

In the assessment plate, a well (specifically, an assessment target) having a color close to a color expressed by a wavelength within a wavelength range of 430 to 460 nm, and a well (specifically, an assessment target) having a color close to a color expressed by a wavelength within a wavelength range of 520 to 620 nm may be present respectively. Accordingly, in a case where light L has the first wavelength and the second wavelength, it is easy to secure luminance of the well with respect to both the color on a side of the wavelength range of 430 to 460 nm, and the color on a side of the wavelength range of 520 to 620 nm. As a result, since the difference in color becomes clearer, the assessment target can be more accurately assessed.

An imaging step of imaging the assessment plate may be further provided.

In the imaging step, light having a wavelength within at least one wavelength range between a wavelength range of 430 to 460 nm, and a wavelength range of 520 to 620 nm may be selectively received. In this case, a luminance difference between wells different in the state of the assessment target or the like in an image obtained by imaging the assessment plate increases. Accordingly, for example, the difference in color becomes clearer. As a result, the assessment target can be more accurately assessed.

In the imaging step, the assessment plate may be imaged from a bottom side of the plurality of wells. For example, in a case where cells are used as the tester, when the cells proliferate to form colonies or when the cells die to occur a foreign matter and the like, the colonies or the foreign matter tend to sink to the bottom of the well. Accordingly, when imaging the assessment plate from the bottom side of the plurality of wells, the colonies, the foreign matter, and the like can be more reliably detected.

According to still another aspect of the invention, there is provided an image processing device configured to execute the assessment method according to the invention.

According to still another aspect of the invention, there is provided a system for assessing an assessment target. The system includes: a plate support configured to hold an assessment plate holding an assessment target in a plurality of wells provided in a plate; an imaging unit configured to image the assessment plate; and the image processing device configured to assess a plate image corresponding to the assessment plate which is obtained on the basis of an imaging result in the imaging unit.

The imaging unit may be disposed on a bottom side of the plurality of wells.

### Advantageous Effects of Invention

According to the invention, a method for assessing an assessment target, an image processing device, and a system for assessing an assessment target which are capable of performing more objective and accurate assessment in biological safety assessment can be provided.

### Brief Description of Drawings

FIG. 1 is a plan view of an assessment plate that is used in a method for assessing an assessment target according to an embodiment.
FIG. 2 is a schematic view of a cross-section of a well.
FIG. 3 is a schematic view of a system for assessing the assessment target according to the embodiment.
FIG. 4 is a functional block diagram of an operation unit in the case of performing a color determination process.
FIG. 5 is a view illustrating an example of an image (a plate image) of an assessment plate which is created in a plate image creation unit.
FIG. 6 is a view illustrating an example of an HSV image.
FIG. 7 is a view illustrating an example of a histogram of a frequency distribution created with respect to wells.
FIG. 8 is a graph illustrating an example of a similarity distribution for purple and yellow which is created for primary determination.
FIG. 9 is a graph illustrating an example of a similarity distribution created for secondary determination.
FIG. 10 is a graph illustrating an example of a similarity distribution for each of eight sections.
FIG. 11 is an example of a flowchart of an assessment method using an example of the color determination process.
FIG. 12 is a functional block diagram of an operation unit in the case of performing another example of the color determination process.
FIG. 13 is a graph illustrating an example of a similarity distribution for each of eight sections so as to determine a color in the other example of the color determination process.
FIG. 14 is a functional block diagram of an operation unit in the case of creating a reference frequency distribution used in still another example of the color determination process.
FIG. 15 is an example of a flowchart of an assessment method using the other example of the color determination process.
FIG. 16 is a functional block diagram of an operation unit in the case of performing a colony detection process.
FIG. 17 is a view for describing the colony detection process by the operation unit illustrated in FIG. 16.
FIG. 18 is an example of a flowchart of an assessment method using the colony detection process.
FIG. 19 is a functional block diagram of an operation unit in the case of performing a transparency change detection process.
FIG. 20 is a chart illustrating similarity of section frequency distribution between eight sections.
FIG. 21 is an example of a flowchart of an assessment method using the transparency change detection process.
FIG. 22 is a functional block diagram of an operation unit in the case of performing a foreign matter detection process.
FIG. 23 is a view for describing the foreign matter detection process by the operation unit illustrated in FIG. 22.
FIG. 24 is an example of a flowchart of an assessment method using the foreign matter detection process.
FIG. 25 is a chart illustrating test conditions in Experimental Example 1.
FIG. 26 is a chart illustrating color determination results in Experimental Example 1.
FIG. 27 is a chart illustrating the color determination results in Experimental Example 1.
FIG. 28 is a chart illustrating the color determination results in Experimental Example 1.
FIG. 29 is a chart illustrating the color determination results in Experimental Example 1.
FIG. 30 is a chart illustrating colony detection results in Experimental Example 1.
FIG. 31 is a chart illustrating colony detection results based on an image obtained by imaging an assessment plate from a side opposite to the bottom of wells in Experimental Example 1.
FIG. 32 is a chart illustrating foreign matter detection results in Experimental Example 1.
FIG. 33 is a chart illustrating foreign matter detection results based on an image obtained by imaging the assessment plate from a side opposite to the bottom of the wells in Experimental Example 1.
FIG. 34 is a schematic view of an observation device in Experimental Example 2.
FIG. 35 is a view illustrating a spectrum distribution for every section based on an imaging result of an assessment plate of Sample No. 1 in Experimental Example 2.
FIG. 36 is a view illustrating a spectrum distribution for every section based on an imaging result of an assessment plate of Sample No. 2 in Experimental Example 2.
FIG. 37 is a view illustrating a spectrum distribution for every section based on an imaging result of an assessment plate of Sample No. 3 in Experimental Example 2.
FIG. 38 is a view illustrating a spectrum distribution for every section based on an imaging result of an assessment plate of Sample No. 4 in Experimental Example 2.
FIG. 39 is a view illustrating a spectrum distribution for every section based on an imaging result of an assessment plate of Sample No. 5 in Experimental Example 2.
FIG. 40 is a graph illustrating a luminance difference between sections for every wavelength in Experimental Example 2.
FIG. 41 is a cross-sectional view illustrating another example of the plate.

### Description of Embodiments

Hereinafter, an embodiment of the invention will be described with reference to the accompanying drawings. The same reference numeral will be given to the same element, and redundant description will be omitted. Dimension ratios of the drawings do not always match described ratios.

In this embodiment, description will be given of an embodiment of assessing safety of a test substance. Specifically, description will be given of a case where safety of the test substance is assessed by using an Ames test.

First, an overview of the Ames test used in this embodiment will be described. In the Ames test, cells modified so that synthesis of amino acid cannot be performed by genetically manipulating an amino acid synthesis gene are used as a tester. In the Ames test, a test substance is added to the cells. Then, the cells are cultured under constant conditions. When mutation occurs in the amino acid synthesis gene of the cells due to the test substance, the cells can synthesize the amino acid again. Genotoxicity is assessed by confirming whether or not the mutation has occurred with presence or absence of cell proliferation.

In addition, typically, in the Ames test, cytotoxicity assessment of confirming whether or not the test substance has toxicity with respect to cells is performed.

In this embodiment, an assessment system, an image processing device, and an assessment method which are applicable to genotoxicity assessment and cytotoxicity assessment by an Ames test will be described. In description of the embodiment based on the following Ames test, the tester is a cell unless otherwise stated.

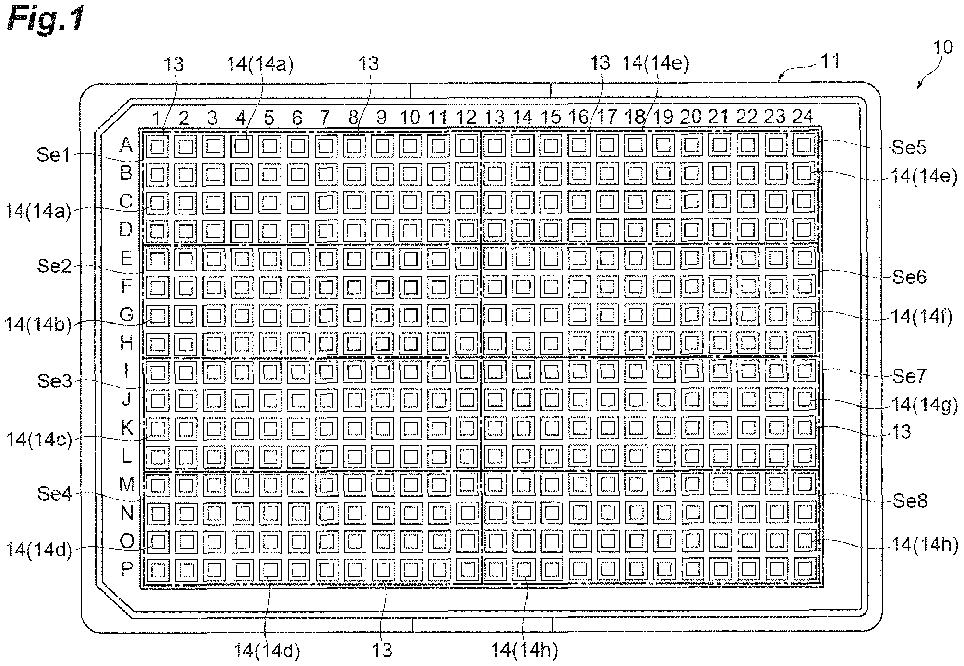

FIG. 1 is a plan view of an assessment plate 10 that is used in the assessment method according to the embodiment. The assessment plate 10 includes a plate 11. The plate 11 includes a plurality of wells 13. In this embodiment, the plate 11 includes 384 wells 13 which are two-dimensionally arranged (16×24).

First to N^{th} sections (N is an integer of two or greater) are virtually set in the plate 11. In this embodiment, description will be given of a case where N is 8, that is, eight sections are virtually set as illustrated in FIG. 1. Hereinafter, the eight sections will be referred to as sections Se1 to Se8. Each of the sections Se1 to Se8 is a region including 48 (4×12) wells 13 (well group). The section Se1 is a solvent control section, and the section Se8 is a positive control section. The solvent control section functions as a negative control section.

The wells 13 are concave portions (recesses) formed in the plate 11. The wells 13 hold an assessment target 14. When assessment targets 14 held in the wells 13 pertaining to each of the sections Se1 to Se8 are distinguished and described, the assessment targets 14 will be referred to as assessment targets 14a to 14h.

The assessment targets 14a to 14h include a culture solution in which cells are dispersed (or suspended). The same amount of culture solution is held in all of the 384 wells 13. The amount of cells in the culture solution held in the wells 13 is the same between the wells 13. Examples of the cells include microorganisms having amino acid-requiring mutation, cells originated from organisms, and the like. Examples of the microorganisms having the amino acid-requiring mutation include histidine-requiring Salmonella typhimurium (for example, TA1535, TA1537, TA97, TA97a, TA98, TA100, TA92, TA94, TA102, TA104, TA1538, TA7001, TA7002, TA7003, TA7004, TA 7005, TA7006, various YG strains, and the like), tryptophan-requiring Escherichia coli (for example, WP2, WP2uvrA, WP2/pKM101, WP2uvrA/pKM101, and the like), other microorganisms, and the like. Examples of the cells originated from organisms include cultured cells originated from mammals, cells originated from other vertebrates, cells originated from insects, and the like. The cells included in the assessment targets 14a to 14h may be cells in which a plurality of kinds of cells are mixed. Examples of the culture solution include a phosphoric acid buffer solution containing a small amount of amino acid.

The assessment targets 14a to 14h further include an indicator. The amount of the indicator is the same in the assessment targets 14a to 14h (that is, the same in all of the wells 13). In this embodiment, the indicator is a pH indicator. Examples of the pH indicator include is Bromocresol purple. In this embodiment, the color of the pH indicator is purple in the case of a negative result (a case where mutation does not occur in cells), and the color is yellow in the case of a positive result (a case where mutation occurs in cells). Accordingly, color candidates in this embodiment include purple and yellow. In this specification, for example, the color candidates are colors which an assessment target is assumed to exhibit before and after a predetermined reaction as in the case of using the indicator (for example, the pH indicator).

The assessment targets 14b to 14g further include a solution (test substance solution) in which a test substance is dissolved or uniformly dispersed in a solvent in addition to the culture solution in which the cells are dispersed, and the indicator. Accordingly, wells 13 in the sections Se2 to Se7 holding the assessment targets 14b to 14g are wells (test substance wells) holding the test substance. The test substance is a substance (for example, a chemical substance) for which safety (in this embodiment, genotoxicity) is to be assessed. Examples of a solvent include water, dimethyl sulfoxide (DMSO), and the like. The assessment targets 14b to 14g are the same as each other except that the concentration of the test substances is different from each other. The amount of the test substance solution in the assessment target 14b of all wells 13 pertaining to the section Se2 is the same in each case. This is also true of the sections Se3 to Se7.

The assessment target 14a includes a solvent control solution in addition to the culture solution containing cells, and the indicator. Accordingly, the wells 13 of the section Se1 holding the assessment target 14a are solvent control wells. The solvent control solution is a solvent that is used in the test substance solution. However, the solvent control solution may be a liquid that have no influence on cells. Examples of the solvent control solution include water and DMSO. In all wells 13 pertaining to the section Se1, the amount of the solvent control solution in the assessment target 14 is the same in each case.

The assessment target 14h includes a positive control solution in addition to the culture solution containing cells, and the indicator. Accordingly, the wells 13 of the section Se8 holding the assessment target 14h are positive control wells. The positive control solution is a solution containing a positive control compound and a solvent. The positive control compound may be a compound known to be positive with respect to cells. Examples of the positive control compound include 9-aminoacridine (9AA), 4-nitroquinoline-N-oxide (4-NQO), 2-aminoanthracene (2-AA), and 2-nitrofluorene (2-NF). The solvent may not be the same as the solvent used in the test substance solution. Examples of the solvent include water, DMSO, and the like. The amount of the positive control solution held in all wells 13 pertaining to the section Se8 that is a positive section is the same in each case.

In the case of investigating an influence due to presence or absence of metabolic activation of the test substance, the assessment targets 14a to 14h further include S9mix. The S9mix is a liquid obtained by adding a coenzyme to an extract of an animal liver. The S9mix can be prepared as a liquid that is adjusted by adding an electron transport system-related cofactor such as NADPH to 9,000×g supernatant (S9) of liver homogenate.

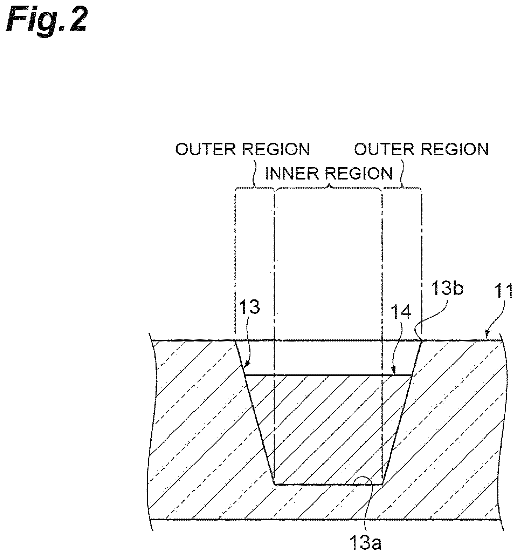

The configuration of the wells 13 will be further described with reference to FIG. 1 and FIG. 2. FIG. 2 is a schematic view of a cross-section of a well. In FIG. 2, the assessment target 14 is schematically illustrated. In this embodiment, a shape of the wells 13 in a plan view (shape when viewed from a depth direction of the wells 13) is a square shape. An example of a length of one side of an opening of the wells 13 is 3.0 to 4.0 mm. An example of the depth of the wells 13 is 9.0 to 15.0 mm. The plate 11 including the wells having the size is known as a microplate. As illustrated in FIG. 2, the wells 13 taper toward the bottom 13a. Accordingly, an area of the bottom 13a is smaller than an area of an opening 13b of the wells 13.

The assessment plate 10 is a plate that enters a state in which safety assessment of the assessment target 14 becomes possible after a corresponding assessment target 14 is injected into each of the wells 13 of the plate 11 and a constant culture treatment is performed.

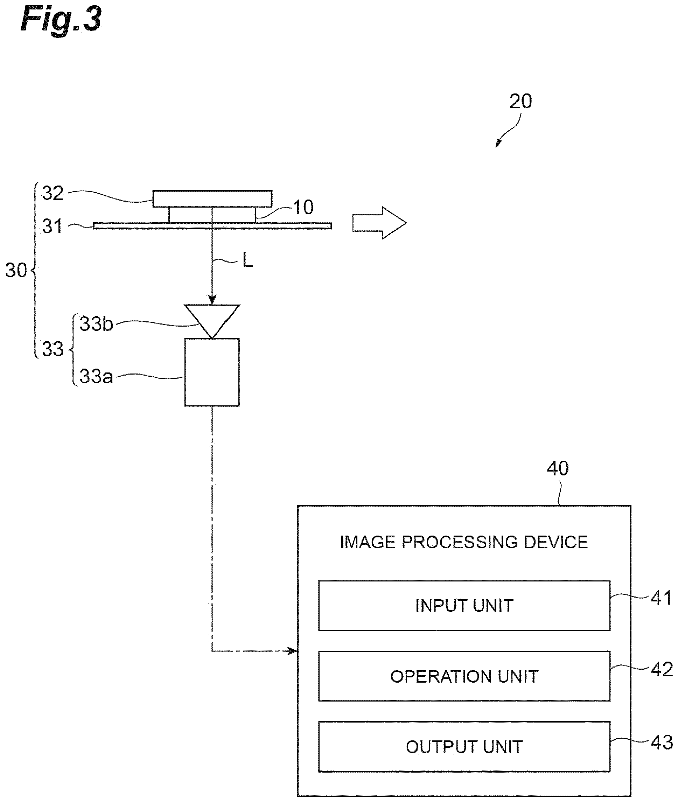

Next, description will be given of an assessment system 20 that is used in a method for assessing safety of the test substance held in the assessment plate 10. FIG. 3 is a schematic view of the assessment system 20 according to an embodiment. The assessment system 20 includes an observation device 30 and an image processing device 40.

### [Observation Device]

The observation device 30 includes a stage (plate support) 31, an illumination device 32, and an imaging unit 33. The stage 31 supports the assessment plate 10. The assessment plate 10 is disposed on the stage 31 in such a manner that the bottom (bottom 13a side of the wells 13) of the assessment plate 10 is located on the stage 31 side. The stage 31 is configured to allow light L output from the illumination device 32 to pass therethrough toward the imaging unit 33. For example, a material of the stage 31 may have optical transparency, openings or windows through which the light L is transmitted may be formed in regions corresponding to regions where the 384 wells 13 are formed in the assessment plate 10. In this embodiment, the stage 31 is configured to be conveyed in a direction of a white blank arrow in FIG. 3.

The illumination device 32 is disposed on the assessment plate 10 (on a side opposite to the stage 31 when viewed from the assessment plate 10). The illumination device 32 irradiates the assessment plate 10 with the light L. Examples of the illumination device 32 include LED lighting, and examples of the light L include light having a wavelength of 400 to 780 nm.

The imaging unit 33 receives the light L transmitted through the assessment plate 10, and images the assessment plate 10. The imaging unit 33 acquires a color image (RGB image) that is substantially the same as a case where the assessment plate 10 is visually observed. The imaging unit 33 includes an imaging device 33a and a condensing unit 33b.

Examples of the imaging device 33a includes a two-dimensional sensor (or camera) and a line sensor. Examples of the two-dimensional sensor include a CCD camera and a CMOS camera.

The condensing unit 33b condenses the light L to be incident to the imaging device 33a. The condensing unit 33b includes at least one lens. In a case where the imaging device 33a has a condensing optical function, the imaging unit 33 may not include the condensing unit 33b.

In this embodiment, an imaging region of the imaging unit 33 is smaller than the size of the assessment plate 10. In this case, the imaging unit 33 images the assessment plate 10 a plurality of times. However, the imaging unit 33 may include a plurality of the imaging units 33, and the imaging region of the imaging unit 33 may be a size capable of imaging the entirety of the assessment plate 10 at a time.

The imaging unit 33 is connected to the image processing device 40 by wire communication or wireless communication, and output an acquired image (specifically, image data) to the image processing device 40. Input of the image from the imaging unit 33 to the image processing device 40 may be performed through a storage medium such as DVD, or the like.

In the observation device 30, positions of the illumination device 32 and the imaging unit 33 with respect to the assessment plate 10 may be inversed from each other. That is, in FIG. 3, in a state in which the imaging unit 33 may be disposed on the assessment plate 10, and the illumination device 32 is disposed on a lower side of the stage 31, the assessment plate 10 may be imaged.

Description will be given of a method for imaging the assessment plate 10 by using the observation device 30. In the case of imaging the assessment plate 10, the assessment plate 10 is disposed on the stage 31. The assessment plate 10 is irradiated with the light L from the illumination device 32 while moving the stage 31 in one direction (irradiation process). In a state in which the assessment plate 10 is irradiated with the light L, the imaging unit 33 images the assessment plate 10 (imaging process).

In this embodiment, since the imaging region of the imaging unit 33 is smaller than the size of the assessment plate 10, a partial image of the assessment plate 10 is obtained in imaging performed once. Accordingly, in the imaging process, the assessment plate 10 is imaged a plurality of times by using the imaging unit 33 so that an image of the entirety of the assessment plate 10 is formed by composing partial images.

The imaging unit 33 inputs the acquired image to the image processing device 40.

### [Image Processing Device]

The image processing device 40 includes an input unit 41, an operation unit 42 (execution unit), and an output unit 43. The image processing device 40 is a computer. Typically, the image processing device 40 may further include a storage unit (memory) provided in the computer, or the like. The image processing device 40 may be a personal computer that functions as the image processing device 40 by executing various programs, or a dedicated device for assessment. The image processing device 40 functions as assessment device that assesses the assessment plate 10 or an assessment support device.

The input unit 41 is an input means to which image data acquired by the imaging unit 33 is input (or which receives the image data).

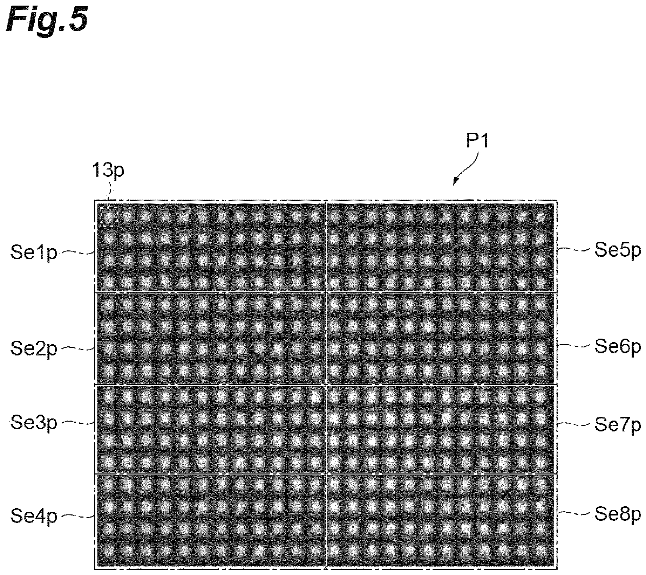

The operation unit 42 creates a plate image P1 (refer to FIG. 5) based on the image input to the input unit 41. FIG. 5 is a view illustrating an example of the plate image P1. The plate image P1 is an image corresponding to the assessment plate 10. In the plate image PI, portions corresponding to the wells 13 and the sections S1 to S8 are referred to as a well image 13p and section images Selp to Se8p. The operation unit 42 performs a color determination process for the wells 13 by processing the plate image P1. The operation unit 42 may further perform at least one among a process of detecting colonies in the wells 13, a process of detecting a transparency change of the wells 13, and a process of detecting foreign matters in the wells 13. In this embodiment, description will be given of a case where the operation unit 42 can perform the colony detection process, the transparency change detection process, and the foreign matter detection process.

In this embodiment, well color means the color of the assessment target 14 held in each of the wells 13. The operation unit 42 determines the color of the wells 13 by determining the color of the well image 13p. In this embodiment, colonies in wells mean colonies as a shape change occurring in cells which are the assessment target 14 held in each of the wells 13. The colonies are shape changes of cells (testers), and thus the colonies are structures in which a contour and an inner boundary are not clear. In this embodiment, transparency change of well means a transparency change of the assessment target 14 held in each of the wells 13, and the operation unit 42 determines the transparency change of the wells 13 by determining the transparency change of the well image 13p. In this embodiment, "foreign matters in wells" are foreign matters occurring in the assessment target 14 held in the wells 13, and are substances (or structures) separated at an interface having a refractive index different from that of the periphery of the foreign matters. Accordingly, a contour and an inner boundary in the foreign matters are clear (clearer in comparison to at least the case of colonies) differently from the shape change of cells (tester) as in the colonies.

For example, in a case where the input unit 41 outputs the plate image P1 to the storage unit, the operation unit 42 may acquire data to be processed by accessing the storage unit. The operation unit 42 will be described later.

The output unit 43 performs display output so that an assessor refers to a result of the operation unit 42. For example, the output unit 43 may output the result of the operation unit 42 to an external display device, or the output unit 43 may have a display function. The output unit 43 may perform data output display to the operation unit 42 during the process.

As described above, in this embodiment, the operation unit 42 can perform the color determination process, the colony detection process, the transparency change detection process, and the foreign matter detection process.

Description will be given of the operation unit 42 in the case of performing each of the processes. Here, description will be given of a case where the imaging region of the imaging unit 33 is smaller than the size of the assessment plate 10, and the imaging unit 33 images different regions of the assessment plate 10 a plurality of times to obtain an image of the entirety of the assessment plate 10.

### [Color Determination Process]

With regard to the color determination process, a first color determination process and a second color determination process will be described. In the color determination, statistical information based on at least one color feature quantity is used. The statistical information includes at least one kind of statistical quantity. In this embodiment, description will be given of a case where hue and saturation are used as two or more color feature quantities, and a frequency distribution (statistical quantity) is used as the statistical information.

### [First Color Determination Process]

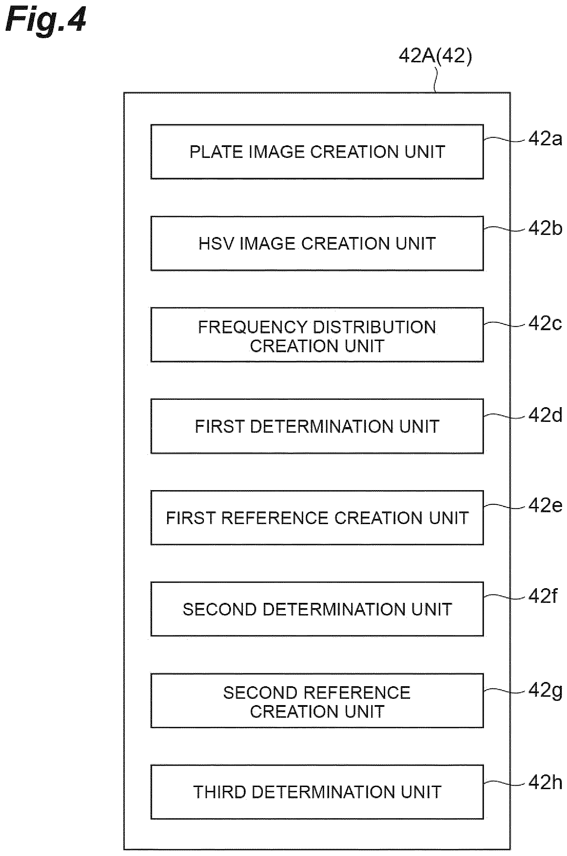

The operation unit 42 in the case of performing the first color determination process will be referred to as an operation unit 42A. FIG. 4 is a functional block diagram of the operation unit 42A. The operation unit 42A includes a plate image creation unit 42a, an HSV image creation unit 42b, a frequency distribution creation unit (statistical information acquisition unit) 42c, a first determination unit 42d, a first reference creation unit (first reference acquisition unit) 42e, a second determination unit 42f, a second reference creation unit (second reference acquisition unit) 42g, and a third determination unit 42h.

The plate image creation unit 42a composes a plurality of images (each image is a partial image of the assessment plate 10) input to the input unit 41, and creates the plate image P1 corresponding to the assessment plate 10 as illustrated in FIG. 5. The plate image P1 is a color image (RGB image).

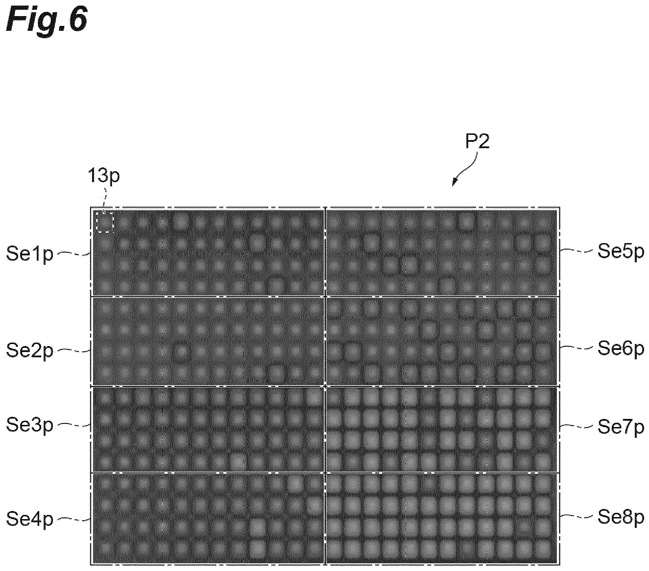

The HSV image creation unit 42b converts the plate image P1 created by the plate image creation unit 42a into an HSV image P2. FIG. 6 is a view illustrating an example of the HSV image P2. The HSV image P2 illustrated in FIG. 6 is an image obtained by HSV-converting the plate image P1 illustrated in FIG. 5. Conversion from the RGB image into the HSV image may be performed by a known conversion formula (or a known program based on the conversion formula). Since the HSV image P2 is an image obtained by HSV-converting the plate image PI, partial images corresponding to the wells 13 and the sections Se1 to Se8 in the HSV image P2 are also referred to as a well image 13p, and section images Selp to Se8p.

The frequency distribution creation unit 42c creates a frequency distribution with hue and saturation as variables (that is, acquires statistical information as a frequency distribution with hue and saturation as variables) for every well image 13p (in other words, for every well 13) by using the HSV image P2 created by the HSV image creation unit 42b. According to this, the frequency distribution creation unit 42c creates a plurality of frequency distributions (in this embodiment 384 frequency distributions) corresponding to all wells 13 provided in the assessment plate 10. An example of a specific method of the frequency distribution by the frequency distribution creation unit 42c will be described.

The frequency distribution creation unit 42c specifies a region of each well image 13p in the HSV image P2. For example, as indicated by a broken line in FIG. 6, a region of interest (ROI) may be set to the well image 13p. In FIG. 6, a state in which the ROI is set to one well image 13p is illustrated for exemplification. The ROI may be set as a region designated by an assessor, or the frequency distribution creation unit 42c may automatically set, for example, on the basis of data (a shape, a size, or the like) of the plate 11, an image of the plate 11 in a state in which nothing is contained in the wells 13, luminance information of the HSV image, or the like, which are input in advance.

The frequency distribution creation unit 42c creates the frequency distribution by using image information (hue, saturation, or the like) of a plurality of pixels included in a specified well image 13p. In this embodiment, the hue and saturation are respectively divided into 36, and 1296 (= 36×36) feature quantities (in this embodiment, the number of pixels) are extracted to create the frequency distribution.

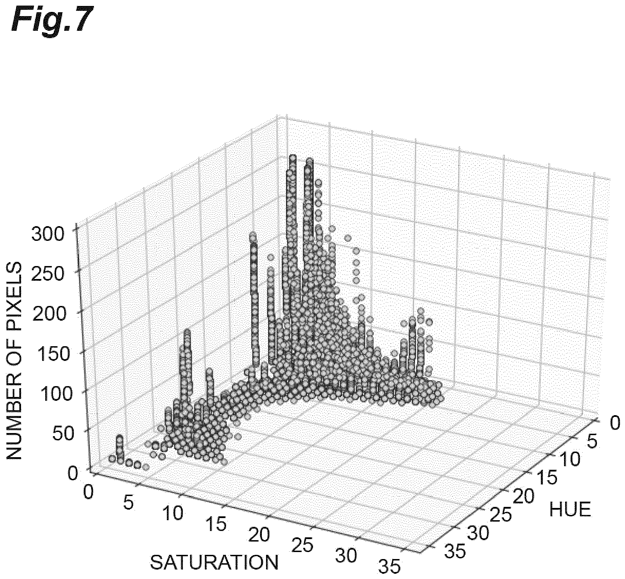

FIG. 7 is a view illustrating a histogram of an example of the created frequency distribution. In FIG. 7, the abscissa represents hue and saturation, and the ordinate represents the number of pixels. FIG. 7 is a histogram for one well image 13p.

First, the first determination unit 42d calculates similarity between a plurality of the frequency distributions created by the frequency distribution creation unit 42c. For example, the similarity may be calculated by using a Euclidean distance or may be calculated by using a correlation coefficient. For example, the similarity may be calculated by using pattern matching. In calculation of the similarity, deep learning may be used.

An example of the case of calculating the similarity between a plurality of frequency distributions will be described. In this embodiment, in order to determine the color of the well image 13p (that is, the color of the wells 13) as purple or yellow, similarity of a purple region (color candidate region) in the respective frequency distributions and similarity of a yellow region (color candidate region) in the respective frequency distributions are calculated. The purple region and the yellow region can be set, for example, by an assessor on the basis of hue and saturation for color determination of the well image 13p. In the example in FIG. 7, for example, a region of hue of 20 to 35 and saturation of 0 to 15 can be set as the purple region, and a region of hue of 0 to 15 and saturation of 0 to 25 can be set as the yellow region.

An arbitrary one of the well images 13p is referred to as an interest well image. In this case, similarity between a frequency distribution of the purple region in a frequency distribution corresponding to the interest well image, and an average frequency distribution of the purple region of all well images 13p in which the sum of the frequency of the purple region is equal to or greater than a constant value is set as similarity of the purple region of the interest well image. Similarly, similarity between a frequency distribution of the yellow region in the interest well image, and an average frequency distribution of the yellow region of all well images 13p in which the sum of the frequency of the yellow region is equal to or greater than a constant value is set as similarity of the yellow region of the interest well image. According to this, since a set of similarities of the purple region and the yellow region is obtained for each well image 13p, and thus similarity distributions for purple and yellow are acquired.

Alternatively, similarity between the frequency distribution corresponding to the interest well image and the average frequency distribution of all well images 13p in which the sum of the frequency of the purple region is equal to or greater than a constant value may be set as the similarity for the purple color of the interest well image. Similarly, similarity between the frequency distribution corresponding to the interest well image and the average frequency distribution of all well images 13p in which the sum of the frequency of the yellow region is equal to or greater than a constant value may be set as the similarity for the purple color of the interest well image. In this case, the similarity distributions for purple and yellow are also acquired.

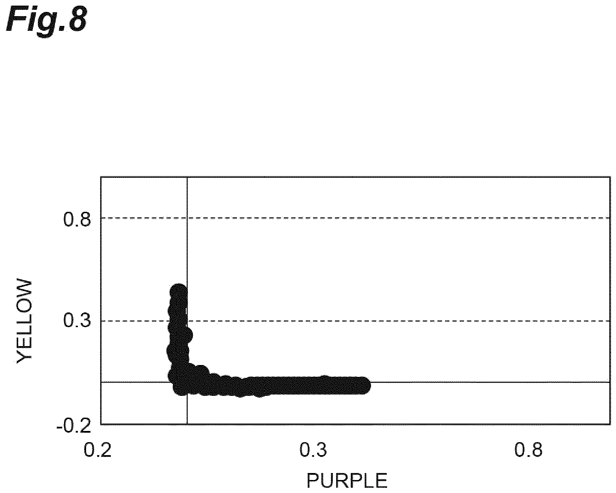

FIG. 8 is a graph of an example of the similarity distributions for purple and yellow. Specifically, FIG. 8 is a graph obtained by mapping a set of the similarity of purple and the similarity of yellow, which are calculated with respect to each of the well images 13p, to the similarity of purple and the similarity of yellow. In FIG. 8, the abscissa represents the similarity of purple, and as a number increases, it is more similar to purple. The ordinate represents similarity of yellow, and as a number increases, it is more similar to yellow.

The first determination unit 42d primarily determines the color of the well image 13p as purple or yellow on the basis of the obtained similarity distributions. For example, the well image 13p may be determined as purple or yellow by using a threshold value for division into purple and yellow. For example, the threshold value may be set by an assessor by presenting the graph illustrated in FIG. 8 to the assessor, or the threshold value may be automatically set by the first determination unit 42d by a hierarchical clustering method such as a Ward method, a non-hierarchical clustering method such as K-means, a linear discrimination method, or the like, in cluster analysis.

The first reference creation unit 42e creates a first reference frequency distribution for each of purple and yellow, on the basis of the frequency distributions of a plurality of well images 13p (frequency distributions created by the frequency distribution creation unit 42c), which are determined as purple and yellow in the first determination unit 42d among all of the well images 13p of the assessment plate 10.

In this embodiment, the first reference frequency distribution of purple is created on the basis of an average frequency distribution obtained by averaging frequency distributions of a plurality of well images 13p determined as purple. Similarly, the first reference frequency distribution of yellow is created on the basis of an average frequency distribution obtained by averaging frequency distributions of a plurality of well images 13p determined as yellow.

The second determination unit 42f secondarily determines all of the well images 13p as purple or yellow on the basis of the first reference frequency distribution for each of purple and yellow which is created by the first reference creation unit 42e, and the frequency distributions of all of the well images 13p of the assessment plate 10.

Specifically, the second determination unit 42f calculates similarity between the first reference frequency distribution for each of purple and yellow and the frequency distributions of all of the well images 13p. Since the first reference frequency distribution for each color is used, as in the example of the method for creating the frequency distribution in the frequency distribution creation unit 42c, similarity may be calculated with respect to all regions without division into the purple region and the yellow region. Since a set of similarity for purple and similarity for yellow is obtained for each of the well images 13p, similarity distributions for purple and yellow are acquired.

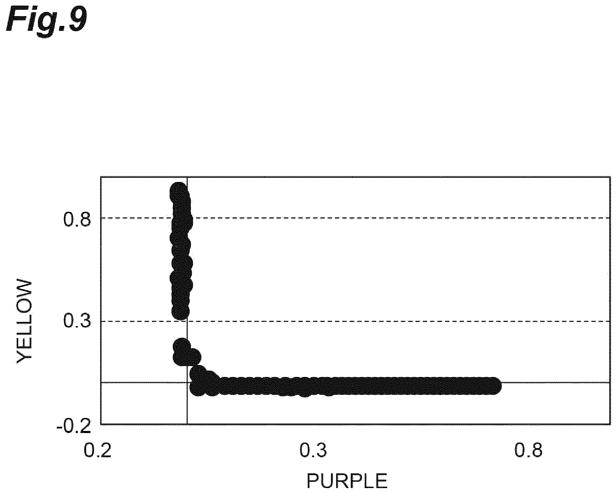

FIG. 9 is a graph of a similarity distribution acquired by the second determination unit 42f. In FIG. 9, the abscissa and the ordinate are similar to the abscissa and the ordinate in FIG. 8.

The second determination unit 42f determines each well image 13p as purple or yellow on the basis of the obtained similarity distributions. For example, the well image 13p may be determined as purple or yellow by using a threshold value for division into purple and yellow. For example, the threshold value may be set by an assessor by presenting the graph illustrated in FIG. 9 to the assessor, or the threshold value may be automatically set by the second determination unit 42f by a hierarchical clustering method such as a Ward method, a non-hierarchical clustering method such as K-means, a linear discrimination method, or the like, in cluster analysis.

The second reference creation unit 42g creates a second frequency distribution for each of purple and yellow on the basis of the frequency distributions of a plurality of well images 13p (frequency distributions created by the frequency distribution creation unit 42c) which are determined as purple and yellow in the second determination unit 42f for each of the section images Selp to Se8p which are images corresponding to the sections Se1 to Se8 of the assessment plate 10.

Specifically, in a case where an arbitrary one of the section images Selp to Se8p is referred to as an interest section image, the second reference frequency distribution of purple is created on the basis of an average frequency distribution obtained by averaging frequency distributions of a plurality of well images 13p determined as purple among a plurality of well images 13p (in this embodiment, 48 well images 13p) pertaining to the interest section image. Similarly, the second reference frequency distribution of yellow is created on the basis of an average frequency distribution obtained by averaging frequency distributions of a plurality of well images 13p determined as yellow in the interest section image. The interest section image corresponds to an i^{th} section image corresponding to an i^{th} section (i is an integer of two or greater) among first to N^{th} section images which are images corresponding to first to N^{th} sections in a case where the plurality of sections set in the plate 11 are referred to as first to N^{th} section as described above.

The third determination unit 42h tertiarily determines the color of a plurality of well images 13p pertaining to each of the section images Selp to Se8p on the basis of the second reference frequency distribution for each of purple and yellow which is created by the second reference creation unit 42g, and frequency distribution of the plurality of well images 13p pertaining to each of the section images Selp to Se8p. According to this, the color of all of the wells 13 in the assessment plate 10 is tertiarily determined. In this embodiment, a color determination result by the third determination unit 42h is a final determination result.

As described above, the determination method by the third determination unit 42h in which an arbitrary one of the section images Selp to Se8p is referred to as the interest section image will be described in detail.

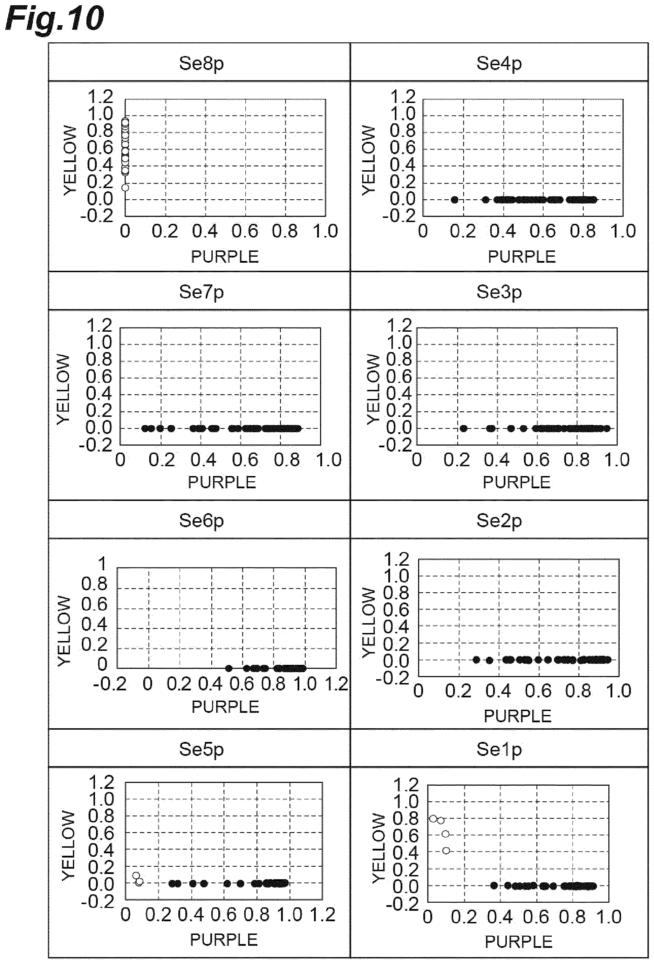

First, a similarity distribution in the well images 13p is acquired in the same manner as in the case of the second determination unit 42f except that the frequency distributions of all well images 13p in the interest section image are used instead of the frequency distributions of all well images 13p in the second determination unit 42f, and the second frequency distribution is used instead of the first reference frequency distribution. FIG. 10 is a graph of a similarity distribution for each of the section images Selp to Se8p. Selp to Se8p in FIG. 10 represent section images Selp to Se8p. The abscissa and the ordinate in FIG. 10 are similar to the abscissa and the ordinate in FIG. 8. A white circle in FIG. 10 corresponds to a well image 13p determined as yellow, and a black circle corresponds to a well image 13p determined as purple.

The third determination unit 42h determines the color of all of the well images 13p in the section images Selp to Se8p on the basis of the obtained similarity distributions. For example, the color of the well images 13p may be determined by using a threshold value for division into purple and yellow. For example, the threshold value may be set by an assessor by presenting the graph illustrated in FIG. 10 to the assessor, or the threshold value may be automatically set by the third determination unit 42h by a hierarchical clustering method such as a Ward method in cluster analysis, a non-hierarchical clustering method such as K-means, a linear discrimination method, or the like. As described above, a determination result of the third determination unit 42h is a final determination result in this embodiment. Accordingly, in determination by the third determination unit 42h, for example, in the case of using the threshold value, the color of a well image 13p (or a well 13) located in a region on the threshold value or near the threshold value may be determined as an intermediate color between purple and yellow.

From the viewpoint that the color of all well images 13p in the interest section image is determined, the color of all of the wells 13 in the assessment plate 10 is determined.

The third determination unit 42h may create data representing a determination result. Specifically, the third determination unit 42h may create output image data in which each well image 13p (or a well 13) is expressed by a color corresponding to the determined color (purple or yellow). In this case, the intermediate color may be expressed, for example, as green.

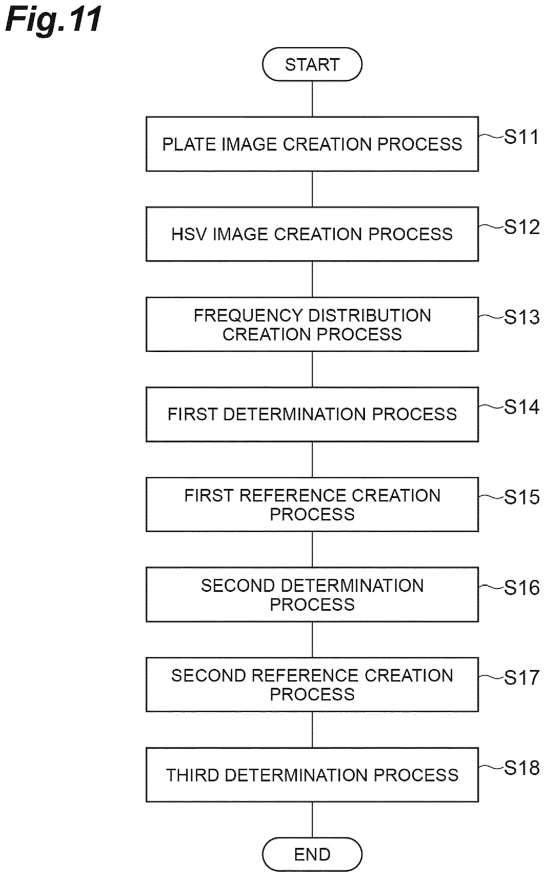

An example of the method for assessing the assessment plate 10 (method for assessing a test substance) by using the first color determination process will be described. FIG. 11 is an example of a flowchart of an assessment method using the first color determination process. In the case of performing the assessment method using the first color determination process, the operation unit 42 functions as the operation unit 42A. Here, a process subsequent to the process (input process) in which the image acquired by the imaging unit 33 of the observation device 30 is received by the input unit 41 will be described.

When the image transmitted from the imaging unit 33 is received by the input unit 41, the image processing device 40 composes a plurality of images, which are transmitted from the imaging unit 33 and are received by the input unit 41, by the plate image creation unit 42a to generate a plate image P1 (plate image creation process S11).

After the plate image creation process S11, the image processing device 40 converts the plate image P1 into an HSV image P2 by the HSV image creation unit 42b (HSV image creation process S12).

After the HSV image creation process S12, the image processing device 40 creates a frequency distribution for each well image 13p by the frequency distribution creation unit 42c (frequency distribution creation process S13). In the frequency distribution creation process (statistical information acquisition process) S13, a plurality of frequency distributions corresponding to all well images 13p in the plate image P1 are created.

After the frequency distribution creation process S13, the image processing device 40 calculates similarity between the plurality of frequency distributions created in the frequency distribution creation process S13 by the first determination unit 42d, and primarily determines whether or not the color of all of the well images 13p is purple or yellow (first determination process S14). A method for calculating the similarity is as described above. When calculating the similarity between the plurality of frequency distributions, similarity distributions for purple and yellow are acquired as described above. In the acquired similarity distributions, the color of all of the well images 13p is determined as purple or yellow by using a threshold value for division into purple and yellow.

After the first determination process S14, the image processing device 40 creates a first reference frequency distribution (first reference statistical information) for each of purple and yellow by the first reference creation unit 42e (first reference creation process S15). A method for creating the first reference frequency distribution is similar to the case described with regard to the first reference creation unit 42e.

After the first reference creation process (first reference statistical information acquisition process) S15, the image processing device 40 secondarily determines the color of the plurality of well images 13p by the second determination unit 42f by using the first reference frequency distribution (second determination process S16). In this embodiment, similarity between each of the first reference frequency distribution for purple and yellow and the frequency distribution of all of the well images 13p is calculated. In the second determination process S16, the similarity may be calculated for the entire range of hue and saturation in the first reference frequency distribution. According to this, since a set of similarities with respect to purple and yellow is obtained for each of the well images 13p, similarity distributions with respect to purple and yellow are acquired. In the similarity distributions obtained in this manner, the color of all the well images 13p is secondarily determined to be purple or yellow by using a threshold value for division into purple and yellow. The threshold value used in the second determination process S16 may be set by an assessor or may be automatically set by the second determination unit 42f as described above.

After the second determination process S16, the image processing device 40 creates a second reference frequency distribution of each of purple and yellow for each of the section images Selp to Se8p by the second reference creation unit 42g (second reference creation process S17). In this embodiment, in each of the section images Selp to Se8p, a second reference frequency distribution (second reference statistical information) of purple is created on the basis of an average frequency distribution obtained by averaging frequency distributions of a plurality of well images 13p determined as purple in the second determination process among a plurality of well images 13p (in this embodiment, 48 well images 13p) pertaining to each of the section images Selp to Se8p. A second reference frequency distribution (second reference statistical information) of yellow is created in a similar manner.

After the second reference creation process (second reference statistical information acquisition process) S17, the image processing device 40 tertiarily determines (finally determines) the color of the well images 13p for each of the section images Selp to Se8p by the third determination unit 42h (third determination process S18). The third determination process S18 in this embodiment will be described in detail.

First, in each of the section images Selp to Se8p, a similarity distribution is acquired with respect to a plurality of well images 13p pertaining to each of the section images Selp to Se8p in a similar manner as in the second determination process S16. The color of all well images 13p pertaining to each of the section images Selp to Se8p is tertiarily determined by using a threshold value for division into purple and yellow on the basis of the acquired similarity distribution. The threshold value may be set by an assessor, or may be automatically set by the third determination unit 42h. In the case of using the threshold value, the color of a well image 13p located in a region on the threshold value or near the threshold value is determined as an intermediate color between purple and yellow.

Since the color of a plurality of well images 13p is determined for each of the section images Selp to Se8p, the color of all of the wells 13p in the assessment plate 10 is determined.

After the third determination process S18, the image processing device 40 may output a result of the third determination process S18 by the output unit 43 to be presented to the assessor (output process).

### [Second Color Determination Process]

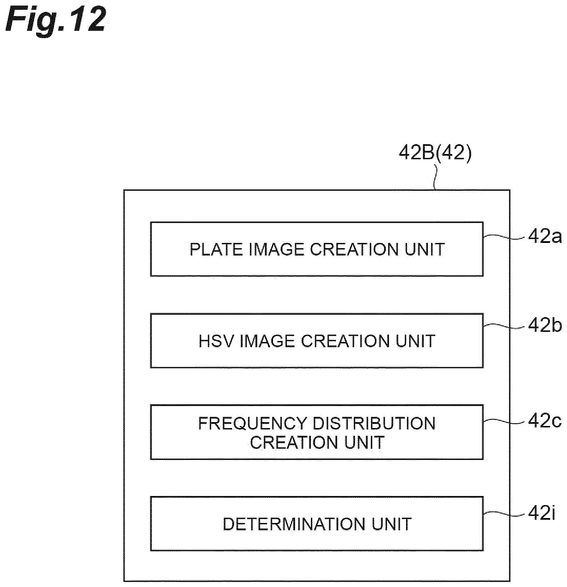

The operation unit 42 in the case of performing a second color determination process will be referred to as an operation unit 42B. FIG. 12 is a functional block diagram of the operation unit 42B. The operation unit 42 includes a plate image creation unit 42a, an HSV image creation unit 42b, a frequency distribution creation unit 42c, and a determination unit 42i. The plate image creation unit 42a, the HSV image creation unit 42b, and the frequency distribution creation unit 42c are similar as in the case of the operation unit 42A, and thus description thereof will be omitted.

The determination unit 42i determines the color of a well image 13p by using a third reference frequency distribution (reference statistical information of a color candidate) with respect to purple and yellow which is stored in the image processing device 40 in advance. In this embodiment, the third reference frequency distribution used in the determination unit 42i has been acquired for each of the section images Selp to Se8p in a similar manner as in the second reference frequency distribution. A method for creating the third reference frequency distribution will be described later. In this embodiment, the determination unit 42i determines the color of the well image 13p for each of the section images Selp to Se8p in a similar manner as in the case of the third determination unit 42h in the operation unit 42 except that the third reference frequency distribution is used instead of the second reference frequency distribution. According to this, the color of all of the wells 13 in the assessment plate 10 is determined.

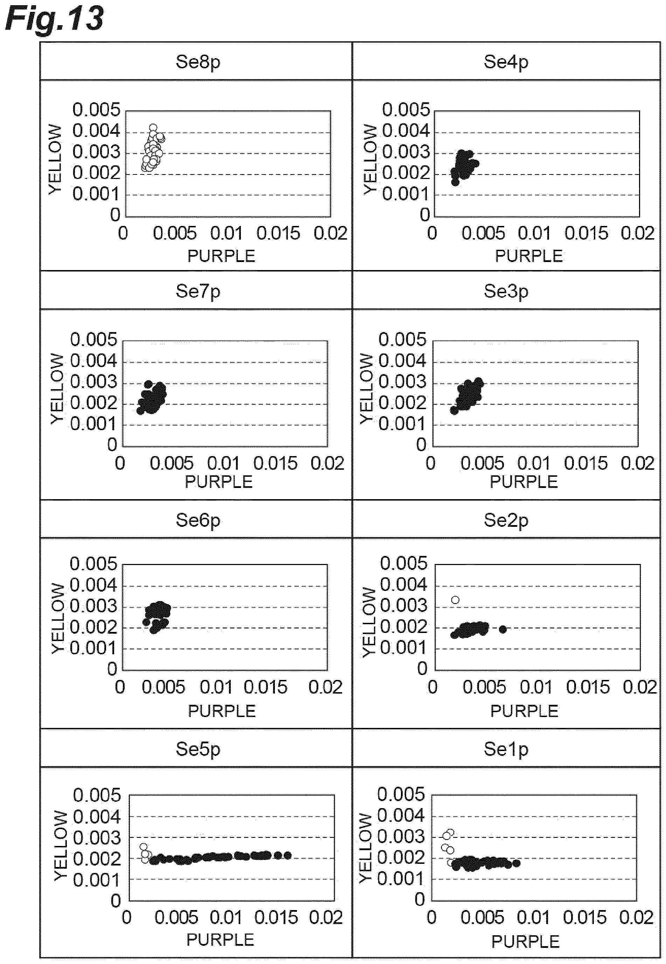

FIG. 13 is a graph of a similarity distribution acquired by the determination unit 42i for each of the section images Selp to Se8p. The abscissa and the ordinate in FIG. 13 are similar to the abscissa and the ordinate in FIG. 10. FIG. 13 illustrates a result in the case of using the same plate image P1 as in the case of FIG. 10. Selp to Se8p in FIG. 13 represent the section images Selp to Se8p.

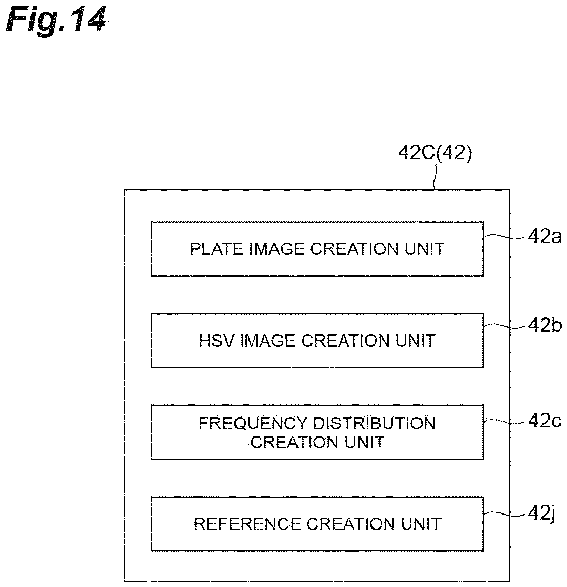

A method for creating the third reference frequency distribution will be described. In the case of creating the third reference frequency distribution, the operation unit 42 is referred to as an operation unit 42C. FIG. 14 is a functional block diagram of the operation unit 42C. The operation unit 42C includes a plate image creation unit 42a, an HSV image creation unit 42b, a frequency distribution creation unit 42c, and a reference creation unit (reference statistical information acquisition unit) 42j.

Here, description will be given of a case where images of three assessment plates 10 prepared under the same conditions are acquired by the observation device 30, the images are input to the image processing device 40 through the input unit 41, and a visual determination result of the three assessment plates 10 by an assessor is input to the image processing device 40 in order to create the third reference frequency distribution. With regard to at least cells, an indicator, a culture solution, and S9mix in the plate under the same conditions, it is preferable to use the same things as those used in actual assessment for a test substance.

The plate image creation unit 42a creates a plate image P1 of three assessment plates 10 in a similar manner as in the case of the plate image creation unit 42a in the operation unit 42A.

The HSV image creation unit 42b converts each of the three plate images P1 into an HSV image P2 in a similar manner as in the case of the HSV image creation unit 42b in the operation unit 42A.

The frequency distribution creation unit 42c creates frequency distributions for each well image 13p with respect to each of the three HSV images P2 created by the HSV image creation unit 42b in a similar manner as in the frequency distribution creation unit 42c in the operation unit 42A.

The reference creation unit 42j creates a third reference frequency distributions for purple and yellow for each of the section images Selp to Se8p on the basis of the frequency distribution created by the frequency distribution creation unit 42c and visual determination result of an assessor. As described above, description will be given by referring to an arbitrary one of the section images Selp to Se8p as an interest section image.

All wells 13 pertaining to the interest section in the three assessment plates 10 are divided into purple and yellow on the basis of the visual determination result. The reference creation unit 42j creates the third reference frequency distribution of purple by creating an average frequency distribution of frequency distributions of all of the well images 13p divided into purple. Similarly, the third reference frequency distribution of yellow is created by creating an average frequency distribution of frequency distributions of all of the wells 13p divided into yellow.

Here, description will be given of the case of using the three assessment plates 10 under the same conditions, but the number of the assessment plates 10 is not limited. The larger the number of assessment plates 10, the more accurately the determination can be made. The third reference frequency distribution may be created once. Alternatively, for example, the second reference frequency distribution created in the past in the first color determination method may be used as the third reference frequency distribution in the second color determination process.

The third reference frequency distribution may be created with respect to the entirety of the assessment plate 10 in a similar manner as in the case of the first reference frequency distribution.

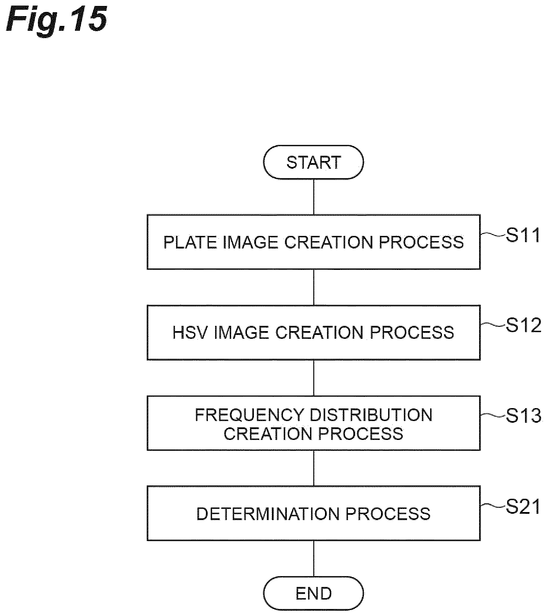

Next, an example of a method for assessing the assessment plate 10 (method for assessing a test substance) by using the second color determination process will be described. FIG. 15 is an example of a flowchart of the assessment method using the second color determination process. In the case of performing the assessment method using the second color determination process, the operation unit 42 functions as the operation unit 42B.

Here, a process subsequent to the process (input process) in which the image acquired by the imaging unit 33 of the observation device 30 is received by the input unit 41 will be described. Description will be given of a case where the third reference frequency distribution of each of purple and yellow has been created in advance by the reference creation unit 42j illustrated in FIG. 14, and has been stored in the image processing device 40.

In a similar manner as in the case illustrated in FIG. 11, a plate image creation process S11, an HSV image creation process S12, and a frequency distribution creation process S13 are performed.

After the frequency distribution creation process S13, the image processing device 40 determines the color of all wells 13p by the determination unit 42i (determination process S21). In this embodiment, the color of the well image 13p is determined by using the third reference frequency distribution stored in the image processing device 40 in advance for each of purple and yellow. In this embodiment, the third reference frequency distribution has been acquired for each of the section images Selp to Se8p in a similar manner as in the second reference frequency distribution described in the second reference creation process S17 illustrated in FIG. 11. In this embodiment, in the determination process S21, the color of all of the wells 13 in the assessment plate 10 is determined by determining the color of the well image 13p for each of the section images Selp to Se8p in a similar manner as in the case of the third determination process S18 in FIG. 11 except that the third reference frequency distribution is used instead of the second reference frequency distribution.

After the determination process S21, the image processing device 40 may output a result of the determination process S21 by the output unit 43 to be presented to the assessor (output process).

### [Colony Detection Process]

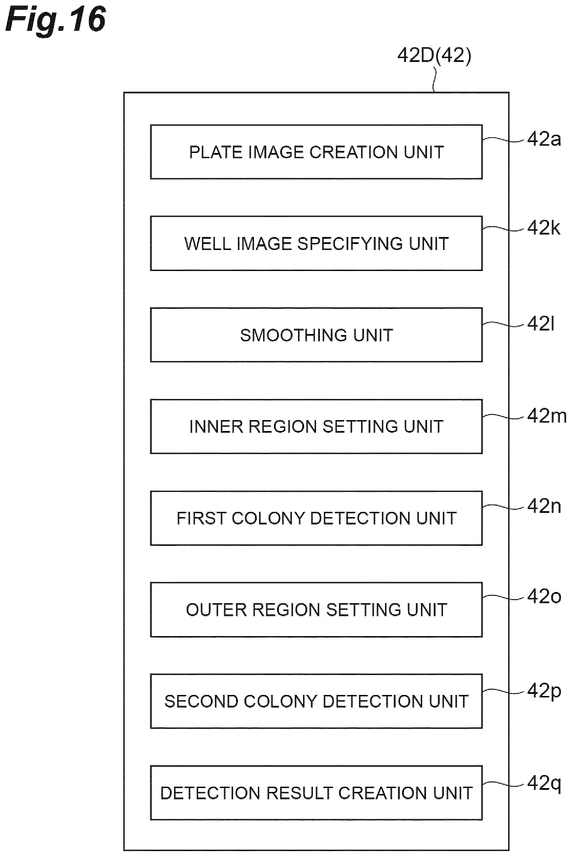

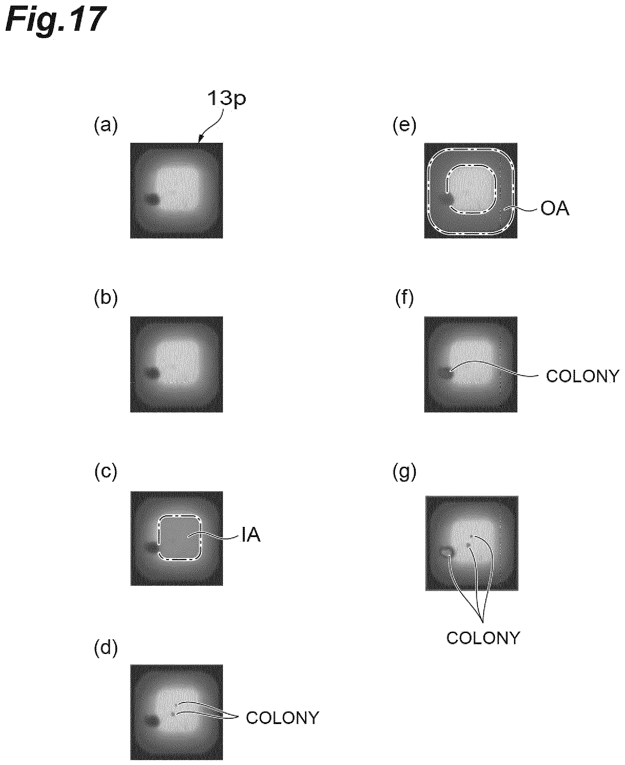

The operation unit 42 in the case of performing the colony detection process is referred to as an operation unit 42D, and the operation unit 42D will be described with reference to FIG. 16 and FIG. 17. FIG. 16 is a functional block diagram of the operation unit 42D. FIG. 17 is a view for describing the colony detection process performed by the operation unit 42D. The operation unit 42 includes a plate image creation unit 42a, a well image specifying unit 42k, a smoothing unit 421, an inner region setting unit 42m, a first colony detection unit 42n, an outer region setting unit 42o, a second colony detection unit 42p, and a detection result creation unit 42q.

The plate image creation unit 42a creates a plate image P1 on the basis of the image of the assessment plate 10 which is input to the image processing device 40 in a similar manner as in the plate image creation unit 42a in the operation unit 42A.

The well image specifying unit 42k specifies a well image (assessment region) 13p that is a region corresponding to a well 13 in the plate image P1. Specifically, a region of interest (ROI) may be set in the well image 13p. In the (a) of FIG. 17, one well image 13p in which the ROI is set for exemplification is illustrated.

The smoothing unit 421 smooths the well image 13p. The smoothing may be performed in a known method in image processing. The (b) of FIG. 17 illustrates a smoothing result of the well image 13p in the (a) of FIG. 17.

The inner region setting unit 42m specifies an inner region IA of the well image 13p. For example, as indicated by a one-dot chain line in the (c) of FIG. 17, the ROI may be set in the inner region IA. The (c) of FIG. 17 is a view illustrating a state in which the ROI is set in the inner region IA with respect to the image in the (b) of FIG. 17. The ROI setting region may be designated by the assessor, or may be automatically set by the inner region setting unit 42m on the basis of information (dimensions, an arrangement state of the well 13, or the like) of the plate 11 that is used as the assessment plate 10, or luminance information of the well image 13p. The inner region IA of the well image 13p is a region corresponding to the bottom 13a of the well 13 when the assessment plate 10 is viewed from a thickness direction thereof. In other words, the inner region IA means a region immediately above the bottom 13a of the well 13 (refer to FIG. 2).

The first colony detection unit 42n detects presence or absence of a colony within the inner region IA by specifying a shape change (a shape change due to a colony) of the assessment target 14 in the inner region IA. In this embodiment, in a case where a colony is formed, it is detected due to the fact that the colony is darker than the periphery.

Specifically, the specified inner region IA is binarized on the basis of a luminance threshold value, and regions in which a luminance value is smaller (darker) than the set luminance threshold value are detected as colony candidates. The luminance threshold value may be designated by the assessor in correspondence with brightness (luminance value) of the inner region IA, or may be set in advance on the basis of past data or the like. In this embodiment, one of the colony candidates that further satisfies a shape (circularity) and a size which are assumed as a colony is detected as the colony. For example, detection of the colony from the colony candidates on the basis of the shape and the size may be performed by the assessor viewing an image presenting the colony candidates, or the first colony detection unit 42n may make a determination on the basis of the shape (circularity) and the size which are set in advance.

In the section Se1 that is the solvent control section, no colony is actually detected for the well 13 determined as purple by the color determination. Accordingly, with regard to the size, for example, in a case where the color determination process is performed in advance and colony candidates are specified in the well image 13p determined as purple in the color determination of the section image Selp (solvent control region) corresponding to the section Se1, in the section images Se2p to Se8p, a size of a largest colony candidate among the colony candidates in the section image Selp may be set as a reference. That is, in the section images Se2p to Se8p, a colony candidate that is larger than the maximum colony candidate in the section image Selp, and satisfies the shape assumed as the colony may be detected as the colony.

The (d) of FIG. 17 is a view illustrating an example of a colony detection result of the inner region IA.

The outer region setting unit 42o specifies an outer region OA of the well image 13p. For example, as indicated by a one-dot chain line in the (e) of FIG. 17, the ROI may be set in the outer region OA. The (e) of FIG. 17 is a view illustrating a state in which the ROI is set in the outer region OA. The ROI setting region may be designated by the assessor, or may be automatically set by the outer region setting unit 42o on the basis of data of the plate 11, or the like. The outer region OA of the well image 13p means a region of a wall portion of the well 13 (refer to FIG. 2).

The second colony detection unit 42p detects presence or absence of a colony in the outer region OA by specifying a shape change (a change due to the colony) of the assessment target 14 in the outer region OA. In this embodiment, in a similar manner as in the case of the first colony detection unit 42n, in a case where the colony is formed, it is detected due to the fact that an inner side of the colony is darker and the periphery thereof is bright.

Specifically, the specified outer region OA is binarized on the basis of a luminance threshold value, and regions in which a luminance value is smaller (darker) than the set luminance threshold value are detected as colony candidates. The luminance threshold value may be designated by the assessor in correspondence with brightness (luminance value) of the outer region OA, or may be set in advance on the basis of past data or the like. Since the outer region OA is darker than the inner region IA, in the outer region OA, the luminance threshold value for detecting the colony is different from the case of the inner region IA. In this embodiment, in the colony candidates, a candidate that further satisfies the shape (circularity) and the size which are assumed as a colony is detected as the colony in a similar manner as in the case of the first colony detection unit 42n. Conditions such as the shape and the size may be different from the case of the inner region IA. In addition, with regard to the size, a result of the section image Selp corresponding to the section Se1 that is the solvent control section may be used in a similar manner as in the case of the first colony detection unit 42n.

The (f) of FIG. 17 is a view illustrating an example of a colony detection result of the outer region OA.

The detection result creation unit 42q integrates the results in the first colony detection unit 42n and the second colony detection unit 42p to create data representing a colony detection result. For example, the detection result creation unit 42q can create data in which regions of the colonies detected by the first colony detection unit 42n and the second colony detection unit 42p is emphasized (for example, colored).

The operation unit 42D outputs a detection result created by the detection result creation unit 42q to the output unit 43 as a final detection result of the colony. The (g) of FIG. 17 is a view illustrating an example of the final detection result obtained by the operation unit 42.

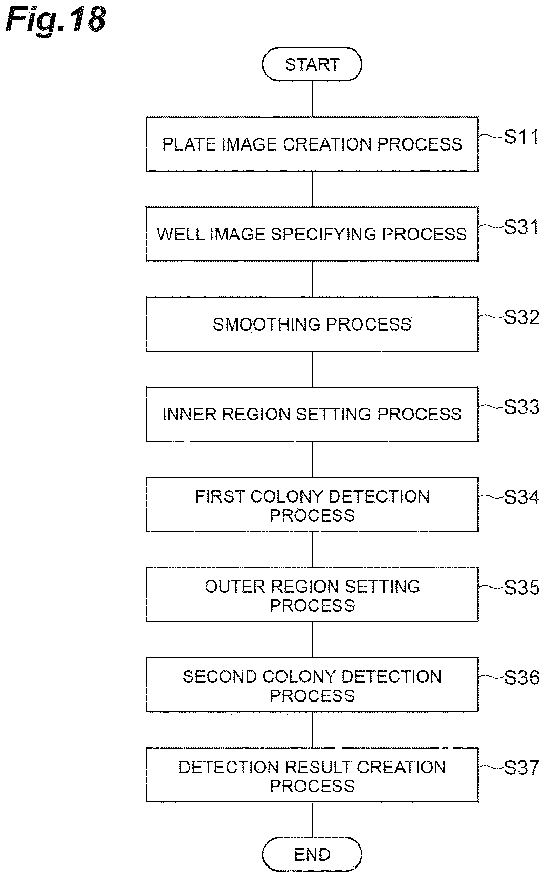

Next, an example of a method for assessing the assessment plate 10 (method for assessing a test substance) by using a colony detection process will be described. FIG. 18 is an example of a flowchart of the method for assessing the assessment plate 10 by using the colony detection process. In the case of performing the method for assessing the assessment plate 10 by using the colony detection process, the operation unit 42 functions as the operation unit 42D. Here, a process subsequent to the process (input process) in which the image acquired by the imaging unit 33 of the observation device 30 is received by the input unit 41 will be described.

In a similar manner as in the case illustrated in FIG. 11, the plate image creation process S11 is performed.

After the plate image creation process S11, the image processing device 40 specifies each well image 13p in the plate image P1 by the well image specifying unit 42k (well image specifying process S31). In this embodiment, the ROI is set in each well image 13p in the plate image. The ROI may be designated by the assessor, or may be automatically set by the well image specifying unit 42k.

After the well image specifying process S31, the image processing device 40 smooths the well image 13p by the smoothing unit 421 (smoothing process S32).

After the smoothing process S32, the image processing device 40 sets an inner region IA of each well image 13p by the inner region setting unit 42m (inner region setting process S33). In this embodiment, the ROI is set in the inner region IA of the well image 13p. The ROI may be designated by the assessor, or may be automatically set by the inner region setting unit 42m.

After the inner region setting process S33, the image processing device 40 detects presence or absence of a colony in the inner region IA of the well image 13p by the first colony detection unit 42n (first colony detection process S34). In this embodiment, the presence or absence of the colony is detected by using a shape (circularity) and a size in combination with a luminance value as described with regard to the first colony detection unit 42n.

After the first colony detection process S34, the image processing device 40 sets an outer region OA of the well image 13p by the outer region setting unit 42o (outer region setting process S35). In this embodiment, the ROI is set in the outer region OA of the well image 13p. The ROI may be designated by the assessor, or may be automatically set by the outer region setting unit 42o.

After the outer region setting process S35, the image processing device 40 detects presence or absence of a colony in the outer region OA of the well image 13p by the second colony detection unit 42p (second colony detection process S36). In this embodiment, the presence or absence of the colony is detected by using a shape (circularity) and a size in combination with a luminance value as described with regard to the second colony detection unit 42p.

After the second colony detection process S36, the image processing device 40 integrates detection results in the first colony detection process S34 and the second colony detection process S36 by the detection result creation unit 42q to create a final detection result in colony detection (detection result creation process S37).

After the detection result creation process S37, the image processing device 40 may output the detection result created in the detection result creation process S37 by the output unit 43 to be presented to the assessor (output process).

### [Transparency change Detection Process]

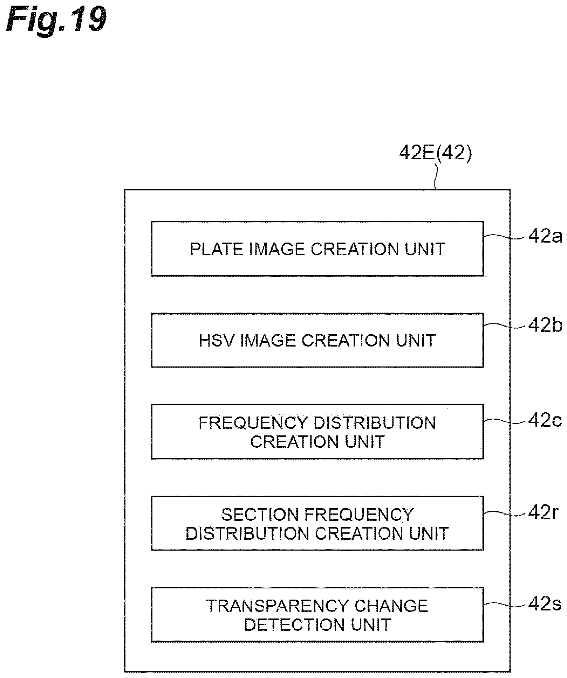

The operation unit 42 in the case of performing the transparency change detection process will be referred to as an operation unit 42E, and the operation unit 42E will be described. FIG. 19 is a functional block diagram of the operation unit 42E. The operation unit 42E includes a plate image creation unit 42a, an HSV image creation unit 42b, a frequency distribution creation unit 42c, a section frequency distribution creation unit 42r, and a transparency change detection unit 42s. The plate image creation unit 42a, the HSV image creation unit 42b, and the frequency distribution creation unit 42c are similar to the case of the operation unit 42A, and description thereof will be omitted.

The section frequency distribution creation unit 42r creates a frequency distribution (hereinafter, referred to as "section frequency distribution") having hue and saturation as variables for each of the section images Selp to Se8p. In a case where an arbitrary one of the section images Selp to Se8p is referred to as an interest section image as described above, the section frequency distribution creation unit 42r creates a section frequency distribution of the interest section image on the basis of an average frequency distribution of frequency distributions of all well images 13p pertaining to the interest section image. In addition, in a case where a color determination process has been performed already, the section frequency distribution of the interest section image may be created on the basis of an average frequency distribution of frequency distributions of, for example, only well images 13p determined as purple (for example, only well images 13p determined as purple in the third determination process S18 (refer to FIG. 11) in the first color determination process) among all well images 13p pertaining to the interest section image.

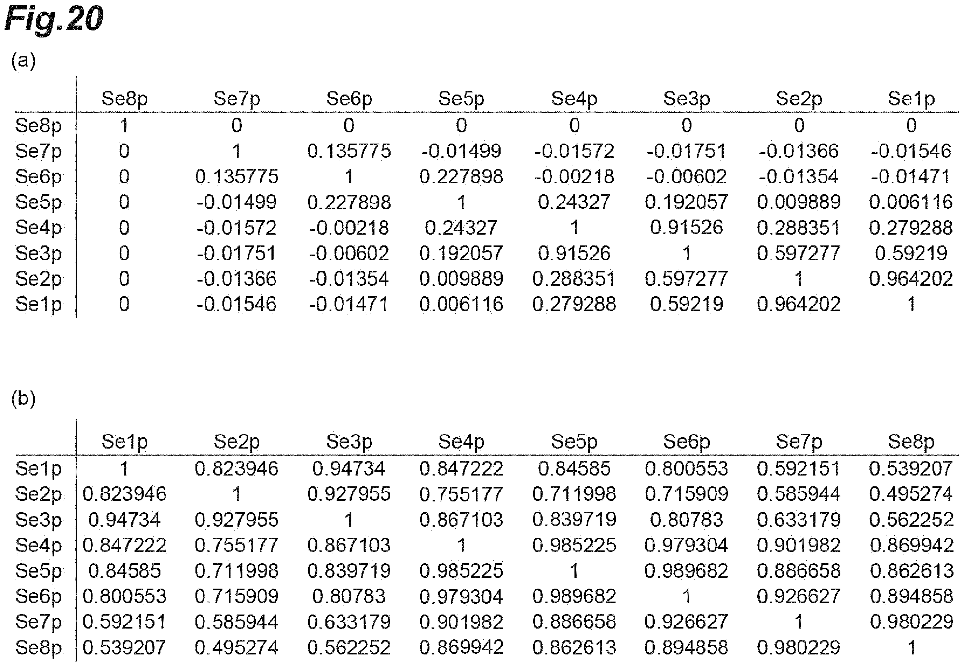

The transparency change detection unit 42s calculates similarity between section frequency distributions corresponding to the section images Selp to Se8p. The transparency change detection unit 42s sets transparency of the section image Selp corresponding to the section Se1 that is the solvent control section as a reference frequency distribution for transparency change detection (reference statistical information for transparency change detection), and detects a transparency change of other section images Se2p to Se8p. Specifically, the similarity between the section frequency distribution of the section image Selp and the section frequency distributions of the other section images Selp to Se8p is calculated. The similarity corresponds to the transparency change of the other sections Se2 to Se8p with respect to the transparency of the section Se1. FIG. 20 is a chart illustrating the similarity of the section frequency distribution between the section images Selp to Se8p, and the (a) and (b) illustrate different examples. The (a) of FIG. 20 illustrates an example in a case where the transparency change is large, and the (b) of FIG. 20 illustrates an example in a case where the transparency change is small to an extent in which the transparency change is not substantially recognized. Selp to Se8p in the chart respectively represent the section images Selp to Se8p. In FIG. 20, a row and a column of the Selp are similarity (transparency change) between the transparency of the section image Selp and the other section images Se2p to S8p, and the section image Selp itself.

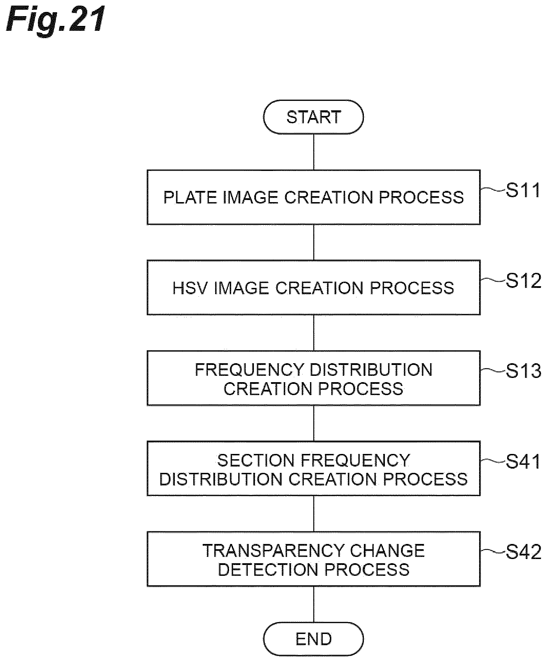

Next, an example of a method for assessing the assessment plate 10 (a method for assessing a test substance) by using the transparency change detection process will be described. FIG. 21 is an example of a flowchart of the assessment method using the transparency change detection process. In the case of performing the assessment method using the transparency change detection process, the operation unit 42 functions as an operation unit 42E. Here, a process subsequent to the process (input process) in which the image acquired by the imaging unit 33 of the observation device 30 is received by the input unit 41 will be described.

In a similar manner as in the case illustrated in FIG. 11, the plate image creation process S11, the HSV image creation process S12, and the frequency distribution creation process S13 are performed.

After the frequency distribution creation process S13, the image processing device 40 creates a section frequency distribution for each of the section images Selp to Se8p by the section frequency distribution creation unit 42r (section frequency distribution creation process S41). In this embodiment, in a case where an arbitrary one of the section images Selp to Se8p is referred to as an interest section image as described above, in the section frequency distribution process S41, a section frequency distribution of the interest section image is created on the basis of an average frequency distribution of frequency distributions of all wells 13 pertaining to the interest section image. As described above, in a case where the color determination process has been performed already, the section frequency distribution of the interest section image may be created on the basis of an average frequency distribution of frequency distributions of, for example, only well images 13p determined as purple among all well images 13p pertaining to the interest section image.

After the section frequency distribution creation process S41, the image processing device 40 detects transparency change in each of other section images Se2p to Se8p with respect to the section image Selp (solvent control region) by the transparency change detection unit 42s (transparency change detection process S42). In this embodiment, similarity between the section frequency distribution of the section image Selp and the section frequency distribution of the other section images Se2p to Se8p is calculated. The similarity corresponds to the transparency change of the other sections Se2 to Se8 with respect to the transparency of the section Se1.

After the transparency change detection process S42, the image processing device 40 may output the detection result in the transparency change detection process S42 by the output unit 43 to be presented to the assessor (output process).

### [Foreign Matter Detection Process]

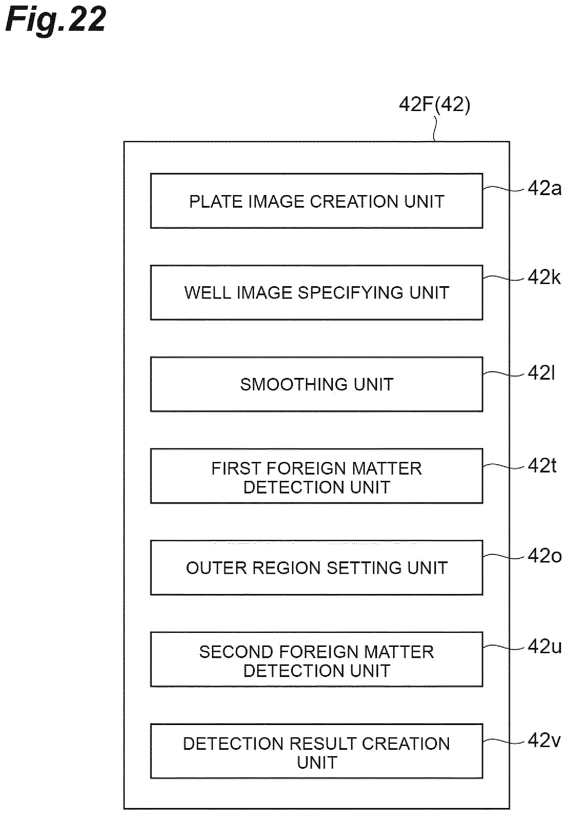

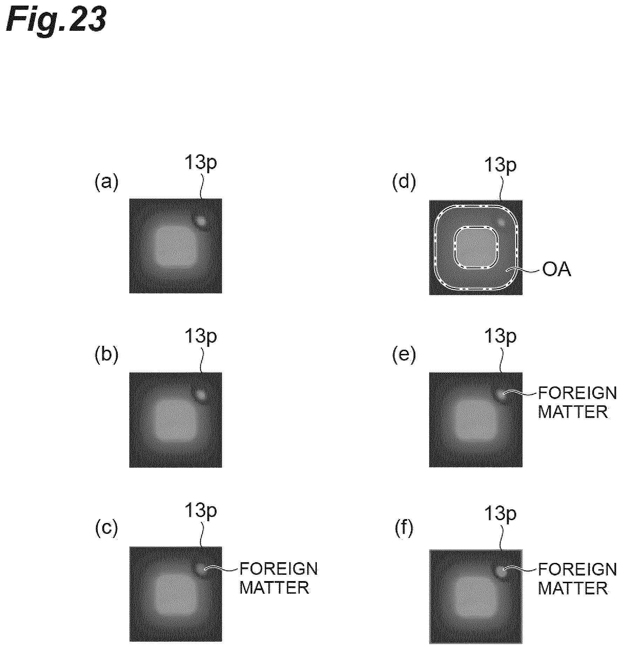

The operation unit 42 in the case of performing the foreign matter detection process will be referred to as an operation unit 42F, and the operation unit 42F will be described. FIG. 22 is a functional block diagram of the operation unit 42F. FIG. 23 is a view for describing the foreign matter detection process by the operation unit 42F. The operation unit 42F includes a plate image creation unit 42a, a well image specifying unit 42k, a smoothing unit 421, a first foreign matter detection unit 42t, an outer region setting unit 42o, a second foreign matter detection unit 42u, and a detection result creation unit 42v. The plate image creation unit 42a, the well image specifying unit 42k, and the smoothing unit 421 are similar to the case of the operation unit 42D, and thus description thereof will be omitted. An example of a well image 13p specified by the well image specifying unit 42k is illustrated in the (a) of FIG. 23. The (b) of FIG. 23 is a view after the well image 13p in the (a) of FIG. 23 is smoothed by the smoothing unit 421.

The first foreign matter detection unit 42t detects foreign matter in the well image 13p smoothed by the smoothing unit 421 by using a first luminance value. Typically, in a foreign matter region, an inner side of foreign matter is bright (luminance is high), and the periphery of the foreign matter is dark (luminance is low). Accordingly, a region where the inner side is bright and the periphery is dark becomes a foreign matter candidate. Accordingly, a first luminance threshold value for foreign matter detection is set, and the well image 13p is binarized. In a binarized image, a region which are surrounded by a luminance value smaller than the first luminance threshold value and in which an inner side is equal to or greater than the first luminance value is detected as the foreign matter candidate. The first luminance threshold value may be set as a luminance value of the inner region of the well image 13p (refer to FIG. 2). The luminance value (first luminance value) of the inner region may be designated by the assessor on the basis of the well image 13p. For example, the inner region may be set in advance by the inner region setting unit 42m. The first luminance value may be automatically set by the first foreign matter detection unit 42t. In a case where the first foreign matter detection unit 42t automatically sets the first luminance value, for example, it may be automatically set on the basis of an average luminance value within a certain range with respect to the center of the well image 13p. Typically, the foreign matters have a circular shape and a constant size. Accordingly, in this embodiment, among the foreign matter candidates, a candidate that further satisfies the shape (circularity) and the size which are assumed as a foreign matter is detected as a foreign matter. For example, detection of the foreign matter from the foreign matter candidates on the basis of the shape and size may be performed by the assessor viewing an image presenting the foreign matter candidates, or the first foreign matter detection unit 42t may make a determination on the basis of the shape (circularity) and the size which are set in advance. The (c) of FIG. 23 is a view illustrating an example of the foreign matter detection result by the first foreign matter detection unit 42t.

The outer region setting unit 42o sets an outer region OA in the well image 13p in a similar manner as in the outer region setting unit 42o in the operation unit 42D (refer to FIG. 16). For example, the outer region setting unit 42o may set an ROI in the outer region OA as indicated by one-dot chain line in the (d) of FIG. 23.

The second foreign matter detection unit 42u detects foreign matters by using a second luminance value. The second luminance value is set so that foreign matters can be detected in correspondence with the brightness of the outer region OA. The second luminance value may be designated by the assessor on the basis of the brightness (luminance) of the outer region OA. The second luminance value may be automatically set by the second foreign matter detection unit 42u. In a case where the second foreign matter detection unit 42u automatically sets the second luminance value, for example, a value obtained by multiplying an average luminance of the outer region OA by a constant value may be set. Since the outer region OA is darker (has a lower luminance) in comparison to the vicinity of the center of the well image 13p, typically, the first luminance threshold value and the second luminance threshold value are different from each other.

The second foreign matter detection unit 42u binarizes the outer region OA by using the second luminance threshold value, and detects a region having a luminance value larger or smaller than the second luminance value in the binarized image as foreign matter candidate. In this embodiment, the second foreign matter detection unit 42u detects, as foreign matter, a candidate that further satisfies the shape (circularity) and the size assumed as a foreign matter among the detected foreign matter candidates. For example, the detection of the foreign matter from the foreign matter candidates on the basis of the shape and the size may be performed by the assessor viewing an image presenting the foreign matter candidates, or the second foreign matter detection unit 42u may make a determination on the basis of the shape (circularity) and the size which are se t in advance. The shape and the size may be the same as the shape and the size in the first foreign matter detection unit 42t, or may be different. The (e) of FIG. 23 is a view illustrating an example of the foreign matter detection result obtained by the second foreign matter detection unit 42u.

The detection result creation unit 42v integrates the results in the first foreign matter detection unit 42t and the second foreign matter detection unit 42u to create data representing a foreign matter detection result. For example, the detection result creation unit 42v can create data in which foreign matter regions detected by the first foreign matter detection unit 42t and the second foreign matter detection unit 42u are emphasized (for example, colored).

The operation unit 42 outputs the detection result created by the foreign matter detection result creation unit 42v to the output unit 43 as a final foreign matter detection result. The (f) of FIG. 23 is a view illustrating an example of the final detection result obtained by the operation unit 42.

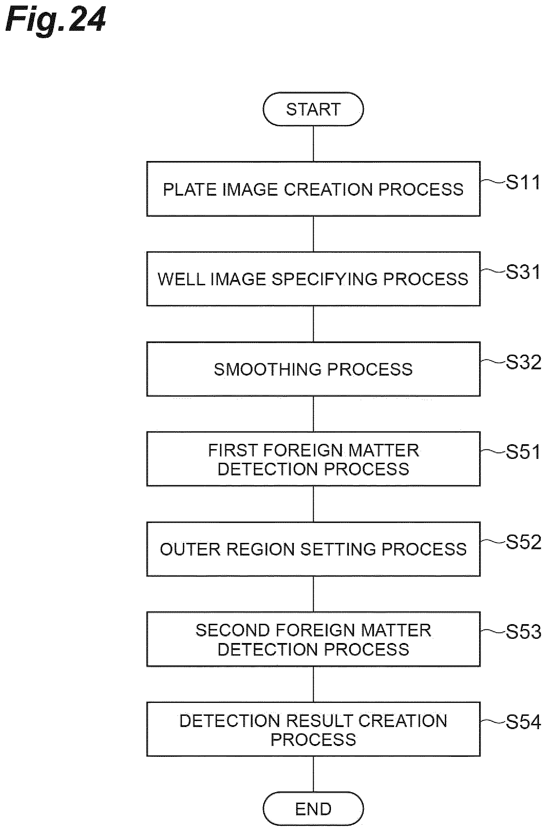

Next, an example of a method for assessing the assessment plate 10 (a method for assessing a test substance) by using the foreign matter detection process will be described. FIG. 24 is as an example of a flowchart of the assessment method using the foreign matter detection process. In the case of performing the assessment method using the foreign matter detection process, the operation unit 42 functions as the operation unit 42F. Here, a process subsequent to the process (input process) in which the image acquired by the imaging unit 33 of the observation device 30 is received by the input unit 41 will be described.

In a similar manner as in the case illustrated in FIG. 18 (the case of the colony detection process), the plate image creation process S11, the well image specifying process S31, and the smoothing process S32 are performed.

After the smoothing process S32, the image processing device 40 detects presence or absence of foreign matter within the well image 13p by the first foreign matter detection unit 42t (first foreign matter detection process S51). In this embodiment, foreign matters are detected on the basis of the first luminance threshold value, the shape (circularity), and the size as described with regard to the first foreign matter detection unit 42t.

After the first foreign matter detection process S51, the image processing device 40 sets an outer region OA of the well image 13p by the outer region setting unit 42o as in a similar manner as in the case of the outer region setting process S35 illustrated in FIG. 18 (outer region setting process S52). In this embodiment, an ROI is set in the outer region OA. The ROI setting region may be designated by the assessor, or may be automatically set by the outer region setting unit 42o on the basis of data of the plate 11.

After the outer region setting process S52, the image processing device 40 detects presence or absence of foreign matter within the well image 13p by the second foreign matter detection unit 42u (second foreign matter detection process S53). In this embodiment, the foreign matters are detected on the basis of the second luminance threshold value, the shape (circularity), and the size as described with regard to the second foreign matter detection unit 42u.

After the second foreign matter detection process S53, the image processing device 40 integrates the results in the first foreign matter detection process S51 and the second foreign matter detection process S53 by the detection result creation unit 42v to create a final foreign matter detection result (detection result creation process S54). In this embodiment, a process of emphasizing the foreign matter region such as coloring of the foreign matter region detected in the first foreign matter detection process S51 and the second foreign matter detection process S53 is performed.

After the detection result creation process S54, the image processing device 40 may output the detection result created in the detection result creation process S54 by the output unit 43 to be presented to the assessor (output process).

Next, a procedure of an example of the Ames test using the plate 11 will be described in detail.

### (1A^{th} Process)

A tester (bacteria having amino acid-requiring mutation, for example, Salmonella typhimurium: TA100 strain, TA98 strain, TA1535 strain, TA1537 strain, Escherichia coli: WP2uvrA strain, or the like) that is used is inoculated into 60 mL of Nutrient broth medium, and is shaken and cultured at 37°C for 9 to 10 hours to be proliferated.

### (2A^{th} Process)

A test substance (for example, a necessary amount of compound of which toxicity is desired to be assessed) is weighed.

### (3A^{th} Process)

The test substance prepared in the 2A^{th} process is dissolved or suspended in an appropriate medium, and the resultant solution is diluted by using the medium in stages to prepare concentration series of the test substance solution. For example, in the case of assessing the same test substance with six concentrations different from each other, six test substance solutions having concentrations different from each other are prepared.

### (4A^{th} Process)

A solution of a positive control compound that is known to exhibit predetermined toxicity is prepared.

### (5A^{th} Process)

The medium used in preparation of the test substance solution, the concentration series of the test substance solution, and the solution of the positive control compound (collectively referred to as "test substance solution and the like") are put into individual wells of 24-well plate (plate including 24 wells) by 10 µL.

### (6A^{th} Process)

0.175 to 0.7 mL of tester culture solution (having a concentration exceeding 1×10⁹ cells/mL), and 6.83 to 6.3 mL of medium (minimum medium to which a carbon source is added and which is deficient in amino acid) are mixed. The resultant mixture is referred as a preparation bacterial solution under conditions of the absence of metabolic activation.

### (7A^{th} Process)

240 µL of preparation bacterial solution under conditions of the absence of metabolic activation prepared in the 6A^{th} process is added to the 24-well plate into which 10 µL of the test substance solution and the like has been put.

### (8A^{th} Process)

The 24-well plate into which the mixed solution in the 7A^{th} process is put is shaken at 37°C and 250 rpm for 90 minutes (pre-incubation).

### (9A^{th} Process)

2.6 mL of indicator medium (a minimum medium which contains a pH indicator and a carbon source and which is deficient in amino acid) is added to each well (250 µL) of the 24-well plate after termination of the pre-incubation.

### (10A^{th} Process)

The mixed solution in the 9A^{th} process is transferred to the 48 wells (corresponding to one section) provided in each of eight sections Se1 to Se8 of the plate 11 that is a 384-well plate by 50 µL so as to correspond to the sections Se1 to Se8. In a similar manner, the mixed solution is seeded to three or more 384-well plates (plates 11).

### (11A^{th} Process)

The 384-well plates (plates 11) are cultured at 37°C for 48 hours. According to this, the assessment plate 10 is obtained.

In the Ames test, the following 12A^{th} process to 14A^{th} process are performed after the 11A^{th} process. In this embodiment, processes subsequent to the 12A^{th} process are performed by the assessment method described by using the assessment system 20.

### (12A^{th} Process)

A color change of each well 13, presence or absence of colonies, presence or absence of foreign matters, and a transparency change in the assessment plate 10 that is the plate 11 for which culture has been terminated are observed, the number of wells in which the color change, the colonies, and the foreign matters are present is counted, and a section in which the transparency change occurred is recorded.

### (13A^{th} Process)

The result of presence or absence of the foreign matters, and the result of the transparency change are combined to determine presence or absence of cytotoxicity.

### (14A^{th} Process)

The result of the color change and the result of presence or absence of the colonies are combined to determine presence or absence of genotoxicity.

The procedure of the 13A^{th} process and the 14A^{th} process is not limited.

In the case of detecting a test substance showing toxicity after being metabolized by various enzymes in a living body, in the 6A^{th} process, a solution (S9 mixture) obtained by adding a coenzyme to an extract of an animal liver is prepared, and 0.175 to 0.7 mL of tester culture solution, 5.78 to 5.25 mL of medium, and 1.05 mL of S9 mixture are mixed. The resultant mixture is referred to as a preparation bacterial solution under conditions of the presence of metabolic activation. Then, the processes subsequent to the 7A^{th} process are performed by using the preparation bacterial solution under conditions of the presence of metabolic activation instead of the preparation bacterial solution under conditions of the absence of metabolic activation.

Next, an operational effect of the assessment system 20, the image processing device 40, and the assessment method described in this embodiment will be described.

In the case of assessing the assessment plate 10, the assessment plate 10 is imaged by using the observation device 30 (imaging process). The image processing device 40 forms the plate image P1 on the basis of an image of the assessment plate 10 which is obtained in the imaging process.

The image processing device 40 performs a color determination process by using the plate image PI, thereby determining the color of a well image 13p. According to this, the color of a well 13 corresponding to the well image 13p is determined. In this embodiment, in a case where the test substance has genotoxicity with respect to cells contained in the assessment target 14, as a positive result, the color of the indicator varies from purple into yellow. Accordingly, the genotoxicity of the test substance can be assessed by the color determination process.

In the color determination process described in this embodiment, the color is determined using a frequency distribution having hue and saturation as variables, which is created for each well image 13p. Accordingly, for example, even in a case where it is difficult to determine the difference in color in visual observation, the color can be reliably determined. Accordingly, the color can be determined objectively and with high accuracy.

In the first color determination process, determination is performed three times in accordance with the flowchart of the first color determination process illustrated in FIG. 11. In this case, the color of the well image 13p (that is, the color of the well 13) can be more clearly determined as purple or yellow by repeating the determination, as illustrated in FIG. 8 to FIG. 10.

FIG. 10 and FIG. 13 are results of the first and second color determination processes with respect to the same plate image PI as described above. From FIG. 10 and FIG. 13, it can be seen that the color of the well image 13p (that is, the color of the well 13) can be more clearly determined as purple or yellow in the first color determination process.

The image processing device 40 determines presence or absence of a colony in the well 13 by performing a colony detection process by using the plate image P1. In this embodiment, in a case where the test substance has genotoxicity with respect to cells contained in the assessment target 14, there is a possibility that the colony may occur. That is, a case where the colony is detected corresponds to a positive result. Accordingly, the genotoxicity of the test substance can be assessed by the colony detection process.

In this embodiment, the colony is detected in each region by dividing the well image 13p into the inner region IA and the outer region OA. Since brightness is different between the inner region IA and the outer region OA, the colony can be more reliably detected by detecting the colony in each region of the inner region IA and the outer region OA.

In the image processing device 40, it is possible to perform the colony detection process as well as the color determination process. In this case, in a case where a result in either the color determination process or the colony detection process is positive, assessment is made as a positive well, so that the genotoxicity of the test substance can be more reliably assessed. When detecting the colony, in the form of detecting colonies in the section images Se2p to Se8p using a colony (or a colony candidate) detected in the section image Selp corresponding to the section Se1 that is a solvent control section, the colony can be more accurately detected. In a case where the color determination process has been performed in advance, in the section image Selp, a colony is not actually detected with respect to a well image 13p determined as purple by the color determination. Therefore, the colony detected in the well image 13p determined as purple in the color determination of the section image Selp can be regarded as a detection error. Accordingly, it is easy to accurately detect an actual colony to be detected by detecting a colony larger than the colony detected in the section image Selp.

In the case of performing the colony detection process in combination with the color determination process with respect to the same assessment plate 10 by using the image processing device 40, data that is used in a previously performed process may be appropriately used in a subsequently performed process. For example, in the case of performing the color determination process before the colony detection process, the plate image PI created in the color determination process may be used in the colony detection process. In this case, in the operation unit 42D illustrated in FIG. 16, the plate image creation unit 42a is not necessary. Similarly, in an assessment method using the colony detection process illustrated in FIG. 18, the plate image creation process S11 can be omitted. This is also true of the case of performing the colony detection process prior to the color determination process. The procedure of the color determination process and the colony detection process is not limited.

The image processing device 40 detect the transparency change of the section images Selp to Se8p by performing the transparency detection process by using the plate image P1. According to this, the transparency change of the sections Se1 to Se8 is detected. In this embodiment, in a case where the test substance has cytotoxicity with respect to cells contained in the assessment target 14, the transparency of the well 13 (specifically, the transparency of the assessment target 14) varies due to cell death or the like. Accordingly, cytotoxicity of the test substance can be assessed by the transparency change detection process.

Even in a case where the test substance precipitates during culture, the transparency of the well 13 (specifically, the transparency of the assessment target 14) varies. Accordingly, presence or absence of precipitation of the test substance can also be assessed by the transparency change detection process.

In the transparency change detection process described in this embodiment, the transparency change is determined by using a frequency distribution with hue and saturation as variables created for each well image 13p. Accordingly, for example, even in a case where it is difficult to determine a transparency difference in visual observation, the transparency change can be reliably determined. Accordingly, the transparency change can be determined objectively and with high accuracy.

In this embodiment, the transparency change is calculated on the basis of a section frequency distribution with respect to the section image Selp (solvent control region), and similarity with section frequency distributions of other sections images Se2p to Se8p in the same plate image P1. Accordingly, an influence by test conditions can be reduced, and thus the transparency can be more accurately detected.

The image processing device 40 determines presence or absence of foreign matters in the well 13 by performing the foreign matter detection process by using the plate image P1. In this embodiment, in a case where the test substance has cytotoxicity with respect to cells contained in the assessment target 14, foreign matters occur due to death of the cells. Accordingly, cytotoxicity of the test substance can be assessed by the foreign matter detection process.

In this embodiment, after performing foreign matter detection once with respect to the well image 13p, the outer region OA is set, and foreign matters in the outer region OA are detected again by using the luminance threshold value (second luminance threshold value) corresponding to the outer region OA. Typically, the outer region OA is dark, and thus foreign matters are difficult to be detected. Therefore, the foreign matters can be more reliably detected by using the luminance threshold value (second luminance threshold value) corresponding to the outer region OA.

In the image processing device 40, the foreign matter detection process can be performed in combination with the transparency change detection process. In this case, in either a case where the transparency change is detected by the transparency change detection process, or a case where the foreign matters are detected by the foreign matter detection process, it is assessed that the cytotoxicity is present, and thus the cytotoxicity of the test substance can be more reliably assessed.

The transparency detection process and the foreign matter detection process as the cytotoxicity assessment are used, for example, in setting of a concentration of a test substance for accurately performing genotoxicity assessment. For example, the concentration of the test substances in a section with a positive result among the sections Se1 to Se8 is not appropriate for the genotoxicity assessment in accordance with the transparency detection process. After at least the transparency detection process or the foreign matter detection process is performed once and a concentration range in which a result of the cytotoxicity assessment result is negative is determined, the genotoxicity assessment may be performed.

As described above, for example, the transparency detection process or the foreign matter detection process is performed to assess cytotoxicity of the test substance prior to the genotoxicity assessment. Accordingly, conditions of the assessment plate 10 in the case of performing the transparency detection process or the foreign matter detection process, and conditions of the assessment plate 10 in the case of performing at least one process between the color determination process and the colony detection process for the genotoxicity assessment may be different from each other (for example, concentration setting of the test substance may be different).

However, for example, conditions of the assessment plate 10 in the case of performing the transparency detection process or the foreign matter detection process, and conditions of the assessment plate 10 in the case of performing at least one process between the color determination process and the colony detection process are the same as each other, data created in the transparency detection process or the foreign matter detection process or the like may be appropriately used in the color determination process and the colony detection process.

For example, in a case where the color determination process is performed after the transparency detection process, since processes up to the frequency distribution creation process S13 are common, the color determination process may be performed by using the frequency distribution for each well image 13p which is created by the frequency distribution creation unit 42c (frequency distribution creation process S13) in the transparency detection process. Alternatively, in the case of performing the colony detection process after the foreign matter detection process, since the processes up to the well image specifying process S31 are common, the colony detection process may be performed by using the well image 13p specified by the well image specifying unit 42k (well image specifying process S31). In a process flow in a case where the operation unit 42D performs the colony detection process, or in a process flow in a case where the operation unit 42F performs the foreign matter detection process, the operation unit 42D and the operation unit 42F may not have a function corresponding to the process omitted as described above.

For example, as illustrated in FIG. 1, in the case of determining the color of 384 wells 13, since a burden on the assessor is heavy and judgement ability of the assessor is likely to deteriorate, the objectivity and the accuracy tend to decrease. In contrast, in the assessment method using the image processing device 40, since the color determination is performed by the image processing device 40, for example, the color can be determined objectively and with high accuracy in comparison to a case where the assessor visually perform determination. This is also true of detection of presence or absence of the colony, detection of the transparency change, and detection of the foreign matters.

Next, Experimental Example 1 in which the assessment system 20 and the assessment method described in this embodiment are applied to the Ames test will be described. Hereinafter, the same reference numeral will be given to the same element as the element described until now unless otherwise stated, and redundant description will be omitted.

In Experimental Example 1, View-Plate-384 (manufactured by Perkinelmer, Inc.) was used as the plate 11. View-Plate-384 included 384 wells 13 as illustrated in FIG. 1. Even in this Experimental Example 1, sections Se1 to Se8 were virtually set in a similar manner as in FIG. 1.

In this Experimental Example 1, an Ames test kit ("Ames MPF^{™} Penta I Microplate Format Mutagenicity Assay") (manufactured by Xenometricx AG) containing an indicator, a culture solution, and the like was used.

Nine kinds of assessment plates 10 different in test conditions were prepared. The assessment plates 10 used in this Experimental Example 1 are assessment plates 10 in a state in which toxicity test can be performed by performing administration of cells, a test substance, and the like into the wells 13, culture, and the like in accordance with manual of the Ames test kit. The nine kinds of assessment plates 10 different in test conditions are referred to as assessment plates 10_1 to 10_9 in this Experimental Example 1.

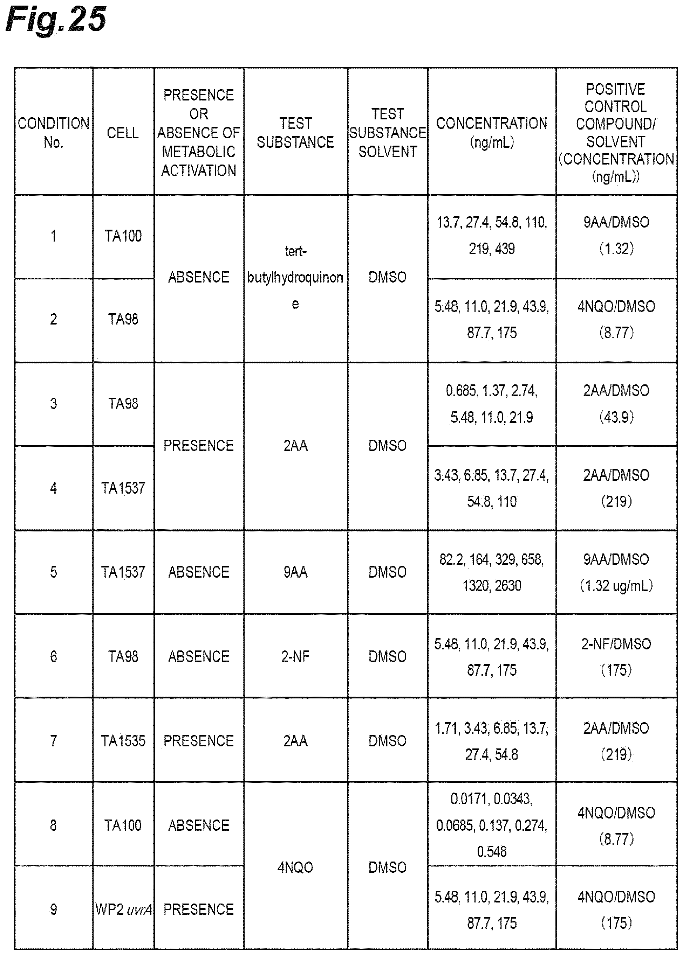

The test conditions of the assessment plates 10_1 to 10_9 are as illustrated in a chart in FIG. 25. In the drawing, Conditions No. 1 to No. 9 represent the assessment plates 10_1 to 10_9. In a column of "presence or absence of metabolic activation", "absence" represents a case where the assessment target 14 does not contain S9mix, and "presence" represents a case where the assessment target 14 contains S9mix. A column of "concentration (ng/ml)" shows the concentration of test substances in the assessment target 14 (specifically, the assessment target 14b to 14g) held by the wells 13 in the sections Se2 to Se7. Six concentrations shown in the column of "concentration (ng/mL)" with respect to No. 1 to No. 9 (assessment plates 10_1 to 10_9) are concentrations of the test substances contained in the assessment targets 14b to 14g. The concentrations of the test substances in the assessment targets 14b to 14g increases from the assessment target 14b toward the assessment target 14g. "Concentration" in "positive control compound/solvent (concentration (ng/mL))" represents the concentration of positive control compounds in the section Se8 (positive control section). As the test substance solution and the solvent of a positive control solution, DMSO was used as illustrated in the drawing. The solvent control solution was also DMSO.

In this Experimental Example 1, N sheets were prepared for each of the assessment plates 10_1 to 10_9. N is three for the assessment plates 10_1, 10_3, 10 4, 10_7, and 10_9, and is one for the remaining assessment plates.

In this Experimental Example 1, first, an imaging process of imaging the assessment plates 10_1 to 10_9 by the observation device 30 was performed. Then, a color determination process, a colony detection process, a transparency change detection process, and a foreign matter detection process were performed by using the image processing device 40. In addition, color determination for the assessment plates 10_1 to 10_9 by visual observation was also performed.

The imaging process in this Experimental Example 1 will be described. In this Experimental Example 1, two imaging processes (hereinafter, referred to as "first imaging process" and "second imaging process") were performed.

The first imaging process was performed with respect to all of the assessment plates 10_1 to 10_9. The first imaging process for the assessment plates 10_1 to 10_9 is the same in each case, and thus the first imaging process for the assessment plate 10_1 will be described as a representative.

In the first imaging process, the observation device 30 illustrated in FIG. 3 was used. As the illumination device 32 provided in the observation device 30, LED lighting that outputs visible light was used. As the condensing unit and the imaging device 33a of the imaging unit 33, a lens and a line sensor (CCD camera) were used, respectively.

In this Experimental Example 1, since an imaging range of the imaging device 33a is smaller than a size of the assessment plate 10_1, a region that is approximately the half of the assessment plate 10_1 was imaged by using the imaging device 33a (and the condensing unit 33b corresponding thereto) while conveying the assessment plate 10_1 in one direction by the stage 31. Then, after shifting the assessment plate by approximately the half in a direction orthogonal to a conveyance direction of the assessment plate, and then the remaining region of the assessment plate 10_1 was imaged while moving the assessment plate 10_1 on one direction.

In this Experimental Example 1, the first imaging process was performed for N assessment plates 10_1.

Similarly, the second imaging process was performed for the assessment plate 10_1 and 10_4. The second imaging process for the assessment plate 10_1 to 10_4 is the same in each case, and thus the second imaging process for the assessment plate 10_1 will be described as a representative.

In the second imaging process, in the observation device 30 illustrated in FIG. 3, imaging of the assessment plate 10_1 was performed in a similar manner as in the first imaging process in a state in which positions of the illumination device 32 and the imaging unit 33 with respect to the assessment plate 10_1 are reversed (that is, in FIG. 3, in a state in which the imaging unit 33 is disposed above the plate, and the illumination device 32 is disposed on a lower side of the stage 31).

In this Experimental Example 1, the second imaging process was performed for N assessment plates 10_1.

Next, in this Experimental Example 1, the color determination process was performed with respect to an image acquired in the imaging process by using the image processing device 40. In the color determination process, the image acquired in the first imaging process was used. In addition, the foreign matter detection process was performed with respect to the assessment plate 10_1. In addition, the colony detection process was performed with respect to the assessment plate 10_4. As to be described later, the colony detection process and the foreign matter detection process were performed with respect to the image acquired in the first imaging process and was also performed with respect to the image acquired in the second imaging process.

### [Color Determination Process]

The color determination process was performed for all of the assessment plates 10_1 to 10_9. A color determination method for the assessment plates 10_1 to 10_9 is the same in each case. Accordingly, the color determination process for the assessment plate 10_1 will be described as a representative.

The color determination process was performed in accordance with the flowchart of the color determination process (first color determination process) illustrated in FIG. 11 by using the image processing device 40. In the first determination process S14, a calculated similarity distribution was presented to an assessor as a graph illustrated in FIG. 8, and the assessor set a threshold value for division into purple and yellow. Similarly, a threshold value was also set in the second determination process S16 and the third determination process S18.

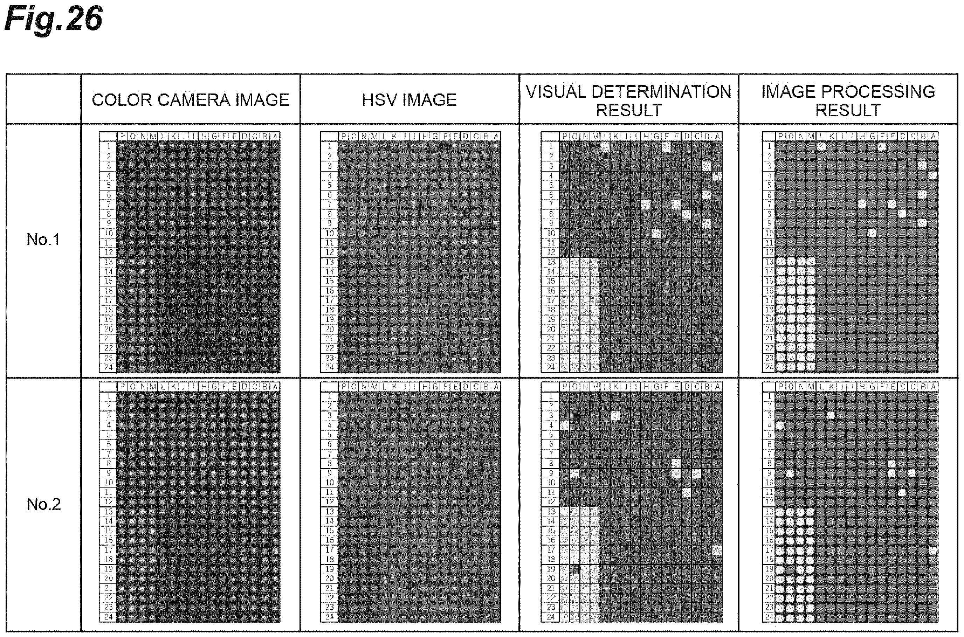

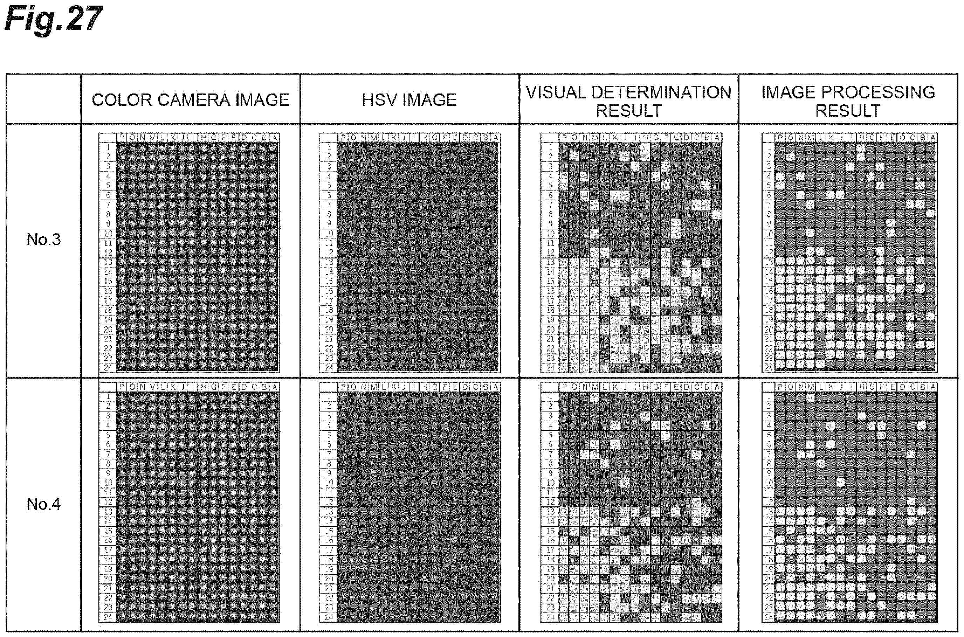

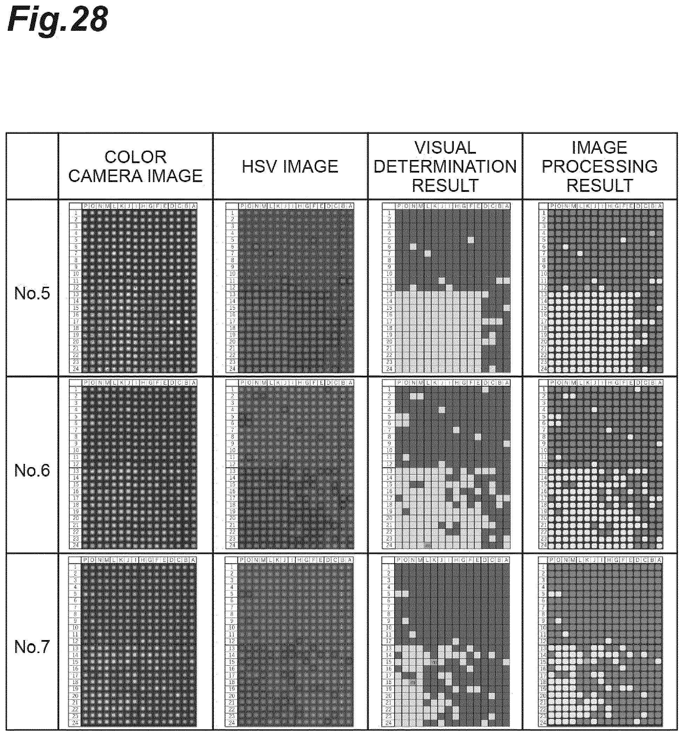

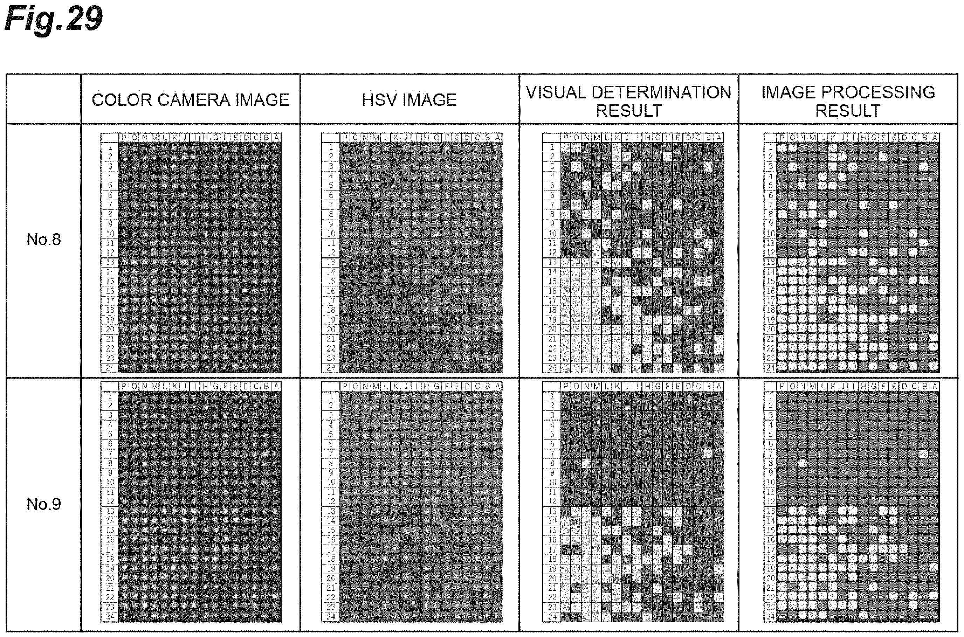

Results of the color determination process were as illustrated in the charts in FIG. 26 to FIG. 29. In FIG. 26 to FIG. 29, a color camera image, an HSV image, and a visual determination result are also shown in addition to a detection result that is the color determination result.

No. 1 to No. 9 in FIG. 26 to FIG. 29 correspond to the assessment plates 10_1 to 10_9. A result of No. 1 in FIG. 26 is a result of one of N assessment plates 10_1. This is also true of results of No. 2 to No. 9 in FIG. 26 to FIG. 29.

"Color camera image" in FIG. 26 to FIG. 29 is the plate image PI created in the plate image creation process S11 illustrated in FIG. 11. "HSV image" in FIG. 26 to FIG. 29 is the HSV image P2 created in the HSV image creation process S12 illustrated in FIG. 11. "Visual determination result" in FIG. 26 to FIG. 29 is an image display result when the assessor input visual determination results for the assessment plates 10_1 to 10_9 into the image processing device 40 and manually divides the results into purple and yellow. "Image processing result" in FIG. 26 to FIG. 29 is a view illustrating a result of the color determination process (first color determination process) by the image processing device 40.

"A to P" and "1 to 24" attached around each image illustrated in FIG. 26 to FIG. 29 correspond to "A to P" and "1 to 24" in FIG. 1.

In the images of the visual determination results and the image processing results, light-colored well images 13p show yellow, and dark-colored well images 13p show purple. In addition, well images 13p of an intermediate color show an intermediate color. In the visual determination results of the assessment plates 10_1 to 10_9, well images 13p attached with "m" show an intermediate color of purple and yellow.

From the results shown in FIG. 26 to FIG. 29, it can be understood that substantially the same result is obtained in the visual determination due to the color determination process using the image processing device 40. Actually, ratios of a difference between the detection results obtained for the assessment plates 10_1, 10_2, 10_5, and 10_6 with the image processing device 40 and the visual determination results (hereinafter, referred to as "difference rate") were 0.0%, 0.2%, 0.1%, and 0.0%, respectively. The difference rate of the assessment plate 10_1 was a value (%) obtained by dividing the sum of the number of wells 13, in which detection results obtained for three assessment plates 10_1 under the same conditions with the image processing device 40 are different from the visual determination results, by the number (384×N) of all of the wells 13, and difference rates of the assessment plates 10 2, 10_5, and 10_6 were values calculated by a similar method.

### [Colony Detection Process]

The colony detection process was performed for the assessment plate 10_4. As described above, the colony detection process was performed with respect to the images acquired by each of the first imaging process and the second imaging process.

First, a colony detection process using an image acquired in the first imaging process will be described.

The colony detection process was performed in accordance with the flowchart of the colony detection process illustrated in FIG. 18 by using the image processing device 40. In the well image specifying process S31, the plate 11 in a state in which nothing is put into the wells 13 was imaged, the image was set as a mask image, and a corresponding well region was set as the ROI. In addition, in the first colony detection process S34 and the second colony detection process S36, the luminance threshold value used in each process was designated by the assessor. Similarly, the shape and the size for colony detection were also designated by the assessor.

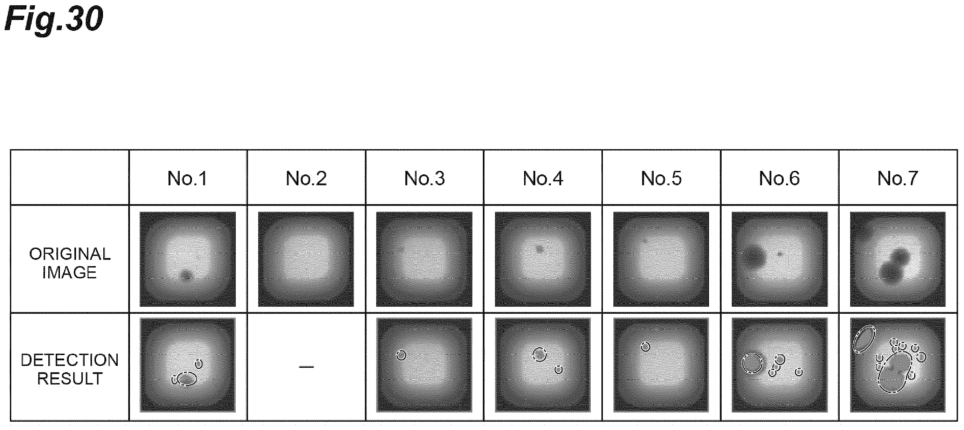

Results of the colony detection processes are as illustrated in FIG. 30. No. 1 to No. 7 in FIG. 30 are excerpts of parts of a plurality of well images 13p ("original image" in FIG. 30) in the plate image PI corresponding to the assessment plate 10_4, and show colony detection results corresponding to the excerpts. In the detection results, a region surrounded by a one-dot chain line is a region detected as a colony. No. 2 shows a well image 13p in which a colony does not occur as shown in the original image. In this case, a colony was not detected in the colony detection process by the image processing device 40. Accordingly, notation of a detection result of No. 2 is omitted.

When comparing the original image and the detection result in FIG. 30, it can be seen that a colony is appropriately detected by the colony detection process in the image processing device 40. In addition, in the colony detection process using the image processing device 40, it can be understood that a small colony difficult to be determined in the well image 13p can also be reliably detected. As in No. 2, in a case where a colony is not present in the well image 13p, the colony was not detected even in the colony detection process by the image processing device 40. That is, it can be understood that erroneous detection does not occur.

Next, a colony detection process using an image acquired in the second imaging process will be described. In this case, a colony was detected in a similar manner as in the case of using the image acquired in the first imaging process except that the image of the assessment plate 10_4 which was acquired in the second imaging process instead of the first imaging process was used.

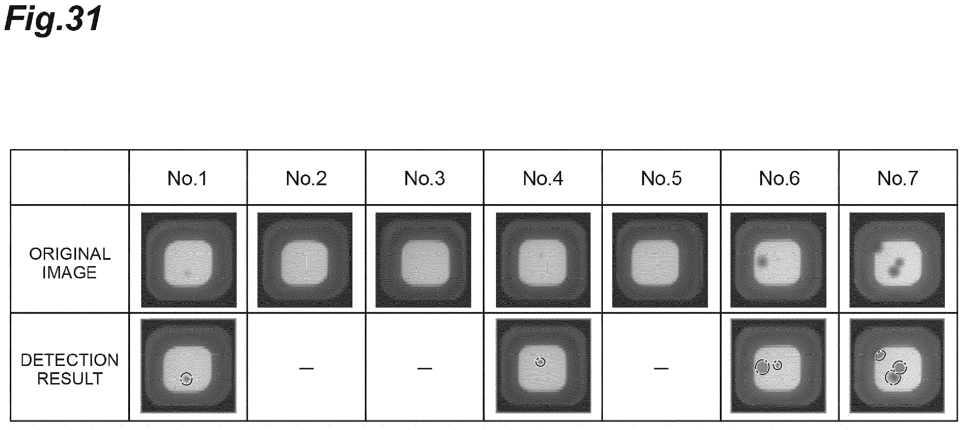

Results of the colony detection process are as illustrated in FIG. 31. No. 1 to No. 7 in FIG. 31 are partial extracts a plurality of well images 13p ("original image" in FIG. 31) in the plate image PI corresponding to the assessment plate 10_4, and show colony detection results corresponding to the excerpts. The well images 13p in FIG. 31 were images for the same wells 13 as in the case of the well images 13p in FIG. 30. In the detection results, a region surrounded by a one-dot chain line is a region detected as a colony. The reason why detection results in No. 2, No. 3, and No. 5 are omitted is similar to the case of No. 2 in FIG. 30. That is, since a colony is not present in an original image, the colony was not detected even in results obtained by using the image processing device 40.

When comparing the original image and the detection result in FIG. 31, it can be seen that some colonies are appropriately detected by the colony detection process in the image processing device 40.

As described above, FIG. 30 and FIG. 31 were results with respect to the well images 13p corresponding to the same wells 13. When comparing the results of FIG. 30 and FIG. 31 with each other, it can be understood that the colony can be more clearly detected in the case of acquiring the image of 10_4 by the first imaging process (that is, in the case of imaging 10 4 from the bottom 13a side of the well 13) in comparison to the case of acquiring the image of 10_4 by the second imaging process (that is, in the case of imaging 10_4 from a side opposite to the bottom 13a of the well 13) (for example, refer to results of Nos. 3 and 5 in FIG. 30 and FIG. 31). The reason for this is considered because the colony typically sinks to the bottom 13a side of the well 13.

### [Foreign Matter Detection Process]

The foreign matter detection process was performed with respect to the assessment plate 10_1. Here, the foreign matter detection was performed by using the images acquired in the first imaging process and the second imaging process described in the colony detection process.

First, foreign matter detection using the image acquired in the first imaging process will be described.

In the foreign matter detection process, detection was performed by using the image processing device 40 in accordance with the flowchart of the foreign matter detection process illustrated in FIG. 24. In the well image specifying process S31, as in the case with the colony detection process, a plate 11 in a state in which noting is put into the wells 13 was imaged, the image was set as a mask image, and a corresponding well region was set as the ROI. In the foreign matter detection process, the first luminance threshold value and the second luminance threshold value were designated by the assessor in the first foreign matter detection process S51 and the second foreign matter detection process S53. Similarly, the shape and the size for foreign matter detection were also designated by the assessor.

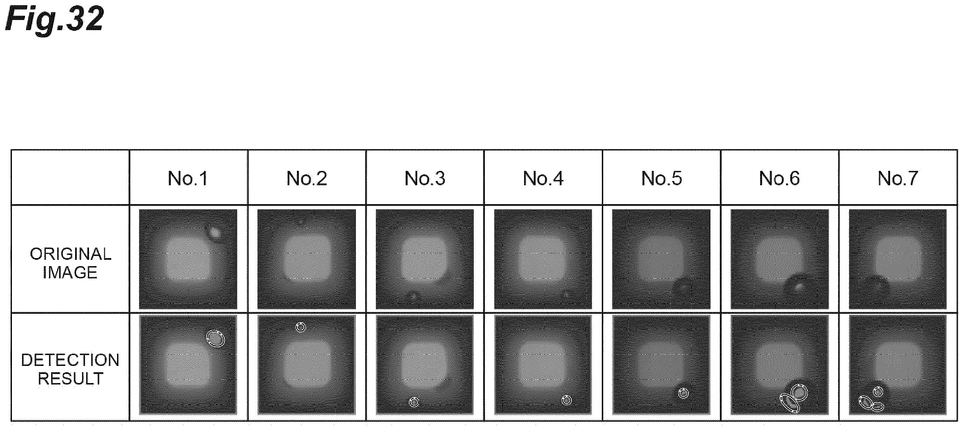

Results of the foreign matter detection process are as illustrated in the chart in FIG. 32. No. 1 to No. 7 in FIG. 32 are excerpts of parts of a plurality of well images 13p ("original image" in FIG. 32) in the plate image PI corresponding to the assessment plate 10_1, and show colony detection results corresponding to the excerpts. In the detection results, a region surrounded by a one-dot chain line is a region detected as foreign matters.

When comparing the original image and the detection result in FIG. 32, it can be seen that foreign matters are appropriately detected by the foreign matter detection process by using the image processing device 40. In addition, in the foreign matter detection process using the image processing device 40, it can be understood that small foreign matters difficult to be determined in the original image can also be reliably detected.

Next, a foreign matter detection process using an image acquired in the second imaging process will be described. In this case, foreign matters were detected in a similar manner as in the case of using the image acquired in the first imaging process except that the image of the assessment plate 10_1 which was acquired in the second imaging process instead of the first imaging process was used.

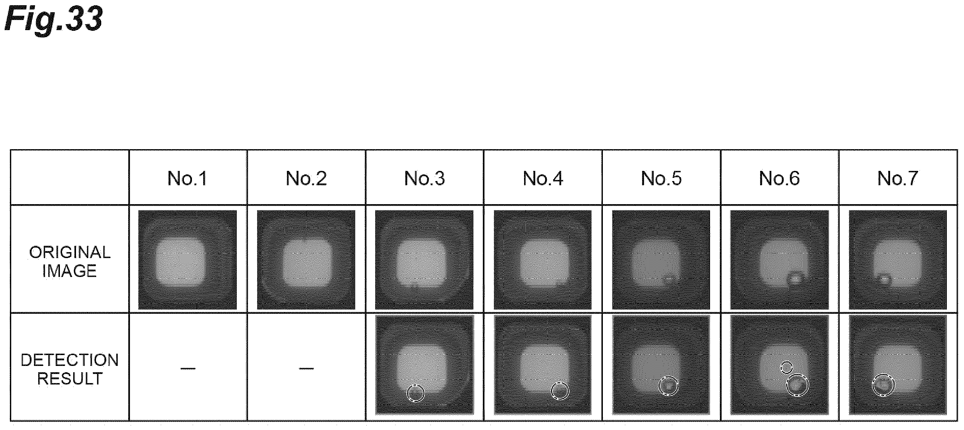

Results of the foreign matter detection process are as illustrated charts in FIG. 33. No. 1 to No. 7 in FIG. 33 are excerpts of parts of a plurality of well images 13p ("original image" in FIG. 33) in the plate image PI corresponding to the assessment plate 10_1, and show foreign matter detection results corresponding to the excerpts. The well images 13p in FIG. 33 were images for the same wells 13 as in the case of the well images 13p in FIG. 32. In the detection results, a region surrounded by a one-dot chain line is a region detected as foreign matters. The reason why detection results in No. 1 and No. 2 are omitted is similar to the case of No. 2 in FIG. 30. That is, since foreign matters is not present in an original image, the foreign matters were not detected even in results obtained by using the image processing device 40.

When comparing the original image and the detection result in FIG. 33, it can be seen that some foreign matters are appropriately detected as foreign matters by the foreign matter detection process with the image processing device 40.

As described above, FIG. 32 and FIG. 33 were results with respect to the well images 13p corresponding to the same wells 13. When comparing the results in FIG. 32 and FIG. 33 with each other, it can be understood that the foreign matters can be more clearly detected in the case of acquiring the image of the assessment plate 10_1 by the first imaging process (that is, in the case of imaging the assessment plate 10_1 from the bottom 13a side of the well 13) in comparison to the case of acquiring the image of the assessment plate 10_1 by the second imaging process (that is, in the case of imaging the assessment plate 10_1 from a side opposite to the bottom 13a of the well 13) (for example, refer to results of Nos. 1 and 2 in FIG. 32 and FIG. 33). The reason for this is considered because the foreign matters typically sink to the bottom 13a side of the well 13.

Next, a modification example of the embodiment will be described.

### (Modification Example 1)

In Modification Example 1, in an irradiation process in the case of capturing an image of the assessment plate 10, the assessment plate is irradiated with light L having a wavelength within at least one wavelength range between a wavelength range of 430 to 460 nm and a wavelength range of 520 to 620 nm.

For example, the irradiation process can be performed by employing LED lighting including at least one of an LED that outputs light having a wavelength in a wavelength range of 430 to 460 nm and an LED that outputs light having a wavelength in a wavelength range of 520 to 620 nm as the illumination device illustrated in FIG. 3. Alternatively, in the irradiation process, the assessment plate 10 may be irradiated with light output from the illumination device 32 through a wavelength filter that selectively passes at least one of an LED that outputs light having a wavelength in the wavelength range of 430 nm to 460 nm and an LED that outputs light having a wavelength in the wavelength range of 520 nm to 620 nm. The wavelength filter may be incorporated in the illumination device 32.

In the irradiation process, the assessment plate 10 may be irradiated with light L having a first wavelength selected in the wavelength range of 430 to 460 nm, and a second wavelength selected in the wavelength range of 520 to 620 nm, that is, mixed light of light of light having the first wavelength and light having the second wavelength. As an example of a combination of the first wavelength and the second wavelength, the first wavelength is 440 nm and the second wavelength is 590 nm.

Modification Example 1 is similar to the embodiment except that the wavelength of the light L that is emitted has the above-described conditions. Accordingly, Modification Example 1 has a similar operational effect as in the embodiment.

When using the light L in the above-described wavelength range, since the luminance difference between the wells 13 (for example, between the sections Se1 to Se8) which are different in a state of the assessment target 14 or the like increases, for example, the difference in color becomes clearer. As a result, the safety assessment can be more accurately performed.

For example, in a case where the color of the wells 13 (the color of the assessment target 14) varies due to a change in color of the pH indicator, or the like, the assessment plate 10 includes wells 13 having a color close to the color shown at a wavelength in the wavelength range of 430 to 460 nm and wells 13 having a color close to the color shown in a wavelength in the wavelength range of 520 to 620 nm. In a case where the light L has the first wavelength and the second wavelength, luminance of the wells 13 is likely to be secured with respect to both the color on a side of the wavelength range of 430 to 460 nm, and the color on a side of the wavelength range of 520 to 620 nm. In this case, the difference in color between the wells 13 (for example, between the sections Se1 to Se8) different in the state of the assessment target 14, or the like becomes clearer. As a result, the safety assessment can be more accurately performed.

Description will be given of a verification experiment relating to why the difference in color between the sections Se1 to Se8 becomes clearer by irradiating an assessment plate with light L having a wavelength within at least one wavelength range of the wavelength range of 430 to 460 nm and the wavelength range of 520 to 620 nm with reference to Experimental Example 2.

In Experimental Example 2, as a model of an assessment plate 10 in a state of color determination, that is, the assessment plate 10 obtained after completion of the 11A^{th} process in the procedure (from the 1A^{th} process to the 14A^{th} process) of the Ames test, five assessment plates of Sample No. 1 to Sample No. 5 were prepared as follows. Sample No. 1 to Sample No. 5 imitate a color tone range that can occur in the assessment plate of an actual Ames test. For convenience of explanation, the assessment plates of Sample No. 1 to Sample No. 5 prepared in Experimental Example 2 are also referred to as "assessment plate 10".

### (Sample No. 1)

Bromocresol purple (pH indicator) was injected into the 384 wells 13 provided in the plate 11 illustrated in FIG. 1. The bromocresol purple was a pH indicator that is variable from pH 5.2 (yellow) to pH 6.8 (purple). A length of a long side of the plate 11 was 127 mm, and a length of a short side thereof was 87 mm. A different amount of hydrochloric acid was further added to the section Se1 to the section Se8 to adjust the color of the wells 13 (the color of the pH indicator) of the sections Se1 to Se8 so as to enter a state (color state) mitigating an example of an assessment result in color assessment of the assessment plate 10. The amount of hydrochloric acid added, pH, and visual color determination in the sections Se1 to section Se8 of Sample No. 1 were as shown in Table 1. An intermediate color in Table 1 is a color between purple and yellow. In color determination, the color of the wells 13 of the section Se1 (negative control section) was regarded as purple, the color of the wells 13 of the section Se8 (positive control section) was regarded as yellow, and the color of the wells 13 of the section Se1 and the color of the wells 13 of the section Se8 were set as a reference.

**[Table 1]**

| No. | Section | pH | Amount of 1M hydrochloric acid added uL/mL | Visual observation |
|---|---|---|---|---|
| 1 | 1 | 6.78 | 0.00 | Purple |
| | 2 | 6.59 | 10.00 | Purple |
| | 3 | 6.57 | 15.00 | Purple |
| | 4 | 6.34 | 20.00 | Intermediate color |
| | 5 | 5.67 | 30.00 | Intermediate color |
| | 6 | 5.5 | 33.33 | Yellow |
| | 7 | 5.44 | 35.42 | Yellow |
| | 8 | 4.77 | 40.00 | Yellow |

### (Sample No. 2 to Sample No. 5)

The color of the wells 13 (the color of the pH indicator) of the section Se1 to the section Se8 of Sample No. 2 to Sample No. 5 was adjusted by adding hydrochloric acid so that Sample No. 2 to Sample No. 5 were in a state (color state) imitating different assessment results in color assessment of the assessment plate 10. The amount of hydrochloric acid added, pH, and visual color determination in the sections Se1 to Se8 of Sample No. 2 to Sample No. 5 were as shown in Table 2, Table 3, Table 4, and Table 5. An intermediate color in Table 2 and Table 3 is a color between purple and yellow. With regard to color determination in Sample No. 2 and Sample No. 3, in Sample Nos. 2 and 3, the color of the wells 13 of the section Se1 and the color of the wells 13 of the section Se8 were set as a reference in a similar manner as in Sample No. 1. With regard to color determination of Sample No. 4 and Sample No. 5, in Sample No. 4 and Sample No. 5, the color of the wells 13 of the section Se1 (negative control section) was regarded as purple, and the color of the wells 13 of the section Se1 was set as a reference.

**[Table 2]**

| No. | Section | pH | Amount of 1M hydrochloric acid added uL/mL | Visual observation |
|---|---|---|---|---|
| 2 | 1 | 5.83 | 30.00 | Purple |
| | 2 | 5.71 | 31.27 | Purple |
| | 3 | 5.58 | 32.33 | Intermediate color |
| | 4 | 5.52 | 33.67 | Intermediate color |
| | 5 | 5.38 | 35.00 | Yellow |
| | 6 | 5.36 | 36.33 | Yellow |
| | 7 | 5.16 | 37.50 | Yellow |
| | 8 | 4.89 | 40.00 | Yellow |

**[Table 3]**

| No. | Section | pH | Amount of 1M hydrochloric acid added uL/mL | Visual observation |
|---|---|---|---|---|
| 3 | 1 | 6.59 | 7.50 | Purple |
| | 2 | 6.49 | 11.78 | Purple |
| | 3 | 6.54 | 16.05 | Purple |
| | 4 | 6.33 | 20.33 | Intermediate color |
| | 5 | 5.94 | 24.60 | Intermediate color |
| | 6 | 5.88 | 28.88 | Intermediate color |
| | 7 | 5.63 | 33.15 | Yellow |
| | 8 | 5.47 | 37.50 | Yellow |

**[Table 4]**

| No. | Section | pH | Amount of 1M hydrochloric acid added uL/mL | Visual observation |
|---|---|---|---|---|
| 4 | 1 | 5.9 | 30.00 | Purple |
| | 2 | 6.02 | 25.71 | Purple |
| | 3 | 6.29 | 21.43 | Purple |
| | 4 | 6.37 | 17.14 | Purple |
| | 5 | 6.66 | 12.86 | Purple |
| | 6 | 6.74 | 8.57 | Purple |
| | 7 | 6.85 | 4.29 | Purple |
| | 8 | 6.84 | 0.00 | Purple |

**[Table 5]**

| No. | Section | pH | Amount of 1M hydrochloric acid added uL/mL | Visual observation |
|---|---|---|---|---|
| 5 | 1 | 6.67 | 7.50 | Purple |
| | 2 | 6.7 | 6.43 | Purple |
| | 3 | 6.76 | 5.36 | Purple |
| | 4 | 6.91 | 4.29 | Purple |
| | 5 | 6.86 | 3.21 | Purple |
| | 6 | 6.82 | 2.14 | Purple |
| | 7 | 6.73 | 1.07 | Purple |
| | 8 | 6.97 | 0.00 | Purple |

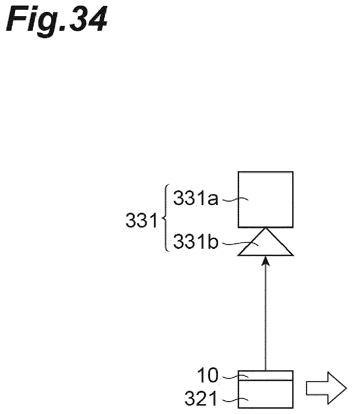

Then, the assessment plate 10 of Sample No. 1 was imaged by using an observation device including an illumination device 321 and an imaging unit 331 disposed to face the illumination device 321 as illustrated in FIG. 34. Specifically, the illumination device 321 was turned on, and while the assessment plate 10 of Sample No. 1 was disposed on the illumination device 321 and conveyed together, and the assessment plate 10 was imaged by the imaging unit 331.

The illumination device 321 was a surface light source device that uses an LED and was a surface light source device (KDB-100, manufactured by KOS21 CO., LTD.) that outputs light L having a wavelength of 400 to 700 nm. The imaging unit 331 includes an imaging device 331a, and a condensing unit 331b mounted to the imaging device 331a. The imaging device 331a was a Hyperspectral camera (Pika-L, manufactured by Resonon Inc.). A frame rate of the imaging device 331a was 249.2 Hz, and exposure time was 3.93 ms. As the condensing unit 331b, Xenoplan 1.4/23-0902 (aperture: F5.6) manufactured by Shneider Electric was used. A conveying speed of the assessment plate 10 was 22 min/s.

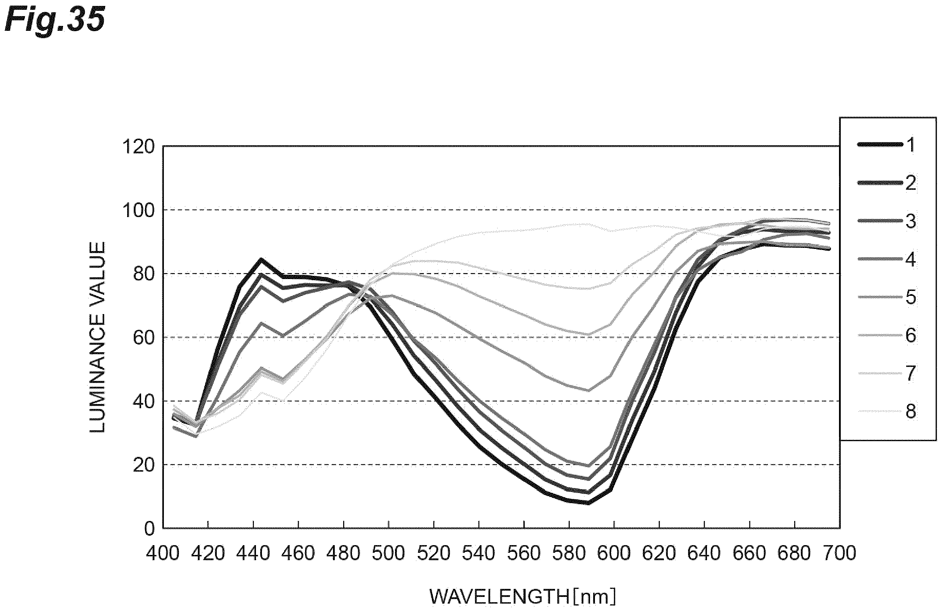

FIG. 35 is a view illustrating a spectrum distribution for every section based on imaging results of the assessment plate 10 of Sample No. 1. In FIG. 35, an average spectrum obtained by averaging spectrums obtained by the imaging device 331a (Hyperspectral camera) for each of the section Se1 to section Se8 is illustrated. In FIG. 35, the abscissa represents a wavelength, and the ordinate represents a luminance value. The Lines with different densities represented by "1" to "8" represent lines of the average spectrum corresponding to each of the section Se1 to the section Se8, and "1" to "8" correspond to the section Se1 to the section Se8, respectively. That is, colors of lines representing average spectrums become dilute in the order of the section Se1, the section Se2, ..., and the section Se8.

The assessment plates 10 of Sample No. 2 to Sample No. 5 were imaged in a similar manner as in the case of the assessment plate 10 of Sample No. 1 except that the assessment plate 10 to be imaged was changed into the assessment plates 10 of Sample No. 2 to Sample No. 5.

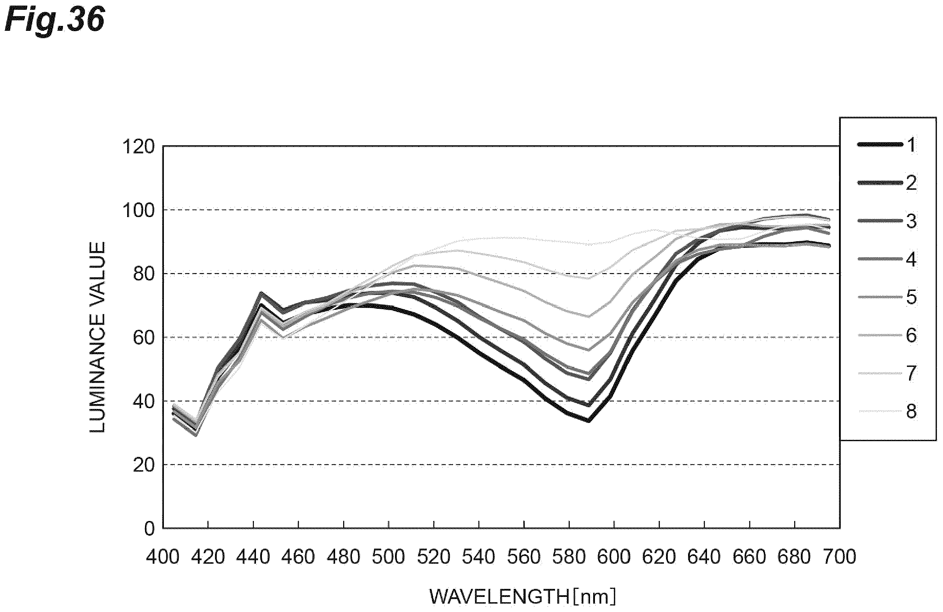

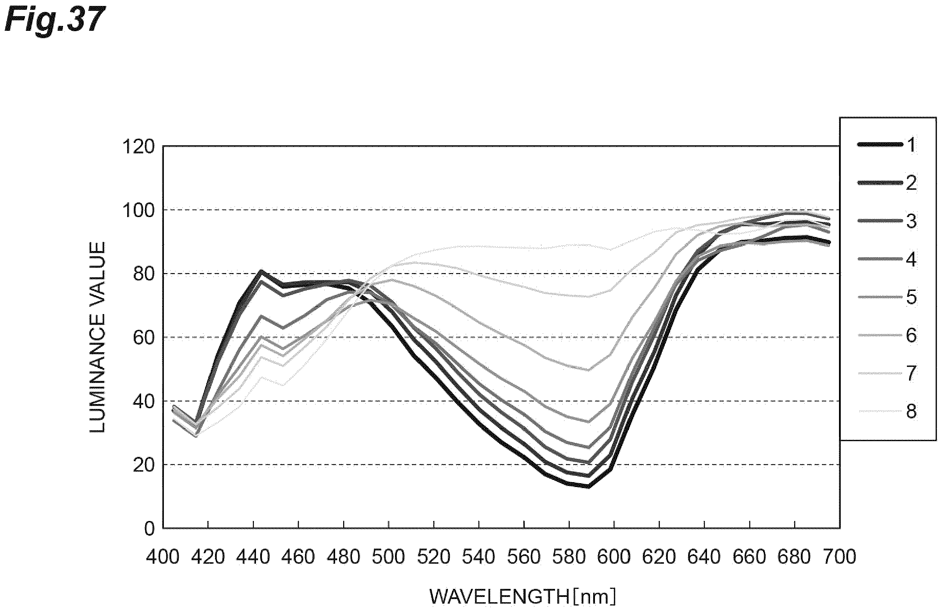

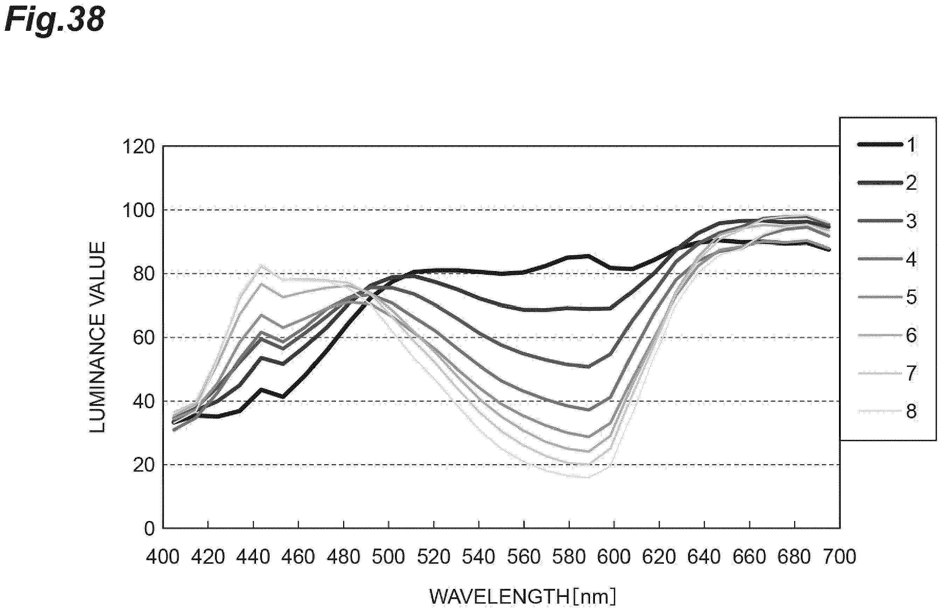

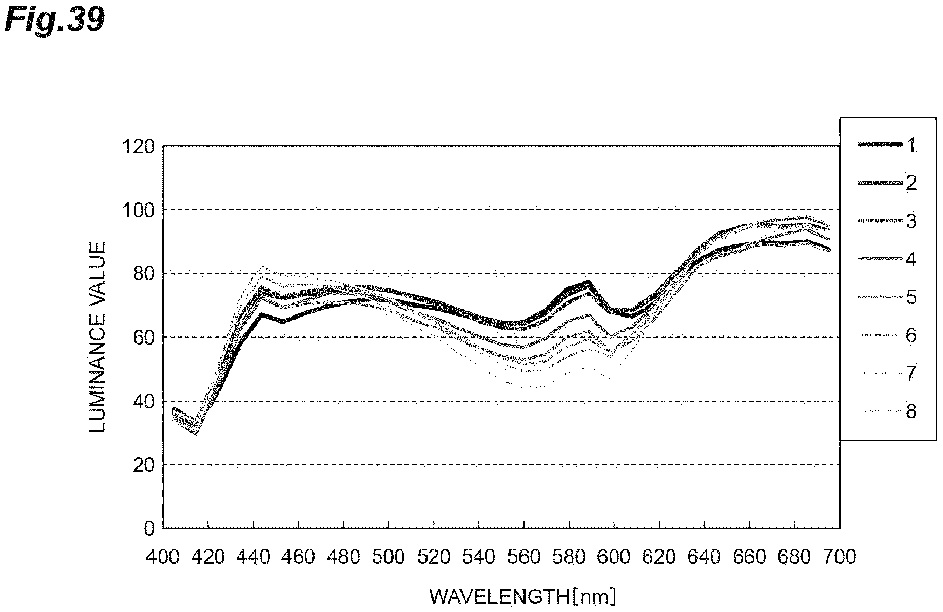

FIG. 36 is a view illustrating a spectrum distribution for every section based on an imaging result of the assessment plate 10 of Sample No. 2. FIG. 37 is a view illustrating a spectrum distribution for every section based on an imaging result of the assessment plate 10 of Sample No. 3. FIG. 38 is a view illustrating a spectrum distribution for every section based on an imaging result of the assessment plate 10 of Sample No. 4. FIG. 39 is a view illustrating a spectrum distribution for every section based on an imaging result of the assessment plate 10 of Sample No. 5. In FIG. 36 to FIG. 39, representation of the abscissa, the ordinate, and lines indicating average spectrums corresponding to the section Se1 to section Se8 is similar to the case of FIG. 35.

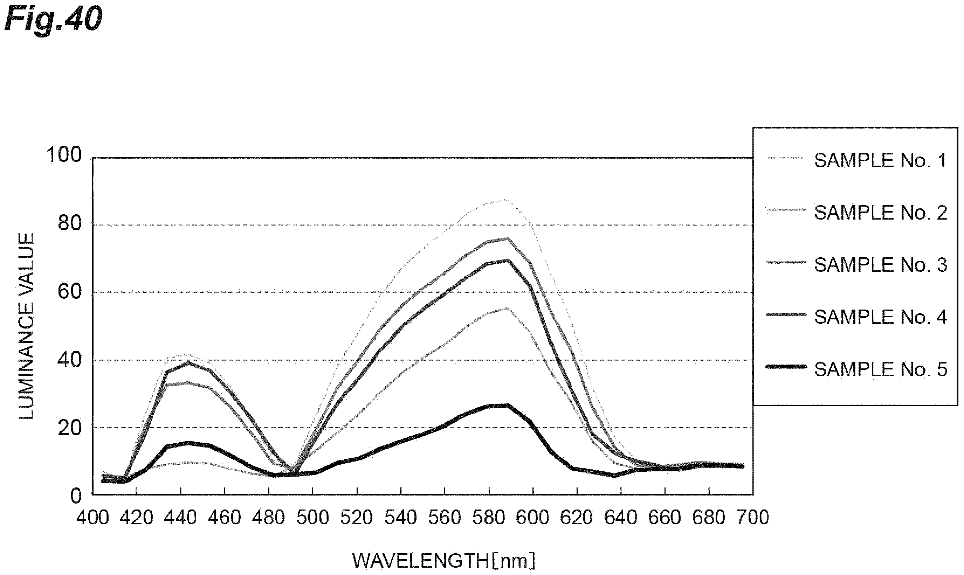

FIG. 40 is a graph illustrating a luminance difference between sections for every wavelength. Specifically, in the results of FIG. 35 to FIG. 39, a maximum value among luminance differences between sections in each wavelength is plotted. A difference in lines indicating results of Sample No. 1 to Sample No. 5 in FIG. 40 is expressed by shading, and a line color becomes darker in the order of Sample No. 4, Sample No. 5, Sample No. 2, Sample No. 3, and Sample No. 1.

As illustrated in FIG. 40, in each of a wavelength range of 430 to 460 nm and a wavelength range of 520 to 620 nm, the luminance difference between sections increases in all of Sample No. 1 to Sample No. 5.

Accordingly, when irradiating the assessment plate with light L having a wavelength within at least one wavelength range in the wavelength range of 430 to 460 nm and the wavelength range of 520 to 620 nm, it is easy to clearly distinguish a color change between sections. As a result, the safety assessment can be more accurately performed.

### (Modification Example 2)

In an irradiation process in the case of capturing an image of the assessment plate 10 in Modification Example 2, the assessment plate is irradiated with light L having a wavelength of 520 nm or greater. Modification Example 2 is similar to the above-described embodiment except for the irradiation. Accordingly, Modification Example 1 has a similar operational effect as in the embodiment.

From FIG. 40 illustrating results in Experimental Example 2 described in Modification Example 1, it can be seen that when the light L has a wavelength of 520 nm or greater, the luminance difference between sections can be increased. Accordingly, when the light L has a wavelength of 520 nm or greater, the difference in color between wells 13 (for example, between the sections Se1 to Se8) different in a state of the assessment target 14 or the like becomes clearer. As a result, the safety assessment can be more accurately performed.

### (Modification Example 3)

In Modification Example 3, in an imaging process of imaging an assessment plate, light having a wavelength within at least one wavelength range between a wavelength range of 430 to 460 nm and a wavelength range of 520 to 620 nm is selectively received. The other configurations are similar as in the above-described embodiment. Accordingly, Modification Example 1 has a similar operational effect as in the embodiment.

In the observation device 30 illustrated in FIG. 3, the imaging process may be performed by receiving light from the assessment plate 10 by the imaging unit 33 through a filter that selectively allows light having a wavelength within at least one wavelength range between a wavelength range of 430 to 460 nm and a wavelength range of 520 to 620 nm to be transmitted.

In Modification Example 3, light having a wavelength within at least one wavelength range between the wavelength range of 430 to 460 nm and the wavelength range of 520 to 620 nm is selectively received. In this case, Modification Example 3 is substantially similar to Modification Example 1 except for the configuration in which a light reception side selects a wavelength. Accordingly, Modification Example 3 has a similar operational effect as in the Modification Example 1.

### (Modification Example 4)

The plate 11 may be a plate that is determined as being appropriate in the following plate determination method. With regard to the plate determination method, the plate determination method will be described with reference to the case of determining the plate 11 illustrated in FIG. 1 as an example. Foreign matters described later in Modification Example 4 are substances (structures) which occur due to death of the cells described in the embodiment.

### (Plate Determination Method)

After an assessment sample including a tester and a test substance selected and adjusted so that the foreign matters occur is injected into a plurality of wells 13 provided in the plate 11 to be assessed (corresponding to wells (assessment wells) to be assessed in the determination method), in a case where at least one well 13 holding the foreign matters is present after passage of constant time from the injection, the plate is determined as being appropriate.

The timing of counting after passage of the constant time (hereinafter, referred to as "counting timing") is time capable of securing reliable occurrence of the foreign matters. For example, in the case of assessing presence or absence of the foreign matters in the Ames test, the timing may be timing of performing the assessment. The counting timing may be different depending on the test, for example. For example, the counting timing may be first counting timing in a certain test, and may be second counting timing after passage of predetermined time from the first counting timing in another test.

An example of the first counting timing is after the test substance and the tester are injected into the plate 11 and cultured for 48 hours as in the 11A^{th} process. For example, the counting timing is within 12, and preferably within three hours, from the end of 48 hours of culture after injection of the assessment sample into the plurality of wells 13. For example, the second counting timing may be a timing after the test substance and tester are injected into the plate 11 as in the 11A^{th} process, the plate 11 is retained at a constant temperature (for example, 10°C or lower) and a predetermined time elapses. For example, the predetermined time may be after one day, after two days, after three days, after four days, or after five days from initiation of retention.

In the plate determination method, the tester is a cell. The assessment sample is a combination of cells and a test substance for which foreign matters are known to occur in advance, and is a sample in which a concentration of the test substances is a concentration at which the foreign matters occur.

An example of the cells employed in the assessment sample is a bacteria. Examples of the bacteria employed in the assessment sample include Escherichia coli (for example, Escherichia coli WP2uvrA strain) or Salmonella typhimurium (for example, Salmonella typhimurium TA100 strain). Examples of the test substance employed in the assessment sample include tert-butylhydroquinone, 1-decanol, 2-ethoxynaphthalene, 4-(1,1,3,3-tetramethylbutyl)phenol, diethyl chlorothiophosphate, or 4-chlorobenzyl chloride. For example, the test substance may be 1-decanol in which a concentration is 62.5 µg/mL or greater, or tert-butylhydroquinone in which a concentration is 667 µg/mL or greater.

Specific examples of the assessment sample include a first assessment sample to a fourth assessment sample described below.

### (First Assessment Sample)

The first assessment sample is an assessment sample employing Escherichia coli WP2uvrA strain as the cells, and 1-decanol in which a concentration is 62.5 µg/mL as the test substance.

### (Second Assessment Sample)

The second assessment sample is an assessment sample employing Escherichia coli WP2uvrA strain as the cells, and 1-decanol in which a concentration is 125 µg/mL as the test substance.

### (Third Assessment Sample)

The third assessment sample is an assessment sample employing Salmonella typhimurium TA100 strain as the cells, and 1-decanol in which a concentration is 667 µg/mL as the test substance.

### (Fourth Assessment Sample)

The fourth assessment sample is an assessment sample employing Salmonella typhimurium TA100 strain as the cells, and 1-decanol in which a concentration is 2000 µg/mL as the test substance.

In the plate determination method, culture of the assessment sample is performed, for example, at 37°C for 48 hours.

In the plate determination method, in order to appropriately determine the plate, the number of wells 13 into which the assessment sample is injected is preferably at least 12, and more preferably 48.

An example of a specific procedure of the plate determination method will be described.

### (1B^{th} process)

A similar process as in the 2A^{th} process is performed except that cells used in the assessment sample of the plate 11 are used as the tester that is used. Specifically, cells for an assessment sample are inoculated into 60 mL of Nutrient broth medium, and are shaken and cultured at 37°C for 9 to 10 hours to be proliferated.

### (2B^{th} Process)

A similar process as in the 1B^{th} process is performed except that a test substance used in the assessment sample of the plate 11 is used as the test substance that is used. Specifically, a necessary amount of test substance for an assessment sample is weighed.

### (3B^{th} Process)

A test substance solution is prepared by dissolving or suspending the test substance in the 2B^{th} process in an appropriate medium so that a concentration of the test substances becomes 25 times a concentration exerting cytotoxicity.

### (4B^{th} Process)

The test substance solution obtained in the 3B^{th} process is put into each of wells of 24-well plate by 10 µL.

### (5B^{th} Process)

In a similar manner as in the 6A^{th} process, a preparation bacterial solution under conditions of the absence of metabolic activation is prepared. Specifically, the preparation bacterial solution under conditions of the absence of metabolic activation is prepared by mixing 0.175 to 0.7 mL of cell culture solution (typically, a concentration exceeding 1×10⁹ cells/mL), and 6.83 to 6.3 mL of medium (minimum medium to which a carbon source is added and which is deficient in amino acid).

### (6B^{th} Process)

240 µL of the preparation bacterial solution under conditions of the absence of metabolic activation which has been prepared in the 5B^{th} process is added to the 24-well plate into which 10 µL of test substance solution or the like has been put.

### (7B^{th} Process)

The 24-well plate into which the mixed solution prepared in the 6B^{th} process is shaken at 37°C and 250 rpm for 90 minutes (pre-incubation).

### (8B^{th} Process)

2.6 mL of indicator medium (minimum medium that contains a pH indicator and a carbon source and is deficient in amino acid) is added to each well (250 µL) of the 24-well plate after termination of the pre-incubation.

### (9B^{th} Process)

The mixed solution obtained in the 8B^{th} process is transferred to 48 wells (corresponding to one section) of the 384-well plate (plate 11) by 50 µL.

### (10B^{th} Process)

the 384-well plate (plate 11) is cultured at 37°C for 48 hours.

### (11B^{th} Process)

In the plate 11 for which culture is terminated, presence or absence of foreign matters in the 48 wells 13 into which the mixed solution has been injected in the 9B^{th} process is observed, and the number of wells 13 in which the foreign matters are present is counted to confirm whether or not at least one well 13 in which the foreign matters are present.

In a plate 11 that is assessed as being appropriate by performing the plate determination method, the foreign matters can be held up to the counting timing after passage of the constant time. Accordingly, in the case of performing the assessment method in the above-described Ames test by using the plate 11 assessed as being appropriate, toxicity of the test substance for cells can be accurately assessed by assessing the assessment plate 10 at the counting timing after passage of the constant time.

Next, Experimental Example 3 to Experimental Example 8 relating to Modification Example 3 will be described. In Experimental Example 3 to Experimental Example 8, the following six plate A to plate H were assessed by the plate determination method. The plate A to the plate H have the same configuration except for a material and a coating state described below, and include 384 wells in a similar manner as in the plate 11 illustrated in FIG. 1.

### (Plate A)

A material of the plate A was polystyrene. With respect to the plate A, a tissue culture (TC) treatment was performed on a surface (including an inner surface and a bottom surface of the well 13).

### (Plate B)

A material of a plate B was polystyrene. Surface coating was not performed on the plate B. That is, the plate B was a polystyrene plate that was not subjected to surface coating.

### (Plate C)

A plate C included a glass plate, and was configured by joining a structure including 384 wells 13 onto the glass plate. A material of the structure was a cyclic olefin copolymer (COC). Accordingly, a material of a bottom surface of each of the wells 13 provided in the plate C was glass, and a material of a wall surface was COC. In the plate C, the bottom surface of the wells 13 was coated with fibronectin.

### (Plate D)

A material of the plate D was polystyrene. A surface of the plate D was coated with Poly-D-Lysine.

### (Plate E)

A plate E included a glass plate and was configured by joining a structure including 384 wells 13 onto the glass plate. A material of the structure was COC. A material of a bottom surface of each of the wells 13 provided in the plate E was glass, and a material of a wall surface was COC. The plate E was not subjected to surface coating.

### (Plate F)

A material of the plate F was a cycloolefin polymer. The plate F was not subjected to surface coating. That is, the plate F was a cycloolefin polymer plate that was not subjected to surface coating.

### (Plate G)

The plate G included a glass plate, and was configured by joining a structure including 384 wells 13 onto the glass plate. Amaterial of the structure was polystyrene. Accordingly, a material of a bottom surface of each of the wells 13 provided in the plate G was glass, and a material of a wall surface was polystyrene. The plate G was not subjected to surface coating.

### (Plate H)

A material of the plate H was COC. A tissue culture (TC) treatment was performed on a surface.

Cells used in Experimental Example 3 to Experimental Example 8 were cells available from National Institute of Health Sciences, National Institute of Technology and Evaluation (NITE) biotechnology center, American Type Culture Collection, Ames test kit ("Ames MPF^{™} Penta I Microplate Format Mutagenicity Assay") manufactured by Xenometrix, and the like. The test substance used in Experimental Example 3 to Experimental Example 8 was a test substance manufactured by Tokyo Chemical Industry Co., Ltd.

### (Experimental Example 3)

In Experimental Example 3, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate B, and the plate C as described in the 9B^{th} process, and the 10B^{th} process and the 11B^{th} process were performed.

### <Cells and Test substance for Assessment Sample in Experimental Example 3>

Cells: Escherichia coli WP2uvrA strain
Test substance: 1-decanol

The medium used in the 3B^{th} process in Experimental Example 3 was DMSO. In the 5B^{th} process, 0.7 mL of cells was mixed to 6.3 mL of medium. The concentration in the 6B^{th} process was 62.5 µg/mL.

In Experimental Example 3, after counting the number of wells in which foreign matters are present by visually observing presence or absence of the foreign matters in the wells 13 in the 11B^{th} process, the plate 11 was maintained at 4°C, and the number of wells in which the foreign matters were present was counted by visually observing presence or absence of the foreign matters in the wells 13 after one day, after two days, after three days, after four days, and after five days, respectively. Results in Experimental Example 3 were as illustrated in Table 6. In Table 6, "immediately after culture termination" represents that visual observation of the foreign matters was performed at a point of time at which culture for 48 hours was terminated.

**[Table 6]**

| Plate | Immediately after culture termination | After one day | After two days | After three days | After four days | After five days |
|---|---|---|---|---|---|---|
| Plate A | 0 | 0 | 0 | 0 | 0 | 0 |
| Plate B | 11 | 0 | 0 | 0 | 0 | 0 |
| Plate C | 18 | 17 | 17 | 17 | 17 | 17 |

### (Experimental Example 4)

In Experimental Example 4, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate B, and the plate C as described in the 9B^{th} process, and the 10B^{th} process and the 11B^{th} process were performed.

### <Cells and Test substance for Assessment Sample in Experimental Example 4>

Cells: Escherichia coli WP2uvrA strain
Test substance: 1-decanol

The medium used in the 3B^{th} process in Experimental Example 4 was DMSO. In the 5B^{th} process, 0.7 mL of cells was mixed to 6.3 mL of medium. The concentration in the 6B^{th} process was 125 µg/mL.

In Experimental Example 4, the presence or absence of the foreign matters in each well 13 was visually observed in the 11B^{th} process, and the number of wells in which foreign matters were present was count. Thereafter, the plate 11 was maintained at 4°C, and the presence or absence of foreign matters in each well 13 was visually observed and the number of wells in which the foreign matters were present was counted after one day, after two days, three days, after four days, and after five days, respectively. Results in Experimental Example 4 were as illustrated in Table 7. In Table 7, "immediately after culture termination" is similar as in the case of Table 6.

**[Table 7]**

| Plate | Immediately after culture termination | After one day | After two days | After three days | After four days | After five days |
|---|---|---|---|---|---|---|
| Plate A | 0 | 0 | 0 | 0 | 0 | 0 |
| Plate B | 15 | 0 | 0 | 0 | 0 | 0 |
| Plate C | 18 | 18 | 18 | 18 | 18 | 18 |

### (Experimental Example 5)

In Experimental Example 5, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate B, and the plate C as described in the 9B^{th} process, and the 10B^{th} process and the 11B^{th} process were performed.

### <Cells and Test substance for Assessment Sample in Experimental Example 5>

Cells: Salmonella typhimurium TA100 strain
Test substance: 1-decanol

The medium used in the 3B^{th} process in Experimental Example 5 was DMSO. In the 5B^{th} process, 0.35 mL of cells was mixed to 6.65 mL of medium. The concentration in the 6B^{th} process was 667 µg/mL.

In Experimental Example 5, after counting the number of wells in which foreign matters were present by visually observing presence or absence of the foreign matters in the wells 13 in the 11B^{th} process, the plate 11 was maintained at 4°C, and the number of wells in which the foreign matters were present was counted by visually observing presence or absence of the foreign matters in the wells 13 after one day, after two days, after three days, after four days, and after five days, respectively. Results in Experimental Example 5 were as illustrated in Table 8. In Table 8, "immediately after culture termination" is similar as in the case of Table 6.

**[Table 8]**

| Plate | Immediately after culture termination | After one day | After two days | After three days | After four days | After five days |
|---|---|---|---|---|---|---|
| Plate A | 0 | 0 | 0 | 0 | 0 | 0 |
| Plate B | 21 | 0 | 0 | 0 | 0 | 0 |
| Plate C | 23 | 23 | 23 | 23 | 23 | 23 |

### (Experimental Example 6)

In Experimental Example 6, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate B, and the plate C as described in the 9B^{th} process, and the 10B^{th} process and the 11B^{th} process were performed.

### <Cells and Test substance for Assessment Sample in Experimental Example 6>

Cells: Salmonella typhimurium TA100 strain
Test substance: 1-decanol

The medium used in the 3B^{th} process in Experimental Example 6 was DMSO. In the 5B^{th} process, 0.35 mL of cells was mixed to 6.65 mL of medium. The concentration in the 6B^{th} process was 2000 µg/mL.

In Experimental Example 6, after counting the number of wells in which foreign matters were present by visually observing presence or absence of the foreign matters in the wells 13 in the 11B^{th} process, the plate 11 was maintained at 4°C, and the number of wells in which the foreign matters were present was counted by visually observing presence or absence of the foreign matters in the wells 13 after one day, after two days, after three days, after four days, and after five days, respectively. Results in Experimental Example 6 were as illustrated in Table 9. In Table 9, "immediately after culture termination" is similar as in the case of Table 6.

**[Table 9]**

| Plate | Immediately after culture termination | After one day | After two days | After three days | After four days | After five days |
|---|---|---|---|---|---|---|
| Plate A | 0 | 0 | 0 | 0 | 0 | 0 |
| Plate B | 27 | 0 | 0 | 0 | 0 | 0 |
| Plate C | 24 | 24 | 24 | 24 | 24 | 24 |

### (Experimental Example 7)

In Experimental Example 7, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate D, and the plate E as described in the 9B^{th} process, and the 10B^{th} process and the 11B^{th} process were performed.

### <Cells and Test substance for Assessment Sample in Experimental Example 7>

Cells: Salmonella typhimurium TA98 strain
Test substance: tert-butylhydroquinone

The medium used in the 3B^{th} process in Experimental Example 7 was DMSO. In addition, the concentration in the 3B^{th} process was 2500 µg/mL. In the 5B^{th} process, 0.7 mL of cells was mixed to 6.3 mL of medium. The concentration in the 6B^{th} process was 2500 µg/mL.

In Experimental Example 7, the number of wells in which foreign matters were present was counted by visually observing presence or absence of foreign matters in the wells 13 in the 11B^{th} process. Results in Experimental Example 7 were as shown in Table 10. Table 10 shows the number of wells confirmed the foreign matters among 48 wells 13. In Table 10, "immediately after culture termination" is similar as in the case of Table 6.

**[Table 10]**

| Plate | Immediately after culture termination |
|---|---|
| Plate A | 0 |
| Plate D | 48 |
| Plate E | 42 |

### (Experimental Example 8)

In Experimental Example 8, the 1B^{th} process to the 8B^{th} process were performed by using the following cells and test substance as cells and a test substance for an assessment sample. Then, in the 9B^{th} process, the mixed solution prepared in the 8B^{th} process was transferred to the plate A, the plate F, the plate G, and the plate H as described in the 9B^{th} process, and the 10B^{th} process and the 11B^{th} process were performed.

### <Cells and Test substance for Assessment Sample in Experimental Example 8>

Cells: Escherichia coli WP2uvrA strain
Test substance: 1-decanol

The medium used in the 3B^{th} process in Experimental Example 8 was DMSO. In the 5B^{th} process, 0.7 mL of cells was mixed to 6.3 mL of medium. The concentration in the 6B^{th} process was 125 µg/mL.

In Experimental Example 8, the number of wells in which foreign matters were present was counted by visually observing presence or absence of foreign matters in the wells 13 in the 11B^{th} process. Results in Experimental Example 8 were as shown in Table 11. Table 11 shows the number of wells confirmed the foreign matters among 48 wells 13. In Table 11, "immediately after culture termination" is similar as in the case of Table 6.

**[Table 11]**

| Plate | Immediately after culture termination |
|---|---|
| Plate A | 0 |
| Plate F | 5 |
| Plate G | 18 |
| Plate H | 48 |

### (Assessment of Experimental Example 3 to Experimental Example 8)

In Experimental Example 3 to Experimental Example 8, when performing counting immediately after culture termination, a well holding foreign matters were present in the plate B, the plate C, the plate D, the plate E, the plate F, the plate G, and the plate H. Accordingly, it can be understood that the combination of the cells and the test substance used in Experimental Example 3 to Experimental Example 8, and the concentration of the test substances are conditions causing foreign matters to occur, and the Plate B, the plate C, the plate D, the plate E, the plate F, the plate G, and the plate H are plates capable of holding foreign matters immediately after culture termination. On the other hand, a well 13 holding foreign matters is not present in the plate A immediately after culture termination in Experimental Example 3 to Experimental Example 8. Accordingly, the plate A is a plate that cannot hold foreign matters.

Accordingly, with respect the cytotoxicity assessment test of determining presence or absence of foreign matters immediately after culture, the plate B to the plate H can be assessed as an appropriate plate (that is, a plate capable of appropriately assessing presence or absence of foreign matters), and the plate A can be assessed as a plate that cannot appropriately assess presence or absence of foreign matters.

From the counting results after one day, two days, three days, four days, and five days in Experimental Example 3 to Experimental Example 6, as in the case with immediately after culture, it can be seen that after one day, after two days, after three days, after four days, and after five days, with respect to the cytotoxicity assessment test of determining presence or absence of foreign matters, the plate C is an appropriate plate, that is, a plate capable of appropriately assessing presence or absence of foreign matters. On the other hand, a well 13 holding foreign matters after one day was not present in the plate A and the plate B. Accordingly, in the case of assessing presence or absence of foreign matters after one day, the plate A and the plate B can be assessed as a plate that cannot appropriately assess presence or absence of foreign matters.

With regard results immediately after culture, the same results are obtained in Experimental Example 3 to Experimental Example 8. Accordingly, for example, in a case where the assessment sample (the cells, the test substance, and the concentration of the test substances which are employed in Experimental Example 3) in Experimental Example 3 is set as a reference sample for plate assessment, it can be understood that a plate can be appropriately assessed even in the case of another combination of cells, a test substance, and a concentration of the test substances as in Experimental Example 4 to Experimental Example 8 when using the reference sample.

This is also true of a case where the assessment sample (the cells, the test substance, and the concentration of the test substances which are employed in Experimental Example 4 to Experimental Example 8) in each of Experimental Example 4 to Experimental Example 8 is set as the reference sample for plate assessment. That is, in a case where the assessment sample in each of Experimental Example 4 to Experimental Example 8 is set as a reference sample, it can be seen that a plate assessed as being appropriate with the reference sample can be appropriately assessed even in the case of a combination of cells, a test substance, and a concentration of the test substances which are employed in experimental examples other than experimental examples employed the reference sample among Experimental Examples 3 to Experimental Example 8.

Accordingly, it was verified that the assessment sample employed in Experimental Example 3 to Experimental Example 8 can be used as an assessment sample for assessing a plate immediately after culture.

This is also true of a case where counting timing is after one day, after two days, after three days, after four days, and after five days. That is, it was verified that the assessment sample employed in each of Experimental Example 3 to Experimental Example 6 can be used as an assessment sample for assessing a plate in a case where each of after one day, after two days, after three days, after four days, and after five days is the counting timing.

Hereinbefore, the experimental examples of the invention have been described in combination with various embodiments and modification examples of the invention. However, the invention is not limited to the exemplified various embodiments, modification examples, and the experimental examples, and is intended to include the scope represented by the appended claims, and all modifications within equivalent meaning and scope as in the appended claims.

For example, in the flowchart illustrated in FIG. 11, the color determination is repeated three times, but the color determination may be performed at least once. For example, the results in the first determination process in the flowchart illustrated in FIG. 11 may be set as final determination. The color of all well images (corresponding to the color of wells) may be finally determined on the basis of similarity between first reference statistical information (for example, a frequency distribution) for each of purple and yellow which is created in the first reference creation process illustrated in the flowchart in FIG. 11, and statistical information (for example, a frequency distribution) of all well images in a plate image.

Color candidates used in the color determination or the like are not limited to purple and yellow. The color candidates may be other two colors, or three or more colors may be employed as the color candidates in accordance with test contents. In addition, the invention is applicable as long as a color feature quantity (for example, hue and saturation) in an image acquired by the imaging unit changes even though the assessment plate, for example, the wells is visually colorless (or the assessment target is colorless). For example, as long as the color feature quantity (for example, hue and saturation) in the image acquired by the imaging unit changes through irradiation with visible light or light having a wavelength other than the visible light, color candidates may be selected from false colors temporarily assigned in the image acquired by the imaging unit. In this case, an image corresponding to an assessment plate obtained by assigning a false color functions as a plate image.

As at least one color feature quantity used in the color determination, luminosity, luminance, gradation value, or the like other than hue and saturation may be used alone or in combination. In addition, a gradation value of R, a gradation value of G, and a gradation value of B in an RGB image may be treated as individual color feature quantities. In addition, with regard to the color feature quantity that is used, three or more color feature quantities may be selected from the above described quantities and be used instead of two quantities.

The statistical information used in the color determination includes at least one kind of statistical quantity. As the statistical quantity, an average value, a central value, or the like of color feature quantities of the entire assessment region may be used other than the frequency distribution. In addition, the statistical quantity may be a maximum value, a minimum value, a deviation (for example, standard deviation), or the like of the color feature quantities in the assessment region.

For example, a gradation value may be employed as the color feature quantity, and the average value and the standard deviation (two kinds of statistical quantities) may be employed as the statistical information.

An example of a color determination method in this case will be described. First, an average value and a standard deviation of respective gradation values of RGB in all pixels included in each of a plurality of well images (assessment region) are acquired. According to this, a set of the average value and the standard deviation is acquired as statistical information for each of the plurality of well images. The color determination may be performed in a similar manner as in the case of using the frequency distribution by using the statistical information (the set of the average value and the standard deviation) for each of the plurality of well images acquired in this manner instead of the frequency distribution. For example, the similarity between the statistical information for the plurality of well images and reference statistical information of yellow and purple is calculated. The color of the plurality of wells is determined on the basis of the calculated similarity. The reference statistical information of yellow and purple can be acquired in a similar manner as in the case of creating the third reference frequency distribution described with regard to the second color determination method using the frequency distribution. For example, an average value and a standard deviation of respective gradation values of RGB of all pixels included in all well images corresponding to all wells visually determined as yellow may be as yellow reference statistical information. This is also true of purple reference statistical information. Here, description has been given of a case where the statistical information includes two statistical quantities (the average value and the standard deviation), but for example, one of the average value and the standard deviation may be used as the statistical information. In the above description, the average value and the standard deviation of the gradation values were employed, but for example, other color feature quantities such as hue and saturation can be employed instead of the gradation values.

In the colony detection process and the foreign matter detection process, the smoothing process may not be performed. In the colony detection process and the foreign matter detection process, the detection result creation process may not be performed. Alternatively, in the colony detection process, the process performed in the detection result creation process may be performed in the second colony detection process illustrated in FIG. 18 (for example, may be performed by the second colony detection unit). Similarly, in the foreign matter detection process, the process performed in the detection result creation process may be performed in the second foreign matter detection process illustrated in FIG. 24 (for example, may be performed by the second foreign matter detection unit).

In the foreign matter detection process, the outer region setting process and the second foreign matter detection process illustrated in FIG. 24 may not be performed. However, as described above, when performing the outer region setting process and the second foreign matter detection process, foreign matters in the outer region can be more reliably detected.

In the color determination process and the transparency detection process using the statistical information (for example, the frequency distribution), for example, a well image corresponding to each well was set as an assessment region, and the statistical information was acquired for each of a plurality well images (a plurality of the assessment regions). However, the assessment region may include at least one well image. Accordingly, a well image group including a plurality of well images may be set as the assessment region, and the statistical information may be acquired for the well image group. For example, in the sections Se1 to Se8 illustrated in FIG. 1, conditions of an assessment target in each of the sections Se1 to Se8 are the same in each case. Accordingly, one piece of statistical information may be created by setting the section images Se1p to Se8p corresponding to the sections Se1 to Se8 as the assessment region, and the color determination process and the transparency detection process may be performed by using the similarity between the statistical information for the plurality of section images.

In the transparency change detection process, statistical information (for example, section frequency distribution) of a section image (solvent control region) corresponding to solvent control section was set as a reference, and the transparency change was detected. However, the transparency change may be detected by using reference statistical information (for example, a frequency distribution) for the transparency. In a case where a plurality of wells include at least one solvent control well, the solvent control region is a region including at least one solvent control well image corresponding to an image of the at least one solvent control well.

The tester is not limited to cells. The test substance is not limited to a chemical material. The tester and the test substance may be a combination in which at least a color change (including a color change in the case of using an indicator) occurs in the combination. Another example of the tester is an antibody, and another example of the test substance is an antigen.

In the embodiment, a colony is detected as a shape change of the tester of an assessment target, but there is no limitation to the colony in a case where a shape of the assessment target changes due to a test result. The foreign matter is a structure different from the shape change of the tester and may be, for example, a substance (or structure) which is not initially included, and for example, has a clear boundary between the contour and the inside. For example, the foreign matter is a substance (or structure) separated at an interface having a refractive index different from that of the periphery of the foreign matter. Examples of the foreign matter include oil clots, air bubbles, and the like.

In the above description, the assessment method performs at least the color determination process. However, the invention also relates to an assessment method of performing at least the transparency change detection process. Even in this case, in the case of performing the transparency change detection process, the transparency change may be detected by using reference statistical information for transparency (for example, by using the frequency distribution).

The present disclosure may include an assessment method including a process of detecting presence or absence of foreign matters in a plurality of wells (hereinafter, referred to as "assessment method based on foreign matter detection" for convenience of description). In the assessment method based on the foreign matter detection, the color determination process may not be performed. In the "process of detecting presence or absence of foreign matters in a plurality of wells" in the assessment method based of the foreign matter detection, presence or absence of foreign matters may be visually detected, or may be detected on the basis of the well image as described in the embodiment.

In the case of detecting presence or absence of foreign matters in a plurality of wells on the basis of the well image, the assessment method based on the foreign matter detection includes,
a process of detecting presence or absence of foreign matters in a plurality of wells on the basis of a well image,
the process of detecting presence or absence of foreign matters in the plurality of wells includes,
a process of detecting presence or absence of the foreign matters on the basis of a first luminance threshold value that is set to each of the plurality of well images, and
a process of detecting presence or absence of the foreign matters in an outer region on the basis of a second luminance threshold value set in the outer region that surrounds an inner region corresponding to a bottom region of a well when viewed from a depth direction of the well in each of the plurality of well image,
the first luminance threshold value and the second luminance threshold value are different from each other, and
an area of the bottom in each of the plurality of wells is smaller than an area of opening opposite to the bottom.

As an aspect of the assessment method that is based on the foreign matter detection and includes the process of detecting presence or absence of the foreign matters in the plurality of wells on the basis of the well image, there may be exemplified an aspect in which the foreign matter detection process is performed with respect to an image obtained by imaging the assessment plate and an assessment target is assessed on the basis of the process result in the above-described embodiment. In the assessment method based on the foreign matter detection, the color determination process may not be performed. In addition, in the case of detecting presence or absence of foreign matters in the plurality of wells on the basis of the well image, presence or absence of foreign matters may be detected by visually observing the well image (image obtained by imaging the assessment plate).

The plate may be a plate in which a bottom surface and a wall surface of a wall are constituted by the same material, or may be a plate in which the bottom surface and the wall surface of the wall are constituted by different materials.

In the assessment method based on the foreign matter detection, it is preferable to use a plate that is determined as being appropriate in the plate determination method described in Modification Example 4. According to this, foreign matters can be more accurately detected.

Examples of the plate determined as being appropriate in the plate determination method include a polystyrene plate whose surface is coated with Poly-D-Lysine, a cyclic olefin copolymer plate whose surface is subjected to a tissue culture (TC) treatment, a cycloolefin copolymer plate in which surface coating is not performed, and the like.

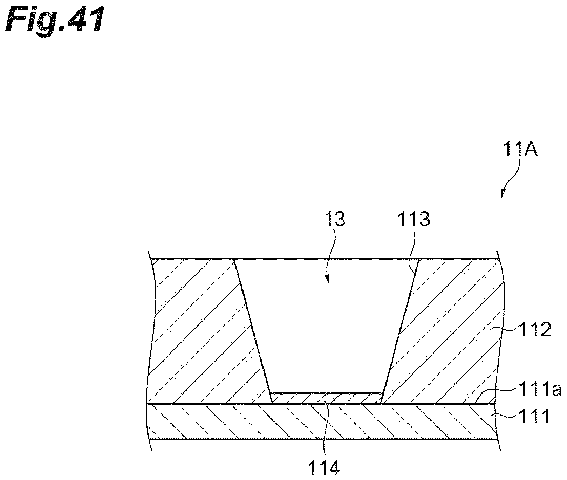

The plate determined as being appropriate in the plate determination method may be a plate 11A illustrated in FIG. 41. The plate 11A includes a glass plate 111, and a resin layer 112 provided on a surface 111a of the glass plate 111. A plurality of through-holes 113 corresponding to a plurality of wells 13 are formed in the resin layer 112. The wells 13 are formed by regions exposed from the through-holes 113 in the glass plate 111 and the through-holes 113. The resin layer 112 is cyclic olefin copolymer or polystyrene. In the plate 11A, a coating layer 114 is formed at least in a region corresponding to a bottom surface of the wells 13 (a region exposed from the through-holes 113 in the glass plate 111). A material of the coating layer 114 is fibronectin. The plate 11A illustrated in FIG. 41 corresponds to the plate C used in the experimental example described in Modification Example 4. Accordingly, the plate 11A is a plate suitable for an assessment method based on the foreign matter detection. The material of the coating layer 114 may be fibronectin, collagen, laminin, or the like. FIG. 41 illustrates an example of a plate including the coating layer 114. However, in the plate 11A, coating may not be performed in a region exposed from the through-holes 113 in the glass plate 111.

Hereinbefore, description has been made by using the Ames test, but the invention is also applicable to another biological safety assessment. For example, the invention is preferably applicable in a case where a color change (including a transparency change) of an assessment target or a shape change of a tester of the assessment target occurs in the safety test. As described above, the color change of the assessment target also includes a color change in a case where a change occurs in an image obtained by an imaging unit due to illumination light for imaging the assessment plate.

The embodiment, the modification examples, and the like which have been described above may be appropriately combined in a range not departing from the gist of the invention.

### Reference Signs List

10: assessment plate, 20: assessment system, 13: well, 13a: bottom, 14, 14a to 14h: assessment target, 33: imaging unit, 40: image processing device, IA: inner region, OA: outer region, PI: plate image, 13p: well image, Se1 to Se8: section (first to N^{th} sections), Se1p: section image (solvent control region).

## Claims

1. A method for assessing an assessment target, comprising:
a step of acquiring statistical information based on at least one color feature quantity with respect to each of a plurality of assessment regions in a plate image corresponding to an image of an assessment plate that holds an assessment target in a plurality of wells provided in the plate; and
a step of determining a color of the plurality of assessment regions by using the statistical information,
wherein the assessment target includes a tester,
the plurality of wells include a test substance well holding the assessment target that further includes a test substance,
the plate image includes a plurality of well images corresponding to the plurality of wells, and
each of the plurality of assessment regions includes at least one well image corresponding to at least one of the wells.

2. The method for assessing an assessment target according to claim 1,
wherein the at least one color feature quantity is two or more color feature quantities.

3. The method for assessing an assessment target according to claim 2,
wherein the two or more color feature quantities include hue and saturation.

4. The method for assessing an assessment target according to claim 2 or 3,
wherein the statistical information is a frequency distribution in which the two or more color feature quantities are variables.

5. The method for assessing an assessment target according to claim 1,
wherein the at least one color feature quantity is one color feature quantity, and
the statistical information includes at least one kind of statistical quantity for the one color feature quantity.

6. The method for assessing an assessment target according to any one of claims 1 to 5,
wherein in the step of determining the color of the plurality of assessment regions, the color of the plurality of assessment regions is determined based on a similarity of the statistical information.

7. The method for assessing an assessment target according to claim 6,
wherein the step of determining the color of the plurality of assessment regions includes,
a step of performing a primary determination of the color of the plurality of assessment regions among a plurality of color candidates based on the similarity of the statistical information,
a step of acquiring first reference statistical information for the at least one color feature quantity with respect to the plurality of color candidates based on a result of the primary determination, and
a step of performing a final determination of the color of the plurality of assessment regions by using a similarity between the first reference statistical information and the statistical information, and
in the step of acquiring the first reference statistical information, the first reference statistical information is acquired based on a statistical information of at least one assessment region determined by each color in the result of the primary determination among the statistical information for the plurality of assessment regions.

8. The method for assessing an assessment target according to claim 6,
wherein the plurality of assessment regions are the plurality of well images,
the step of determining the color of the plurality of assessment regions includes,
a step of performing a primary determination of a color of the plurality of well images among a plurality of color candidates based on the similarity of the statistical information,
a step of acquiring a first reference statistical information for the at least one color feature quantity with respect to the plurality of color candidates based on a result of the primary determination,
a step of performing a secondary determination of a color of the plurality of well images among the plurality of color candidates by using a similarity between the statistical information and the first reference statistical information,
a step of acquiring a second reference statistical information for the at least one color feature quantity with respect to the plurality of color candidates based on a result of the secondary determination, and
a step of performing a tertiary determination of a color of the plurality of well images by using the second reference statistical information,
first to N^{th} sections (N is an integer of two or greater) including at least one well among the plurality of wells are set in the plate,
in the step of acquiring the second reference statistical information, in an i^{th} section image among first to N^{th} section images corresponding to the first to N^{th} sections in the plate image, the second reference statistical information for the plurality of color candidates is acquired based on a statistical information of at least one well image determined as the same color candidate among the statistical information for the plurality of well images,
in the step of performing the tertiary determination of the color of the plurality of well images, the color of the plurality of well images is finally determined by determining a color of a well image pertaining to the i^{th} section among the plurality of well images based on similarity of the second reference statistical information acquired for the i^{th} section image, and statistical information of at least one well pertaining to the i^{th} section image among the statistical information for the plurality of well images.

9. The method for assessing an assessment target according to any one of claims 1 to 5,
wherein in the step of determining the color of the plurality of assessment regions, the color of the plurality of assessment regions is determined based on a similarity between the statistical information and a reference statistical information that is reference statistical information for each of a plurality of color candidates and is based on the at least one color feature quantity.

10. The method for assessing an assessment target according to any one of claims 1 to 9, further comprising:
a step of detecting a transparency change of the plurality of assessment regions based on a similarity between the statistical information and a reference statistical information for detecting a transparency change based on the at least one color feature quantity.

11. The method for assessing an assessment target according to claim 10,
wherein the plurality of wells further include at least one a solvent control well for solvent control,
one of the plurality of assessment regions is a solvent control region including at least one solvent control well image corresponding to the at least one solvent control well, and
in the step of detecting the transparency change of the plurality of assessment regions,
a statistical information of the solvent control region among the statistical information for the plurality of assessment regions is set as a reference statistical information for detecting a transparency change, and a transparency change of the plurality of assessment regions is detected based on a similarity between the statistical information of the solvent control region and a remaining statistical information among the statistical information for the plurality of assessment regions.

12. The method for assessing an assessment target according to any one of claims 1 to 11, further comprising:
a step of detecting presence or absence of a shape change of the tester of the assessment target held in the plurality of wells based on the plurality of well images.

13. The method for assessing an assessment target according to any one of claims 1 to 12, further comprising:
a step of detecting presence or absence of a foreign matter in the plurality of wells based on the plurality of well images.

14. The method for assessing an assessment target according to claim 13,
wherein the step of detecting presence or absence of the foreign matter in the plurality of wells includes,
a step of detecting presence or absence of the foreign matter based on a first luminance threshold value set to each of the plurality of well images, and
a step of detecting presence or absence of the foreign matter in an outer region based on a second luminance threshold value set in the outer region that surrounds an inner region corresponding to a bottom region of a well when viewed from a depth direction of the well in each of the plurality of well image,
the first luminance threshold value and the second luminance threshold value are different from each other, and
an area of the bottom in each of the plurality of wells is smaller than an area of opening opposite to the bottom.

15. The method for assessing an assessment target according to claim 13 or 14,
wherein the plate is a plate that is determined to be appropriate by a plate assessing method in which the plate is determined to be appropriate in a case at least one assessment well among a plurality of assessment wells holds the foreign matter after a constant time has elapsed since an assessment sample including the tester and the test substance selected and adjusted so that the foreign matter occur is injected into a plurality of assessment wells provided in a plate to be assessed.

16. The method for assessing an assessment target according to claim 15,
wherein the plate is,
a polystyrene plate with no surface coating,
a polystyrene plate in which a surface is coated with Poly-D-Lysine,
a cyclic olefin copolymer plate in which a tissue culture treatment is performed on a surface, or
a cycloolefin polymer plate with no surface coating.

17. The method for assessing an assessment target according to claim 15,
wherein the plate includes,
a glass plate, and
a resin layer provided on a surface of the glass plate,
the resin layer is formed from a cyclic olefin copolymer or polystyrene,
a plurality of through-holes corresponding to the plurality of wells are formed in the resin layer, and
regions exposed by the plurality of through-holes in the surface are not subjected to coating.

18. The method for assessing an assessment target according to claim 15,
wherein the plate includes,
a glass plate, and
a resin layer provided on a surface of the glass plate,
the resin layer is formed from a cyclic olefin copolymer or polystyrene,
a plurality of through-holes corresponding to the plurality of wells are formed in the resin layer,
a coating layer is formed in regions exposed by the plurality of through-holes in the surface, and
a material of the coating layer is fibronectin, collagen, or laminin.

19. A method for assessing an assessment target, comprising:
a step of acquiring statistical information based on at least one color feature quantity with respect to each of a plurality of assessment regions in a plate image corresponding to an image of an assessment plate that holds an assessment target in a plurality of wells provided in a plate; and
a step of detecting a transparency change of the plurality of assessment regions based on a similarity of between the statistical information and a reference statistical information for detecting a transparency change based on the at least one color feature quantity,
wherein the assessment target includes a tester,
the plurality of wells include a test substance well holding the assessment target that further includes a test substance, and
each of the plurality of assessment regions includes at least one well image corresponding to at least one of the wells.

20. The method for assessing an assessment target according to any one of claims 1 to 19, further comprising:
an irradiation step of irradiating the assessment plate with light,
wherein in the irradiation step, the assessment plate is irradiated with light having a wavelength within at least one wavelength range between a wavelength range of 430 to 460 nm and a wavelength range of 520 to 620 nm.

21. The method for assessing an assessment target according to claim 20,
wherein the light emitted to the assessment plate in the irradiation step is light having a first wavelength selected in a wavelength range of 430 to 460 nm and a second wavelength selected in a wavelength range of 520 to 620 nm.

22. The method for assessing an assessment target according to any one of claims 1 to 21, further comprising:
an imaging step of imaging the assessment plate.

23. The method for assessing an assessment target according to claim 22,
wherein in the imaging step, light having a wavelength within at least one wavelength range between a wavelength range of 430 to 460 nm, and a wavelength range of 520 to 620 nm is selectively received.

24. The method for assessing an assessment target according to claim 22 or 23,
wherein in the imaging step, the assessment plate is imaged from a bottom side of the plurality of wells.

25. An image processing device configured to execute the method for assessing an assessment target according to any one of claims 1 to 19.

26. A system for assessing an assessment target, comprising:
a plate support configured to hold an assessment plate holding an assessment target in a plurality of wells provided in a plate;
an imaging unit configured to image the assessment plate; and
the image processing device configured to assess a plate image corresponding to the assessment plate which is obtained based on an imaging result in the imaging unit according to claim 25.

27. The system for assessing an assessment target according to claim 26,
wherein the imaging unit is disposed on a bottom side of the plurality of wells.
